(19) Europäisches Patentamt
European Patent Office
Office européen des brevets

(11) EP 2 216 404 A1

(12) **EUROPEAN PATENT APPLICATION**

(43) Date of publication:
**11.08.2010 Bulletin 2010/32**

(21) Application number: **10162932.7**

(22) Date of filing: **26.10.2005**

(51) Int Cl.:
***C12N 15/00*** (2006.01) ***A01H 5/00*** (2006.01)
***A01K 67/027*** (2006.01) ***A61K 39/395*** (2006.01)
***A61P 9/00*** (2006.01) ***A61P 29/00*** (2006.01)
***A61P 31/04*** (2006.01) ***A61P 31/12*** (2006.01)
***A61P 35/00*** (2006.01) ***A61P 37/00*** (2006.01)
***A61P 37/08*** (2006.01)

(84) Designated Contracting States:
**AT BE BG CH CY CZ DE DK EE ES FI FR GB GR HU IE IS IT LI LT LU LV MC NL PL PT RO SE SI SK TR**

(30) Priority: **22.12.2004 PCT/JP2004/019261**

(62) Document number(s) of the earlier application(s) in accordance with Art. 76 EPC:
**05800043.1 / 1 829 962**

(71) Applicant: **Chugai Seiyaku Kabushiki Kaisha**
**Kita-ku**
**Tokyo 115-8543 (JP)**

(72) Inventors:
- **Tsuchiya, Masayuki**
**Gotenba-shi**
**Shizuoka 412-8513 (JP)**
- **Iijima, Shigeyuki**
**Gotenba-shi**
**Shizuoka 412-8513 (JP)**
- **Sugo, Izumi**
**Gotenba-shi**
**Shizuoka 412-8513 (JP)**
- **Sekimori, Yasuo**
**Gotenba-shi**
**Shizuoka 412-8513 (JP)**
- **Habu, Kiyoshi**
**Gotenba-shi**
**Shizuoka 412-8513 (JP)**
- **Sugimoto, Masamichi**
**Kamakura-shi**
**Kanagawa 247-8530 (JP)**

(74) Representative: **Dempster, Robert Charles**
**D Young & Co LLP**
**120 Holborn**
**London EC1N 2DY (GB)**

Remarks:
This application was filed on 17-05-2010 as a divisional application to the application mentioned under INID code 62.

# (54) Method of producing an antibody using a cell in which the function of fucose transporter is inhibited

(57) The present invention relates to a method of producing a recombinant protein particularly an antibody using a cell in which the function of a fucose transporter is inhibited. According to the present invention, a cell in which the expression of fucose transporter genes on both homologous chromosomes is artificially suppressed is provided.

EP 2 216 404 A1

SEQUENCE LISTING

<110> Chugai Seiyaku Kabushiki Kaisha

<120> A method of producing an antibody using a cell in which the function of fucose transporter is inhibited

<130> PH-2616-PCT

<150> PCT/JP2004/019261
<151> 2004-12-22

<160> 142

<170> PatentIn Ver. 2.1

<210> 1
<211> 10939
<212> DNA
<213> Cricetulus griseus

<400> 1
gagctcaatt aaccctcact aaagggagtc gactcgatcc tttacagaaa acttgcaaac 60
cctcttggag tagaaaagta gtagtatctg acacaagtat cagcaaaatg caaacttctc 120
cccatcccca gaaaaccatt ataaaaaccc ccatatctta tgcccaactg tagtgatata 180
ttatttatga tttattaaaa cttgcttaag gattcagaaa gcaaagtcag ccttaagcta 240
tagagaccag gcagtcagtg gtggtacaca cctttaatcc caggactcag gattaagaag 300
tagacggacc tctgttagtt caagtctacc attacctaca caagagtgaa gagtaaccga 360
tctcatgcct ttgatcccag cagctgggat catgtgcatt caatcccagc attcgggagt 420

tatataagac aggagcaagg tctcagagct ggcattcatt ctccagccac attgaggata 480
ggaaaacatt gaagtgtcag gatgctgagg agaggcagca gtttgaggtt tggtagaacc 540
aggatcacct tttggtctga ggtagagtaa gaactgtggc tggctgcttt gcttttctga 600
tcttcagctt gaagcttgaa ctccaatatt tgtctctggg tctattatta tcatgttaca 660
cctaacttta aagctgattt acgcaagaca gttgtaggtg gacctttctt tcctgcccac 720
cagttcccaa ataactgaca cggagactca atattaatta taaatgattg gttaatagct 780
cagtcttgtt actggctaac tcttacattt taaattaact catttccatc cctttacttg 840
ctgccatgtg gttcatggct tgttcaagtc ctgcttcttc tgtctctggc tggtgatgcc 900
tctggttctg ccctttatcc cagaattctc ctagtctggc tctcctgccc agctataggc 960
cagtcagctg tttattaacc aatgagaata atacatattt atagtgtaca aagattgctc 1020
ctcaacaccc aatttttat gtgcaacctg agaatctgga ctcattgccc tcatgcttgc 1080
agaggcggca cccttaccca ctaagccacc tttctagccc tgttgctttt gttttttgag 1140
acaggttcca ctatgtagcc caggctggcc tcaaactgac cattctcctg cctaaacctc 1200
ccgaacactg gaattatagt caaggcctac ctgccctggc attttcacac ttttatttcc 1260
tggctgagtc cattgacttt acactcatca aggttgaacc agttggagtt taattacagt 1320
gccaatcgca ctgaatccca cataatcaaa caacttcaag gaagcaaaaa accagttttt 1380
cctgaagatc aatgtcagct tgcctgattc agaatagacc cccgaaaaaa ggcaaatgct 1440
tgataaccaa tttcttctta ttgttcaatc ccctgctgct gtgtgtaagc tcctgagaaa 1500
ggacagtaag gggacattca tgatcagaga aagagcccca actccccccc cagccccacc 1560
cccaccctgt ccacagtctg ttggtttggt ttccccctgg ctgacaccca gaaatcacaa 1620
cataatcacc taggtcactg taacaagttc ctttctggaa aatgctacaa atgatattgg 1680
taacatgagt aatgaataat gcctggagtc caactccctt gtgacccagc aatgttttcc 1740
gtgggtgctc ccttccccag ctgcaggcct gacatgtacc ttaaaaagcc tcccctggag 1800
gacagaattt tgtgggtact atagtgttct cacaaatact tcccctaata cccttactta 1860
gttaccataa ataacatgca gcccctggtg aggcacacag ggctccaatg tacagcttct 1920
cagacactgc aggaaccttc ctctcctaat gcagcactgg tctcttcagg ctggacagca 1980
ggaacccata ccactccaat cctagtgtgg agtagagctg tctacgaaaa ccagcagatc 2040
tatagctaaa tgtgtttcaa ttttatgctt tgacaaattg tactgacccc acccccaccc 2100
cttccccctt gctgtgctgg gaattgaacc caggaccttg tgcatgccag gcaagtactc 2160

taacactgag ctatagcccc aatctttcat ccaagtctct atgtgtgccc acactcgctt 2220
tttattttga gacaaaaggt tcttattttg agataaggtc tcactatgtt gccttgactt 2280
tttttttttt ttttttttga acttttgacc ttcctacctc agctgagact acaagtcttt 2340
taccatcagg cccggctgat ggtaaaataa cagtatttga aatagtttaa acacatcatc 2400
ttaatggtca accacacaat ttccgaaatg ttgctggctc agtctggggc aaacctgtcc 2460
gccccaacat tggtgctagg aagaaagcac agacaagtag ccctcccagc tcaggagtaa 2520
aagacctgga gggggtggcc cacttcggtc aagttcacgg gatggggagg ggtaccctcc 2580
tccagtagtg gtggtatttg gcagttcctc caccgacgcc ctctggaagc acctgcttgg 2640
acccgcaaag ccaggaatgc agcttcctca agggactcgc cagcgagggt aacaggacag 2700
aggcgtccca agagggctgg ggcggaaggg ggaagacagg gtcggcctta gatagggcaa 2760
agggccttct ggctgtgttc ccggggtaac cgccccacca cgcctggagc ccgacgtggc 2820
gagcgatggg gacagcgagc aggaagtcgt actggggagg gccgcgtagc agatgcagcc 2880
gagggcggcg ctgccaggta cacccgaggg caccgcgggg gtgagcgcca ggtccctgaa 2940
ccagccaggc ctccagagcc gagtccggcg gaccgacggt acgttctgga atgggaaggg 3000
atccgggaca ccgaattgct gcattgaggg gctcagaggt tctgatgtgg gagtccagaa 3060
agggttttat ctaccggagg tgatgtgact tccggcctct ggaagtgctg ttggagtctc 3120
tgggaccttg ggtcctctcg actaggtttg gaaggggtga aatagggggta gggagaaagg 3180
agaggactgc agcaatgtct tcccgaacga cctgggttcg ggaggggtcg aaggacaagg 3240
ggctgttgtg gggggtcttc agacgcggag gggtggtatt ctattttctg ggaagatggt 3300
gtcgatgcac ttgaccaagt ctagtcgatc tgaagaggct aggggaacag acagtgagag 3360
aggatggtgg agggagtggc agaacccttc cagaaactgg gagaggctct agcacctgca 3420
accccttccc tggcctccgg ggagtcccag aagagggcag gaccatggac acaggtgcat 3480
tcgtgccggc gcgctccggc ctggcgaagg tgcgcgctct tggaggccgc gggagggcca 3540
gacgcgcgcc cggagagctg gccctttaag gctacccgga ggcgtgtcag gaaatgcgcc 3600
ctgagcccgc ccctcccgga acgcggcccg agacctggca agctgagacg gaactcggaa 3660
ctagcactcg gctcgcggcc tcggtgaggc cttgcgcccg ccatgcctct gtcattgccc 3720
ctcgggccgc ctccctgaac ctccgtgacc gccctgcagt cctccctccc ccccttcgac 3780
tcggcgggcg cttccgggcg ctcccgcagc ccgccctcca cgtagcccac acctccctct 3840
cggcgctccg cttcccacgc ggtccccgac ctgttctttc ctcctccacc ctgcccttct 3900

gtccctctcc cttcctttct cccctcgact cgtccctatt aggcaacagc ccctgtggtc 3960
cagccggcca tggctgtcaa ggctcacacc cttagctagg cccettctcc cttccctggg 4020
tcttgtctca tgaccccctg ccccgcccgg gagcgagcgc gatgtggagc agtgcctctg 4080
gcaagcagaa cttcacccaa gccatgtgac aattgaaggc tgtacccca gaccctaaca 4140
tcttggagcc ctgtagacca gggagtgctt ctggccgtgg ggtgacctag ctcttctacc 4200
accatgaaca gggcccctct gaagcggtcc aggatcctgc gcatggcgct gactggaggc 4260
tccactgcct ctgaggaggc agatgaagac agcaggaaca agccgtttct gctgcgggcg 4320
ctgcagatcg cgctggtcgt ctctctctac tgggtcacct ccatctccat ggtattcctc 4380
aacaagtacc tgctggacag cccctccctg cagctggata cccctatctt cgtcactttc 4440
taccaatgcc tggtgacctc tctgctgtgc aagggcctca gcactctggc cacctgctgc 4500
cctggcaccg ttgacttccc caccctgaac ctggacctta aggtggcccg cagcgtgctg 4560
ccactgtcgg tagtcttcat tggcatgata agtttcaata acctctgcct caagtacgta 4620
ggggtggcct tctacaacgt ggggcgctcg ctcaccaccg tgttcaatgt gcttctgtcc 4680
tacctgctgc tcaaacagac cacttccttc tatgccctgc tcacatgtgg catcatcatt 4740
ggtgagtggg gcccgggggc tgtgggagca ggatgggcat cgaactgaag ccctaaaggt 4800
caacactgta ggtaccttta cttactgtcc caggtccctt gcatcagcag ttacaggaag 4860
agccctgtag aaaacaaata acttccttat ggtcattcaa caagttaggg acccagccag 4920
ggtgaaaata atgttagcag caactacagc aaagatggct ctcgccactt gcatgattaa 4980
aatgtgccag gtactcagat ctaagcattg gatccacatt aactcaacta atccctatta 5040
caaggtaaaa tatatccgaa ttttacagag ggaaaaccaa ggcacagaga ggctaagtag 5100
cttgaccagg atcacacagc taataatcac tgacatagct gggatttaaa cataagcagt 5160
tacctccata gatcacacta tgaccaccat gccactgttc cttctcaaga gttccaggat 5220
cctgtctgtc cagttctctt taaagaggac aacacatctg acattgctac cttgaggtaa 5280
catttgaaat agtgggtaga catatgtttt aagttttatt cttacttttt atgtgtgtgt 5340
gtttgggggg ccaccacagt gtatgggtgg agataagggg acaacttaag aattggtcct 5400
ttctcccacc acatgggtgc tgaggtctga actcaggtca tcaggattgg cacaaatccc 5460
tttacccact gagccatttc actggtccaa tatatgtgtg cttttaagag gctttaacta 5520
ttttcccaga tgtgaatgtc ctgctgatca ttatcccctt ttacccggaa gccctctggg 5580
aggtgccatc cctgtggtcg tctgcataca aatggggaaa ctgcaactca gagaaacaag 5640

gctacttgcc agggccccac aagtaagata ggctgggatg ccatcccaga ctggccacac 5700
tccctggcct gtgcttcaag ccagtttact ttgttcctgc ccattggaag ttagcatgtt 5760
gcagtcaaac acaataacta caggccaaaa gtgcttttaa attaaagtca gatgaacttt 5820
taaacatcca gagctcctca actgcaggag ttacaacctg attctgcaac catctttgca 5880
gtgcccggta gtcatatgta gctagaggct cttggctagg acagcatgtg ttaggaaaca 5940
tctggccctg agatcattga attgagtgac tgctgggtga caaagaccaa ggcatccgtt 6000
ccctgagagt cctgggcaag cagcaatgtg accttcattt gtacctactc aggttcttta 6060
tctgtcctgt ttgacctact tagtctcctc tggtgtctca gaggcccagg ctgggtactc 6120
tggatgtcag gatcaggcca atgcgcacat ctgccctaga aatgtccccc tggttgagca 6180
gctcctgaat ccatcggtaa agggtctgga ccagggagga gtcagataaa aagctgacag 6240
cactggggga ctccatgggg aactcccacc tgcccccaca catccatcct aagagaactg 6300
gtattccttg tttcctcttt gtcctacaag gcaccctggg atcccacttc agtctcccag 6360
ccttgccagg gttagagggc atgagcctcc ttgtggggaa tttagatgca agaaggtaca 6420
gtcactagag aacctgagct cagatcccca aagtaaccag tacctgatag tgaggcagct 6480
gagaaccgca gcagcctgcc tgagtggctg aactctgcgg cctccggaac tggccccaac 6540
tgttgggtct cctcttcctt cctcctgtga gggagggccc atctctgata agtgctgtgg 6600
ggactctaga gtagggagga ggaggagcaa tctaagcagg ccttactgag aagtccttgc 6660
tggcatgtgg ctgcctgagg agtacagact gggaacaccc atttgaatga gtaaggtttt 6720
tcctgaaggc catggggagc cacggaggaa aatcatttta gttacaagac aaagagtaga 6780
ttggttaaca tgggagcaag gacatggccc caattttcat agatgaagga aattggaact 6840
cagagaggtt aagtaacttc tcccaaatag ctcagcttca aaatcacaga acagtcagag 6900
tctagatctc tctgatgcct gtgatggtcc tgccattcca tgttgctgat ccctgtggca 6960
tcagtaagcc tctaccttgt gggaatgcag gatctaaatg aagagaggaa gtgctggccc 7020
catgctgtgg tctggaaagc tatgcaggct ctttgagcag agagtgaccc acaagtgaat 7080
agagtcctat gagactcaaa gcaacatcca cccttaagca gctctaacca aatgctcaca 7140
ctgagggagc caaagccaag ttagagtcct gtgcttgccc aaggtcactt tgcctggccc 7200
tcctcctata gcacccgtgt tatcttatag ccctcattac agtgattaca attataatta 7260
gagaggtaac agggccacac tgtccttaca cattcccctg ctagattgta gctgggagag 7320
ggggagatgt aggtggctgg gggagtggga gggaagatgc agattttcat tctgggctct 7380

actccctcag ccatttttttg gtgtgggagt tagactttgg atatgttgat gatgaggtaa 7440
gggccacaga acagtctgaa ctgtggtatc agaatcctgt ccctctccct ctctcctcat 7500
ccctcttcac cttgtcactc ctctgtctgc tacaggtggt ttctggctgg gtatagacca 7560
agagggagct gagggcaccc tgtccctcat aggcaccatc ttcggggtgc tggccagcct 7620
ctgcgtctcc ctcaatgcca tctataccaa gaaggtgctc ccagcagtgg acaacagcat 7680
ctggcgccta accttctata acaatgtcaa tgcctgtgtg ctcttcttgc ccctgatggt 7740
tctgctgggt gagctccgtg ccctccttga ctttgctcat ctgtacagtg cccacttctg 7800
gctcatgatg acgctgggtg gcctcttcgg ctttgccatt ggctatgtga caggactgca 7860
gatcaaattc accagtcccc tgacccacaa tgtatcaggc acagccaagg cctgtgcgca 7920
gacagtgctg gccgtgctct actatgaaga gactaagagc ttcctgtggt ggacaagcaa 7980
cctgatggtg ctgggtggct cctcagccta tacctgggtc aggggctggg agatgcagaa 8040
gacccaagag gaccccagct ccaaagaggg tgagaagagt gctattgggg tgtgagcttc 8100
ttcagggacc tgggactgaa cccaagtggg gcctacacag cactgaaggc ttcccatgga 8160
gctagccagt gtggccctga gcaatactgt ttacatcctc cttggaatat gatctaagag 8220
gagccagggt ctttcctggt aatgtcagaa agctgccaaa tctcctgtct gccccatctt 8280
gttttgggaa aaccctacca ggaatggcac ccctacctgc ctcctcctag agcctgtcta 8340
cctccatatc atctctgggg ttgggaccag ctgcagcctt aaggggctgg attgatgaag 8400
tgatgtcttc tacacaaggg agatgggttg tgatcccact aattgaaggg atttgggtga 8460
ccccacacct ctgggatcca gggcaggtag agtagtagct taggtgctat taacatcagg 8520
aacacctcag cctgcctttg aagggaagtg ggagcttggc caagggagga aatggccatt 8580
ctgccctctt cagtgtggat gagtatggca gacctgttca tggcagctgc accctggggt 8640
ggctgataag aaaacattca cctctgcatt tcatatttgc agctctagaa cgggggagag 8700
ccacacatct tttacgggtt aagtagggtg atgagctcct ccgcagtccc taaccccagc 8760
tttacctgcc tggcttccct tggcccagct acctagctgt actccctttc tgtactcttc 8820
tcttctccgt catggcctcc cccaacacct ccatctgcag gcaggaagtg gagtccactt 8880
gtaacctctg ttcccatgac agagcccttt gaatacctga acccctcatg acagtaagag 8940
acatttatgt tctctggggc tggggctgaa ggagcccact ggttctcact tagcctatct 9000
ggctcctgtc acaaaaaaaa aaaaagaaaa aaaaaaagca taaaccaagt tactaagaac 9060
agaagttggt ttataacgtt ctggggcagc aaagcccaga tgaagggacc catcgacccc 9120

ctctgtccat atcctcatgc tgcagaagta caggcaagct cctttaagcc tcatatagga 9180
acactagcct cactcatgag ggttttactc catgacctgt caacctcaaa gccttcaaca 9240
tgaggactcc aacgtaaatt tggggacaga agcactcaga ccatacccca gcaccacacc 9300
ctcctaacct cagggtagct gtcattctcc tagtctcctc tcttgggcct ttagaacccc 9360
catttccttg ggtaatgtc tgatgttttt gtccctgtca taaaaagatg gagagactgt 9420
gtccagcctt tgattcctac ttcctacaat cccaggttct aatgaagttt gtggggcctg 9480
atgccctgag ttgtatgtga tttaataata aaaaagcaag atacagcatg tgtgtggact 9540
gagtgagggc cacagggatc taaaagccaa gtgtgagggg acccagctac agcaggcagc 9600
atcctgagcc tggaatctct tcaggacaag aattctccat atacctacct actctgggga 9660
gtaggtggcc agagttcaag cttcccttag taccaactac cactggctgt gctcttactg 9720
aaggcagaca tggcactgag tgctgtccat ctgtcactca tctccacagc cattcctaat 9780
gtgtggggtg ggagccatca ccaaacccca ttttcagata aggacacagg ctcagagagg 9840
cttgtgtgga gaaaagtagc agcagaattc agagagctgg gtctcctgca gcaccttgga 9900
ctgccagcag ccacagtgct tgtcacacag cacatactca aaagaatgcc agccccctca 9960
gcctagagtg cctggccttt ctttcagatg aggaagaggg tcaaagctgt tagcttgccc 10020
accatatgac cacatacatg accaacagct tgagggaggg aggattactg tggctcccag 10080
cctgagaggt gggacaccca aatgtattag gtccttgaat cagggctgac cttgtgattc 10140
agtcactcct accagaatgc tggggaatgg ggatgccaaa ggcaaaggag gctttctaag 10200
gtgtggtgta agataggcat ttctgcttcc atgtacacct gtgagcagag taggaaggcc 10260
ctgtggagaa tatatcccac aaaccagtag cccttcctgg cagtgggtga atactgccac 10320
cctatacccc tatgcaaggc cagtagaacc acccaaccca caacatctag agaaattaca 10380
ggtcatctta agcctctaaa ttgtggagaa actcgacatg cgcacgattc ctaacctgct 10440
agcctagggt gcggggtgga taatttaagg aaactggggt ttcttataga atcggaggct 10500
ccatgaagtc accctgacaa gaggtcagca atagccagca gcagtggcta ctcctaagcc 10560
tccagacaga gcaccctgtg aatgtacctt attctcacat ctgggtgtct ataggtgtga 10620
ctgggtcaga tgtcacccag gccattgcaa tgggccctta gccccatggg gtgttgggat 10680
agcagccaag cagctcccat gctgagatac tgcctgcagt agactgatgg ataagaaaac 10740
aaggcccaaa atgttttctt tccagacttg atctttcttt gttcaaaaat gctgttttcc 10800
cttaaacttg cccaaaccca ttgttttgca gttgaggaaa ataaggcata gaaagattaa 10860

aggaagtttc tgaggttaca gagcaaagta ctggcttcac ctgaaataga caggtgtgcc 10920
ctgatcctga tttgagctc 10939

<210> 2
<211> 352
<212> PRT
<213> Cricetulus griseus

<400> 2
Met Ala Leu Thr Gly Gly Ser Thr Ala Ser Glu Glu Ala Asp Glu Asp
1 5 10 15

Ser Arg Asn Lys Pro Phe Leu Leu Arg Ala Leu Gln Ile Ala Leu Val
20 25 30

Val Ser Leu Tyr Trp Val Thr Ser Ile Ser Met Val Phe Leu Asn Lys
35 40 45

Tyr Leu Leu Asp Ser Pro Ser Leu Gln Leu Asp Thr Pro Ile Phe Val
50 55 60

Thr Phe Tyr Gln Cys Leu Val Thr Ser Leu Leu Cys Lys Gly Leu Ser
65 70 75 80

Thr Leu Ala Thr Cys Cys Pro Gly Thr Val Asp Phe Pro Thr Leu Asn
85 90 95

Leu Asp Leu Lys Val Ala Arg Ser Val Leu Pro Leu Ser Val Val Phe

100 105 110

Ile Gly Met Ile Ser Phe Asn Asn Leu Cys Leu Lys Tyr Val Gly Val
115 120 125

Ala Phe Tyr Asn Val Gly Arg Ser Leu Thr Thr Val Phe Asn Val Leu
130 135 140

Leu Ser Tyr Leu Leu Leu Lys Gln Thr Thr Ser Phe Tyr Ala Leu Leu
145 150 155 160

Thr Cys Gly Ile Ile Ile Gly Gly Phe Trp Leu Gly Ile Asp Gln Glu
165 170 175

Gly Ala Glu Gly Thr Leu Ser Leu Ile Gly Thr Ile Phe Gly Val Leu
180 185 190

Ala Ser Leu Cys Val Ser Leu Asn Ala Ile Tyr Thr Lys Lys Val Leu
195 200 205

Pro Ala Val Asp Asn Ser Ile Trp Arg Leu Thr Phe Tyr Asn Asn Val
210 215 220

Asn Ala Cys Val Leu Phe Leu Pro Leu Met Val Leu Leu Gly Glu Leu
225 230 235 240

Arg Ala Leu Leu Asp Phe Ala His Leu Tyr Ser Ala His Phe Trp Leu
245 250 255

Met Met Thr Leu Gly Gly Leu Phe Gly Phe Ala Ile Gly Tyr Val Thr
260 265 270

Gly Leu Gln Ile Lys Phe Thr Ser Pro Leu Thr His Asn Val Ser Gly
275 280 285

Thr Ala Lys Ala Cys Ala Gln Thr Val Leu Ala Val Leu Tyr Tyr Glu
290 295 300

Glu Thr Lys Ser Phe Leu Trp Trp Thr Ser Asn Leu Met Val Leu Gly
305 310 315 320

Gly Ser Ser Ala Tyr Thr Trp Val Arg Gly Trp Glu Met Gln Lys Thr
325 330 335

Gln Glu Asp Pro Ser Ser Lys Glu Gly Glu Lys Ser Ala Ile Gly Val
340 345 350

<210> 3
<211> 32
<212> DNA
<213> Artificial Sequence

<220>
<223> Description of Artificial Sequence: Primer

<400> 3
ggatcctgcg catgaaaaag cctgaactca cc 32

<210> 4
<211> 29
<212> DNA
<213> Artificial Sequence

<220>
<223> Description of Artificial Sequence: Primer

<400> 4
gcggccgcct attcctttgc cctcggacg 29

<210> 5
<211> 33
<212> DNA
<213> Artificial Sequence

<220>
<223> Description of Artificial Sequence: Primer

<400> 5
atgcatgcca ccatgaaaaa gcctgaactc acc 33

<210> 6

<211> 28

<212> DNA

<213> Artificial Sequence

<220>

<223> Description of Artificial Sequence: Primer

<400> 6

ggatcccagg ctttacactt tatgcttc 28

<210> 7

<211> 25

<212> DNA

<213> Artificial Sequence

<220>

<223> Description of Artificial Sequence: Primer

<400> 7

gctgtctgga gtactgtgca tctgc 25

<210> 8

<211> 27

<212> DNA

<213> Artificial Sequence

<220>

<223> Description of Artificial Sequence: Primer

<400> 8

ggaatgcagc ttcctcaagg gactcgc 27

<210> 9

<211> 27

<212> DNA

<213> Artificial Sequence

<220>

<223> Description of Artificial Sequence: Primer

<400> 9

tgcatcaggt cggagacgct gtcgaac 27

<210> 10

<211> 27

<212> DNA

<213> Artificial Sequence

<220>

<223> Description of Artificial Sequence: Primer

<400> 10

gcactcgtcc gagggcaaag gaatagc 27

<210> 11
<211> 24
<212> DNA
<213> Artificial Sequence

<220>
<223> Description of Artificial Sequence: Primer

<400> 11
tgtgctggga attgaaccca ggac 24

<210> 12
<211> 22
<212> DNA
<213> Artificial Sequence

<220>
<223> Description of Artificial Sequence: Primer

<400> 12
ctacttgtct gtgctttctt cc 22

<210> 13
<211> 27
<212> DNA

<213> Artificial Sequence

<220>

<223> Description of Artificial Sequence: Primer

<400> 13

ctcgactcgt ccctattagg caacagc 27

<210> 14

<211> 27

<212> DNA

<213> Artificial Sequence

<220>

<223> Description of Artificial Sequence: Primer

<400> 14

tcagaggcag tggagcctcc agtcagc 27

<210> 15

<211> 31

<212> DNA

<213> Artificial Sequence

<220>

<223> Description of Artificial Sequence: Primer

<400> 15
gatatcatgg ccgggaccgt gcgcaccgcg t 31

<210> 16
<211> 31
<212> DNA
<213> Artificial Sequence

<220>
<223> Description of Artificial Sequence: Primer

<400> 16
gctagctcag tgcaccagga agaagaagca c 31

<210> 17
<211> 1743
<212> DNA
<213> Artificial Sequence

<220>
<223> Description of Artificial Sequence: Primer

<400> 17
atggccggga ccgtgcgcac cgcgtgcttg gtggtggcga tgctgctcag cttggacttc 60
ccgggacagg cgcagccccc gccgccgccg ccggacgcca cctgtcacca agtccgctcc 120
ttcttccaga gactgcagcc cggactcaag tgggtgccag aaactcccgt gccaggatca 180
gatttgcaag tatgtctccc taagggccca acatgctgct caagaaagat ggaagaaaaa 240

taccaactaa cagcacgatt gaacatggaa cagctgcttc agtctgcaag tatggagctc 300
aagttcttaa ttattcagaa tgctgcggtt ttccaagagg cctttgaaat tgttgttcgc 360
catgccaaga actacaccaa tgccatgttc aagaacaact acccaagcct gactccacaa 420
gcttttgagt ttgtgggtga atttttcaca gatgtgtctc tctacatctt gggttctgac 480
atcaatgtag atgacatggt caatgaattg tttgacagcc tgtttccagt catctatacc 540
cagctaatga acccaggcct gcctgattca gccttggaca tcaatgagtg cctccgagga 600
gcaagacgtg acctgaaagt atttgggaat ttccccaagc ttattatgac ccaggtttcc 660
aagtcactgc aagtcactag gatcttcctt caggctctga atcttggaat tgaagtgatc 720
aacacaactg atcacctgaa gttcagtaag gactgtggcc gaatgctcac cagaatgtgg 780
tactgctctt actgccaggg actgatgatg gttaaaccct gtggcggtta ctgcaatgtg 840
gtcatgcaag gctgtatggc aggtgtggtg gagattgaca agtactggag agaatacatt 900
ctgtcccttg aagaacttgt gaatggcatg tacagaatct atgacatgga gaacgtactg 960
cttggtctct tttcaacaat ccatgattct atccagtatg tccagaagaa tgcaggaaag 1020
ctgaccacca ctattggcaa gttatgtgcc cattctcaac aacgccaata tagatctgct 1080
tattatcctg aagatctctt tattgacaag aaagtattaa aagttgctca tgtagaacat 1140
gaagaaacct tatccagccg aagaagggaa ctaattcaga agttgaagtc tttcatcagc 1200
ttctatagtg ctttgcctgg ctacatctgc agccatagcc ctgtggcgga aaacgacacc 1260
ctttgctgga atggacaaga actcgtggag agatacagcc aaaaggcagc aaggaatgga 1320
atgaaaaacc agttcaatct ccatgagctg aaaatgaagg gccctgagcc agtggtcagt 1380
caaattattg acaaactgaa gcacattaac cagctcctga gaaccatgtc tatgcccaaa 1440
ggtagagttc tggataaaaa cctggatgag gaagggtttg aaagtggaga ctgcggtgat 1500
gatgaagatg agtgcattgg aggctctggt gatggaatga taaaagtgaa gaatcagctc 1560
cgcttccttg cagaactggc ctatgatctg gatgtggatg atgcgcctgg aaacagtcag 1620
caggcaactc cgaaggacaa cgagataagc acctttcaca acctcgggaa cgttcattcc 1680
ccgctgaagc ttctcaccag catggccatc tcggtggtgt gcttcttctt cctggtgcac 1740
tga 1743

<210> 18

<211> 580

<212> PRT

<213> Artificial Sequence

<220>

<223> Description of Artificial Sequence: Primer

<400> 18

Met Ala Gly Thr Val Arg Thr Ala Cys Leu Val Val Ala Met Leu Leu
1 5 10 15

Ser Leu Asp Phe Pro Gly Gln Ala Gln Pro Pro Pro Pro Pro Pro Asp
20 25 30

Ala Thr Cys His Gln Val Arg Ser Phe Phe Gln Arg Leu Gln Pro Gly
35 40 45

Leu Lys Trp Val Pro Glu Thr Pro Val Pro Gly Ser Asp Leu Gln Val
50 55 60

Cys Leu Pro Lys Gly Pro Thr Cys Cys Ser Arg Lys Met Glu Glu Lys
65 70 75 80

Tyr Gln Leu Thr Ala Arg Leu Asn Met Glu Gln Leu Leu Gln Ser Ala
85 90 95

Ser Met Glu Leu Lys Phe Leu Ile Ile Gln Asn Ala Ala Val Phe Gln
100 105 110

Glu Ala Phe Glu Ile Val Val Arg His Ala Lys Asn Tyr Thr Asn Ala
115 120 125

Met Phe Lys Asn Asn Tyr Pro Ser Leu Thr Pro Gln Ala Phe Glu Phe
130 135 140

Val Gly Glu Phe Phe Thr Asp Val Ser Leu Tyr Ile Leu Gly Ser Asp
145 150 155 160

Ile Asn Val Asp Asp Met Val Asn Glu Leu Phe Asp Ser Leu Phe Pro
165 170 175

Val Ile Tyr Thr Gln Leu Met Asn Pro Gly Leu Pro Asp Ser Ala Leu
180 185 190

Asp Ile Asn Glu Cys Leu Arg Gly Ala Arg Arg Asp Leu Lys Val Phe
195 200 205

Gly Asn Phe Pro Lys Leu Ile Met Thr Gln Val Ser Lys Ser Leu Gln
210 215 220

Val Thr Arg Ile Phe Leu Gln Ala Leu Asn Leu Gly Ile Glu Val Ile
225 230 235 240

Asn Thr Thr Asp His Leu Lys Phe Ser Lys Asp Cys Gly Arg Met Leu
245 250 255

Thr Arg Met Trp Tyr Cys Ser Tyr Cys Gln Gly Leu Met Met Val Lys
260 265 270

Pro Cys Gly Gly Tyr Cys Asn Val Val Met Gln Gly Cys Met Ala Gly
275 280 285

Val Val Glu Ile Asp Lys Tyr Trp Arg Glu Tyr Ile Leu Ser Leu Glu
290 295 300

Glu Leu Val Asn Gly Met Tyr Arg Ile Tyr Asp Met Glu Asn Val Leu
305 310 315 320

Leu Gly Leu Phe Ser Thr Ile His Asp Ser Ile Gln Tyr Val Gln Lys
325 330 335

Asn Ala Gly Lys Leu Thr Thr Thr Ile Gly Lys Leu Cys Ala His Ser
340 345 350

Gln Gln Arg Gln Tyr Arg Ser Ala Tyr Tyr Pro Glu Asp Leu Phe Ile
355 360 365

Asp Lys Lys Val Leu Lys Val Ala His Val Glu His Glu Glu Thr Leu
370 375 380

Ser Ser Arg Arg Arg Glu Leu Ile Gln Lys Leu Lys Ser Phe Ile Ser
385 390 395 400

Phe Tyr Ser Ala Leu Pro Gly Tyr Ile Cys Ser His Ser Pro Val Ala
405 410 415

Glu Asn Asp Thr Leu Cys Trp Asn Gly Gln Glu Leu Val Glu Arg Tyr

420 425 430

Ser Gln Lys Ala Ala Arg Asn Gly Met Lys Asn Gln Phe Asn Leu His
435 440 445

Glu Leu Lys Met Lys Gly Pro Glu Pro Val Val Ser Gln Ile Ile Asp
450 455 460

Lys Leu Lys His Ile Asn Gln Leu Leu Arg Thr Met Ser Met Pro Lys
465 470 475 480

Gly Arg Val Leu Asp Lys Asn Leu Asp Glu Glu Gly Phe Glu Ser Gly
485 490 495

Asp Cys Gly Asp Asp Glu Asp Glu Cys Ile Gly Gly Ser Gly Asp Gly
500 505 510

Met Ile Lys Val Lys Asn Gln Leu Arg Phe Leu Ala Glu Leu Ala Tyr
515 520 525

Asp Leu Asp Val Asp Asp Ala Pro Gly Asn Ser Gln Gln Ala Thr Pro
530 535 540

Lys Asp Asn Glu Ile Ser Thr Phe His Asn Leu Gly Asn Val His Ser
545 550 555 560

Pro Leu Lys Leu Leu Thr Ser Met Ala Ile Ser Val Val Cys Phe Phe
565 570 575

Phe Leu Val His
580

<210> 19
<211> 31
<212> DNA
<213> Artificial Sequence

<220>
<223> Description of Artificial Sequence: Primer

<400> 19
atagaattcc accatggccg ggaccgtgcg c 31

<210> 20
<211> 31
<212> DNA
<213> Artificial Sequence

<220>
<223> Description of Artificial Sequence: Primer

<400> 20
ataggatccc ttcagcgggg aatgaacgtt c 31

<210> 21

<211> 21

<212> DNA

<213> Artificial Sequence

<220>

<223> Description of Artificial Sequence: Primer

<400> 21

gggccagtgg atagacagat g 21

<210> 22

<211> 24

<212> DNA

<213> Artificial Sequence

<220>

<223> Description of Artificial Sequence: Primer

<400> 22

caggggccag tggatagacc gatg 24

<210> 23

<211> 24

<212> DNA

<213> Artificial Sequence

<220>

<223> Description of Artificial Sequence: Primer

<400> 23

caggggccag tggatagact gatg 24

<210> 24

<211> 23

<212> DNA

<213> Artificial Sequence

<220>

<223> Description of Artificial Sequence: Primer

<400> 24

gctcactgga tggtgggaag atg 23

<210> 25

<211> 1392

<212> DNA

<213> Mus musculus

<400> 25

atgaacttcg ggctcacctt gattttcctt gtccttactt taaaaggtgt ccagtgtgag 60

gtgcaactgg tggagtctgg gggaggctta gtgaagcctg gaggatccct gaaactctcc 120

tgtgcagcct ctggattcac tttcagtcgc tatgccatgt cttgggttcg ccagattcca 180

gagaagatac tggagtgggt cgcagccatt gatagtagtg gtggtgacac ctactattta 240

gacactgtga aggaccgatt caccatctcc agagacaatg ccaataatac cctgcacctg 300

caaatgcgca gtctgaggtc tgaggacaca gccttgtatt actgtgtaag acaggggggg 360
gcttactggg gccaagggac tctggtcact gtctctgcag ctagcaccaa gggcccatcg 420
gtcttccccc tggcaccctc ctccaagagc acctctgggg gcacagcggc cctgggctgc 480
ctggtcaagg actacttccc cgaaccggtg acggtgtcgt ggaactcagg cgccctgacc 540
agcggcgtgc acaccttccc ggctgtccta cagtcctcag gactctactc cctcagcagc 600
gtggtgaccg tgccctccag cagcttgggc acccagacct acatctgcaa cgtgaatcac 660
aagcccagca acaccaaggt ggacaagaaa gttgagccca aatcttgtga caaaactcac 720
acatgcccac cgtgcccagc acctgaactc ctggggggac cgtcagtctt cctcttcccc 780
ccaaaaccca aggacaccct catgatctcc cggacccctg aggtcacatg cgtggtggtg 840
gacgtgagcc acgaagaccc tgaggtcaag ttcaactggt acgtggacgg cgtggaggtg 900
cataatgcca agacaaagcc gcgggaggag cagtacaaca gcacgtaccg tgtggtcagc 960
gtcctcaccg tcctgcacca ggactggctg aatggcaagg agtacaagtg caaggtctcc 1020
aacaaagccc tcccagcccc catcgagaaa accatctcca aagccaaagg gcagccccga 1080
gaaccacagg tgtacaccct gcccccatcc cgggatgagc tgaccaagaa ccaggtcagc 1140
ctgacctgcc tggtcaaagg cttctatccc agcgacatcg ccgtggagtg ggagagcaat 1200
gggcagccgg agaacaacta caagaccacg cctcccgtgc tggactccga cggctccttc 1260
ttcctctaca gcaagctcac cgtggacaag agcaggtggc agcaggggaa cgtcttctca 1320
tgctccgtga tgcatgaggc tctgcacaac cactacacgc agaagagcct ctccctgtct 1380
ccgggtaaat ga 1392

<210> 26
<211> 342
<212> DNA
<213> Mus musculus

<400> 26
gaggtgcacc tggtggagtc tgggggaggc ttagtgaagc ctggagggtc cctgaaactc 60
tcctgtgcag cctctggatt cactttcagt aactatgcca tgtcttgggt tcgccagact 120

ccagagaaga ggctggagtg ggtcgcagcc attaataata atggtgatga cacctactat 180
ttagacactg tgaaggaccg attcaccatc tccagagaca atgccaagaa caccctgtac 240
ctgcaaatga gcagtctgag gtctgaggac acagccctgt attactgtgt aagacaaggg 300
ggggcttact ggggccaagg gactctggtc actgtctctg ca 342

<210> 27
<211> 1413
<212> DNA
<213> Mus musculus

<400> 27
atgggatgga actggatctt tattttaatc ctgtcagtaa ctacaggtgt ccactctgag 60
gtccagctgc agcagtctgg acctgagctg gtgaagcctg gggcttcagt gaagatatcc 120
tgcaaggctt ctggttactc attcactggc tactacatgc actgggtgaa gcaaagtcct 180
gaaaagagcc ttgagtggat tggagagatt aatcctagca ctggtggtac tacctacaac 240
cagaagttca aggccaaggc cacattgact gtagacaaat cctccagcac agcctacatg 300
cagctcaaga gcctgacatc tgaggactct gcagtctatt actgtgcaag gaggggcgga 360
ttaactggga cgagcttctt tgcttactgg ggccaaggga ctctggtcac tgtctctgca 420
gctagcacca agggcccatc ggtcttcccc ctggcaccct cctccaagag cacctctggg 480
ggcacagcgg ccctgggctg cctggtcaag gactacttcc ccgaaccggt gacggtgtcg 540
tggaactcag gcgccctgac cagcggcgtg cacaccttcc cggctgtcct acagtcctca 600
ggactctact ccctcagcag cgtggtgacc gtgccctcca gcagcttggg cacccagacc 660
tacatctgca acgtgaatca caagcccagc aacaccaagg tggacaagaa agttgagccc 720
aaatcttgtg acaaaactca cacatgccca ccgtgcccag cacctgaact cctgggggga 780
ccgtcagtct tcctcttccc cccaaaaccc aaggacaccc tcatgatctc ccggacccct 840
gaggtcacat gcgtggtggt ggacgtgagc cacgaagacc ctgaggtcaa gttcaactgg 900
tacgtggacg gcgtggaggt gcataatgcc aagacaaagc cgcgggagga gcagtacaac 960
agcacgtacc gtgtggtcag cgtcctcacc gtcctgcacc aggactggct gaatggcaag 1020

gagtacaagt gcaaggtctc caacaaagcc ctcccagccc ccatcgagaa aaccatctcc 1080
aaagccaaag ggcagccccg agaaccacag gtgtacaccc tgcccccatc ccgggatgag 1140
ctgaccaaga accaggtcag cctgacctgc ctggtcaaag gcttctatcc cagcgacatc 1200
gccgtggagt gggagagcaa tgggcagccg gagaacaact acaagaccac gcctcccgtg 1260
ctggactccg acggctcctt cttcctctac agcaagctca ccgtggacaa gagcaggtgg 1320
cagcagggga acgtcttctc atgctccgtg atgcatgagg ctctgcacaa ccactacacg 1380
cagaagagcc tctccctgtc tccgggtaaa tga 1413

<210> 28
<211> 354
<212> DNA
<213> Mus musculus

<400> 28
caggtcactc tgaaagagtc tggccctggg atattgcagc cctcccagac cctcagtctg 60
acttgttctt tctctgggtt ttcactgagc acttatggta tgggtgtagg ttggattcgt 120
cagccttcag ggatgggtct ggagtggctg gccaacattt ggtggtatga tgctaagtac 180
tataactctg acctgaagag ccggctcaca atctccaagg atacctccaa caaccaggtg 240
ttcctcaaga tctccagtgt ggacacttca gatactgcca catactactg tgctcaaatg 300
ggactggcct ggtttgctta ctggggccaa gggactctgg tcactgtctc tgca 354

<210> 29
<211> 354
<212> DNA
<213> Mus musculus

<400> 29

caggtcactc tgaaagagtc tggccctggg atattgcagc cctcccagac cctcagtctg 60
acttgttctt tctctgggtt ttcactgagc atttatggta tgggtgtagg ttggattcgt 120
cagccttcag ggaagggtct ggagtggctg gccaacattt ggtggaatga tgataagtac 180
tataactcag ccctgaagag ccggctcaca atctccaagg atacctccaa caaccaggta 240
ttcctcaaga tctccagtgt ggacactgca gatactgcca catactactg tgctcaaata 300
ggttacttct actttgacta ctggggccaa ggcaccactc tcacagtctc ctca 354

<210> 30
<211> 1416
<212> DNA
<213> Mus musculus

<400> 30
atgaacttcg ggctcacctt gattttcctc gtccttactt taaaaggtgt ccagtgtgag 60
gtgcagctgg tggagtctgg gggagactta gtgaagcctg gagggaccct gaaactctcc 120
tgtgcagcct ctggatccac tttcagtaac tatgccatgt cttgggttcg ccagactcca 180
gagaagaggc tggagtgggt cgcagccatt gatagtaatg gaggtaccac ctactatcca 240
gacactatga aggaccgatt caccatttcc agagacaatg ccaagaacac cctgtacctg 300
caaatgaaca gtctgaggtc tgaagacaca gccttttatc actgtacaag acataatgga 360
gggtatgaaa actacggctg gtttgcttac tggggccaag ggactctggt cactgtctct 420
gcagctagca ccaagggccc atcggtcttc cccctggcac cctcctccaa gagcacctct 480
gggggcacag cggccctggg ctgcctggtc aaggactact tccccgaacc ggtgacggtg 540
tcgtggaact caggcgccct gaccagcggc gtgcacacct tcccggctgt cctacagtcc 600
tcaggactct actccctcag cagcgtggtg accgtgccct ccagcagctt gggcacccag 660
acctacatct gcaacgtgaa tcacaagccc agcaacacca aggtggacaa gaaagttgag 720
cccaaatctt gtgacaaaac tcacacatgc ccaccgtgcc cagcacctga actcctgggg 780
ggaccgtcag tcttcctctt ccccccaaaa cccaaggaca ccctcatgat ctcccggacc 840
cctgaggtca catgcgtggt ggtggacgtg agccacgaag accctgaggt caagttcaac 900

tggtacgtgg acggcgtgga ggtgcataat gccaagacaa agccgcggga ggagcagtac 960
aacagcacgt accgtgtggt cagcgtcctc accgtcctgc accaggactg gctgaatggc 1020
aaggagtaca agtgcaaggt ctccaacaaa gccctcccag cccccatcga gaaaaccatc 1080
tccaaagcca aagggcagcc ccgagaacca caggtgtaca ccctgccccc atcccgggat 1140
gagctgacca agaaccaggt cagcctgacc tgcctggtca aaggcttcta tcccagcgac 1200
atcgccgtgg agtgggagag caatgggcag ccggagaaca actacaagac cacgcctccc 1260
gtgctggact ccgacggctc cttcttcctc tacagcaagc tcaccgtgga caagagcagg 1320
tggcagcagg ggaacgtctt ctcatgctcc gtgatgcatg aggctctgca caaccactac 1380
acgcagaaga gcctctccct gtctccgggt aaatga 1416

<210> 31
<211> 366
<212> DNA
<213> Mus musculus

<400> 31
gaggtgcagc tggtggagtc tgggggagac ttagtgaagc ctggagggtc cctgaaactc 60
tcctgtgcag cctctggatt cactttcagt agctatgcca tgtcttgggt tcgccagact 120
ccagagaaga ggctggagtg ggtcgcagcc attaatagta atggaggtac cacctactat 180
ccagacacta tgaaggaccg attcaccatc tccagagaca atgccaagaa caccctgtac 240
ctgcaaatga gcagtctgag gtctgaagac tcagccttgt attactgtac aagacataat 300
ggagggtatg aaaactacgg ctggtttgct tactggggcc aagggactct ggtcactgtc 360
tctgca 366

<210> 32
<211> 1413
<212> DNA

<213> Mus musculus

<400> 32

atggaatcta actggatact tccttttatt ctgtcggtag cttcaggggt ctactcagag 60
gttcagctcc agcagtctgg gactgtgctg gcaaggcctg gggcttcagt gaagatgtcc 120
tgcaaggctt ctggctacac ctttactggc tactggatgc gctgggtaaa acagaggcct 180
ggacagggtc tggaatggat tggcgctatt tatcctggaa atagtgatac aacatacaac 240
cagaagttca agggcaaggc caaactgact gcagtcacat ctgtcagcac tgcctacatg 300
gaactcagca gcctgacaaa tgaggactct gcggtctatt actgttcaag atcgggggac 360
ctaactgggg ggtttgctta ctggggccaa gggactctgg tcactgtctc tacagccaaa 420
gctagcacca agggcccatc ggtcttcccc ctggcaccct cctccaagag cacctctggg 480
ggcacagcgg ccctgggctg cctggtcaag gactacttcc ccgaaccggt gacggtgtcg 540
tggaactcag gcgccctgac cagcggcgtg cacaccttcc cggctgtcct acagtcctca 600
ggactctact ccctcagcag cgtggtgacc gtgccctcca gcagcttggg cacccagacc 660
tacatctgca acgtgaatca caagcccagc aacaccaagg tggacaagaa agttgagccc 720
aaatcttgtg acaaaactca cacatgccca ccgtgcccag cacctgaact cctgggggga 780
ccgtcagtct tcctcttccc cccaaaaccc aaggacaccc tcatgatctc ccggacccct 840
gaggtcacat gcgtggtggt ggacgtgagc cacgaagacc ctgaggtcaa gttcaactgg 900
tacgtggacg gcgtggaggt gcataatgcc aagacaaagc cgcgggagga gcagtacaac 960
agcacgtacc gtgtggtcag cgtcctcacc gtcctgcacc aggactggct gaatggcaag 1020
gagtacaagt gcaaggtctc caacaaagcc ctcccagccc ccatcgagaa aaccatctcc 1080
aaagccaaag ggcagccccg agaaccacag gtgtacaccc tgcccccatc ccgggatgag 1140
ctgaccaaga accaggtcag cctgacctgc ctggtcaaag gcttctatcc cagcgacatc 1200
gccgtggagt gggagagcaa tgggcagccg gagaacaact acaagaccac gcctcccgtg 1260
ctggactccg acggctcctt cttcctctac agcaagctca ccgtggacaa gagcaggtgg 1320
cagcagggga acgtcttctc atgctccgtg atgcatgagg ctctgcacaa ccactacacg 1380
cagaagagcc tctccctgtc tccgggtaaa tga 1413

<210> 33

<211> 357

<212> DNA

<213> Mus musculus

<400> 33

gaggttcagc tccagcagtc tgggactgtg ctggcaaggc ctggggcttc agtgaagatg 60

tcctgcaagg cttctggcta cacctttacc ggctactgga tgcactgggt aaaacagagg 120

cctggacagg gtctggaatg gattggcgct atttatcctg gaaatagtga tactaactac 180

aaccagaagt tcaagggcaa ggccaaactg actgcagtca catctgccag cactgcctac 240

atggagctca gcagcctgac aaatgaggac gctgcggtct atcactgtac aagatcgggg 300

gacctaactg gggggcttgc ttactggggc caaggggactc tggtcactgt ctctgca 357

<210> 34

<211> 372

<212> DNA

<213> Mus musculus

<400> 34

caggtccagc tgcagcagcc tggggctgaa ctggtgaagc ctggggcttc agtgaaactg 60

tcctgcaagg cttctggata caccttcact agctactgga tgcattgggt gaagcagagg 120

cctggacaag gccttgagtg gatcggagag attgatcctt ctgatagtta tacttactac 180

aatcaaaagt tcaggggcaa ggccacattg actgtagaca aatcctccaa cacagcctac 240

atgcaactca gcagcctgac atctgaggac tctgcggtct attactgttc aagatcaaat 300

ctgggtgatg gtcactaccg gtttcctgcg tttccttact ggggccaagg gactctggtc 360

actgtctctg ca 372

<210> 35

<211> 372

<212> DNA

<213> Mus musculus

<400> 35

caggtccaac tgcagcagcc tggggctgaa ctggtgaaac ctggggcttc agtgaagctg 60

tcctgcaagg cttctggcta caccttcacc agctactgga tgcactgggt gaaacagagg 120

cctggacaag gccttgaatg gattggtaca attgaccctt ctgatagtga aactcactac 180

aatctacagt tcaaggacac ggccacattg actgtagaca aatcctccag cacagcctac 240

atgcagctca gcagcctgac atctgaggac tctgcggtct attattgtat aagaggcgcc 300

ttctatagtt cctatagtta ctgggcctgg tttgcttact ggggccaagg gactctggtc 360

actgtctctg ca 372

<210> 36

<211> 463

<212> PRT

<213> Mus musculus

<400> 36

Met Asn Phe Gly Leu Thr Leu Ile Phe Leu Val Leu Thr Leu Lys Gly
1 5 10 15

Val Gln Cys Glu Val Gln Leu Val Glu Ser Gly Gly Gly Leu Val Lys
20 25 30

Pro Gly Gly Ser Leu Lys Leu Ser Cys Ala Ala Ser Gly Phe Thr Phe
35 40 45

Ser Arg Tyr Ala Met Ser Trp Val Arg Gln Ile Pro Glu Lys Ile Leu
50 55 60

Glu Trp Val Ala Ala Ile Asp Ser Ser Gly Gly Asp Thr Tyr Tyr Leu
65 70 75 80

Asp Thr Val Lys Asp Arg Phe Thr Ile Ser Arg Asp Asn Ala Asn Asn
85 90 95

Thr Leu His Leu Gln Met Arg Ser Leu Arg Ser Glu Asp Thr Ala Leu
100 105 110

Tyr Tyr Cys Val Arg Gln Gly Gly Ala Tyr Trp Gly Gln Gly Thr Leu
115 120 125

Val Thr Val Ser Ala Ala Ser Thr Lys Gly Pro Ser Val Phe Pro Leu
130 135 140

Ala Pro Ser Ser Lys Ser Thr Ser Gly Gly Thr Ala Ala Leu Gly Cys
145 150 155 160

Leu Val Lys Asp Tyr Phe Pro Glu Pro Val Thr Val Ser Trp Asn Ser
165 170 175

Gly Ala Leu Thr Ser Gly Val His Thr Phe Pro Ala Val Leu Gln Ser
180 185 190

Ser Gly Leu Tyr Ser Leu Ser Ser Val Val Thr Val Pro Ser Ser Ser

195 200 205

Leu Gly Thr Gln Thr Tyr Ile Cys Asn Val Asn His Lys Pro Ser Asn
210 215 220

Thr Lys Val Asp Lys Lys Val Glu Pro Lys Ser Cys Asp Lys Thr His
225 230 235 240

Thr Cys Pro Pro Cys Pro Ala Pro Glu Leu Leu Gly Gly Pro Ser Val
245 250 255

Phe Leu Phe Pro Pro Lys Pro Lys Asp Thr Leu Met Ile Ser Arg Thr
260 265 270

Pro Glu Val Thr Cys Val Val Val Asp Val Ser His Glu Asp Pro Glu
275 280 285

Val Lys Phe Asn Trp Tyr Val Asp Gly Val Glu Val His Asn Ala Lys
290 295 300

Thr Lys Pro Arg Glu Glu Gln Tyr Asn Ser Thr Tyr Arg Val Val Ser
305 310 315 320

Val Leu Thr Val Leu His Gln Asp Trp Leu Asn Gly Lys Glu Tyr Lys
325 330 335

Cys Lys Val Ser Asn Lys Ala Leu Pro Ala Pro Ile Glu Lys Thr Ile
340 345 350

Ser Lys Ala Lys Gly Gln Pro Arg Glu Pro Gln Val Tyr Thr Leu Pro
355 360 365

Pro Ser Arg Asp Glu Leu Thr Lys Asn Gln Val Ser Leu Thr Cys Leu
370 375 380

Val Lys Gly Phe Tyr Pro Ser Asp Ile Ala Val Glu Trp Glu Ser Asn
385 390 395 400

Gly Gln Pro Glu Asn Asn Tyr Lys Thr Thr Pro Pro Val Leu Asp Ser
405 410 415

Asp Gly Ser Phe Phe Leu Tyr Ser Lys Leu Thr Val Asp Lys Ser Arg
420 425 430

Trp Gln Gln Gly Asn Val Phe Ser Cys Ser Val Met His Glu Ala Leu
435 440 445

His Asn His Tyr Thr Gln Lys Ser Leu Ser Leu Ser Pro Gly Lys
450 455 460

<210> 37
<211> 114
<212> PRT
<213> Mus musculus

<400> 37
Glu Val His Leu Val Glu Ser Gly Gly Gly Leu Val Lys Pro Gly Gly

1 5 10 15

Ser Leu Lys Leu Ser Cys Ala Ala Ser Gly Phe Thr Phe Ser Asn Tyr

20 25 30

Ala Met Ser Trp Val Arg Gln Thr Pro Glu Lys Arg Leu Glu Trp Val

35 40 45

Ala Ala Ile Asn Asn Asn Gly Asp Asp Thr Tyr Tyr Leu Asp Thr Val

50 55 60

Lys Asp Arg Phe Thr Ile Ser Arg Asp Asn Ala Lys Asn Thr Leu Tyr

65 70 75 80

Leu Gln Met Ser Ser Leu Arg Ser Glu Asp Thr Ala Leu Tyr Tyr Cys

85 90 95

Val Arg Gln Gly Gly Ala Tyr Trp Gly Gln Gly Thr Leu Val Thr Val

100 105 110

Ser Ala

<210> 38

<211> 470

<212> PRT

<213> Mus musculus

<400> 38

Met Gly Trp Asn Trp Ile Phe Ile Leu Ile Leu Ser Val Thr Thr Gly
1 5 10 15

Val His Ser Glu Val Gln Leu Gln Gln Ser Gly Pro Glu Leu Val Lys
20 25 30

Pro Gly Ala Ser Val Lys Ile Ser Cys Lys Ala Ser Gly Tyr Ser Phe
35 40 45

Thr Gly Tyr Tyr Met His Trp Val Lys Gln Ser Pro Glu Lys Ser Leu
50 55 60

Glu Trp Ile Gly Glu Ile Asn Pro Ser Thr Gly Gly Thr Thr Tyr Asn
65 70 75 80

Gln Lys Phe Lys Ala Lys Ala Thr Leu Thr Val Asp Lys Ser Ser Ser
85 90 95

Thr Ala Tyr Met Gln Leu Lys Ser Leu Thr Ser Glu Asp Ser Ala Val
100 105 110

Tyr Tyr Cys Ala Arg Arg Gly Gly Leu Thr Gly Thr Ser Phe Phe Ala
115 120 125

Tyr Trp Gly Gln Gly Thr Leu Val Thr Val Ser Ala Ala Ser Thr Lys
130 135 140

Gly Pro Ser Val Phe Pro Leu Ala Pro Ser Ser Lys Ser Thr Ser Gly

145 150 155 160

Gly Thr Ala Ala Leu Gly Cys Leu Val Lys Asp Tyr Phe Pro Glu Pro
165 170 175

Val Thr Val Ser Trp Asn Ser Gly Ala Leu Thr Ser Gly Val His Thr
180 185 190

Phe Pro Ala Val Leu Gln Ser Ser Gly Leu Tyr Ser Leu Ser Ser Val
195 200 205

Val Thr Val Pro Ser Ser Ser Leu Gly Thr Gln Thr Tyr Ile Cys Asn
210 215 220

Val Asn His Lys Pro Ser Asn Thr Lys Val Asp Lys Lys Val Glu Pro
225 230 235 240

Lys Ser Cys Asp Lys Thr His Thr Cys Pro Pro Cys Pro Ala Pro Glu
245 250 255

Leu Leu Gly Gly Pro Ser Val Phe Leu Phe Pro Pro Lys Pro Lys Asp
260 265 270

Thr Leu Met Ile Ser Arg Thr Pro Glu Val Thr Cys Val Val Val Asp
275 280 285

Val Ser His Glu Asp Pro Glu Val Lys Phe Asn Trp Tyr Val Asp Gly
290 295 300

Val Glu Val His Asn Ala Lys Thr Lys Pro Arg Glu Glu Gln Tyr Asn
305 310 315 320

Ser Thr Tyr Arg Val Val Ser Val Leu Thr Val Leu His Gln Asp Trp
325 330 335

Leu Asn Gly Lys Glu Tyr Lys Cys Lys Val Ser Asn Lys Ala Leu Pro
340 345 350

Ala Pro Ile Glu Lys Thr Ile Ser Lys Ala Lys Gly Gln Pro Arg Glu
355 360 365

Pro Gln Val Tyr Thr Leu Pro Pro Ser Arg Asp Glu Leu Thr Lys Asn
370 375 380

Gln Val Ser Leu Thr Cys Leu Val Lys Gly Phe Tyr Pro Ser Asp Ile
385 390 395 400

Ala Val Glu Trp Glu Ser Asn Gly Gln Pro Glu Asn Asn Tyr Lys Thr
405 410 415

Thr Pro Pro Val Leu Asp Ser Asp Gly Ser Phe Phe Leu Tyr Ser Lys
420 425 430

Leu Thr Val Asp Lys Ser Arg Trp Gln Gln Gly Asn Val Phe Ser Cys
435 440 445

Ser Val Met His Glu Ala Leu His Asn His Tyr Thr Gln Lys Ser Leu
450 455 460

Ser Leu Ser Pro Gly Lys
465 470

<210> 39
<211> 118
<212> PRT
<213> Mus musculus

<400> 39
Gln Val Thr Leu Lys Glu Ser Gly Pro Gly Ile Leu Gln Pro Ser Gln
1 5 10 15

Thr Leu Ser Leu Thr Cys Ser Phe Ser Gly Phe Ser Leu Ser Thr Tyr
20 25 30

Gly Met Gly Val Gly Trp Ile Arg Gln Pro Ser Gly Met Gly Leu Glu
35 40 45

Trp Leu Ala Asn Ile Trp Trp Tyr Asp Ala Lys Tyr Tyr Asn Ser Asp
50 55 60

Leu Lys Ser Arg Leu Thr Ile Ser Lys Asp Thr Ser Asn Asn Gln Val
65 70 75 80

Phe Leu Lys Ile Ser Ser Val Asp Thr Ser Asp Thr Ala Thr Tyr Tyr
85 90 95

Cys Ala Gln Met Gly Leu Ala Trp Phe Ala Tyr Trp Gly Gln Gly Thr
100 105 110

Leu Val Thr Val Ser Ala
115

<210> 40
<211> 118
<212> PRT
<213> Mus musculus

<400> 40
Gln Val Thr Leu Lys Glu Ser Gly Pro Gly Ile Leu Gln Pro Ser Gln
1 5 10 15

Thr Leu Ser Leu Thr Cys Ser Phe Ser Gly Phe Ser Leu Ser Ile Tyr
20 25 30

Gly Met Gly Val Gly Trp Ile Arg Gln Pro Ser Gly Lys Gly Leu Glu
35 40 45

Trp Leu Ala Asn Ile Trp Trp Asn Asp Asp Lys Tyr Tyr Asn Ser Ala
50 55 60

Leu Lys Ser Arg Leu Thr Ile Ser Lys Asp Thr Ser Asn Asn Gln Val
65 70 75 80

Phe Leu Lys Ile Ser Ser Val Asp Thr Ala Asp Thr Ala Thr Tyr Tyr

85 90 95

Cys Ala Gln Ile Gly Tyr Phe Tyr Phe Asp Tyr Trp Gly Gln Gly Thr
100 105 110

Thr Leu Thr Val Ser Ser
115

<210> 41
<211> 471
<212> PRT
<213> Mus musculus

<400> 41
Met Asn Phe Gly Leu Thr Leu Ile Phe Leu Val Leu Thr Leu Lys Gly
1 5 10 15

Val Gln Cys Glu Val Gln Leu Val Glu Ser Gly Gly Asp Leu Val Lys
20 25 30

Pro Gly Gly Thr Leu Lys Leu Ser Cys Ala Ala Ser Gly Ser Thr Phe
35 40 45

Ser Asn Tyr Ala Met Ser Trp Val Arg Gln Thr Pro Glu Lys Arg Leu
50 55 60

Glu Trp Val Ala Ala Ile Asp Ser Asn Gly Gly Thr Thr Tyr Tyr Pro
65 70 75 80

Asp Thr Met Lys Asp Arg Phe Thr Ile Ser Arg Asp Asn Ala Lys Asn
85 90 95

Thr Leu Tyr Leu Gln Met Asn Ser Leu Arg Ser Glu Asp Thr Ala Phe
100 105 110

Tyr His Cys Thr Arg His Asn Gly Gly Tyr Glu Asn Tyr Gly Trp Phe
115 120 125

Ala Tyr Trp Gly Gln Gly Thr Leu Val Thr Val Ser Ala Ala Ser Thr
130 135 140

Lys Gly Pro Ser Val Phe Pro Leu Ala Pro Ser Ser Lys Ser Thr Ser
145 150 155 160

Gly Gly Thr Ala Ala Leu Gly Cys Leu Val Lys Asp Tyr Phe Pro Glu
165 170 175

Pro Val Thr Val Ser Trp Asn Ser Gly Ala Leu Thr Ser Gly Val His
180 185 190

Thr Phe Pro Ala Val Leu Gln Ser Ser Gly Leu Tyr Ser Leu Ser Ser
195 200 205

Val Val Thr Val Pro Ser Ser Ser Leu Gly Thr Gln Thr Tyr Ile Cys
210 215 220

Asn Val Asn His Lys Pro Ser Asn Thr Lys Val Asp Lys Lys Val Glu

225 230 235 240

Pro Lys Ser Cys Asp Lys Thr His Thr Cys Pro Pro Cys Pro Ala Pro
245 250 255

Glu Leu Leu Gly Gly Pro Ser Val Phe Leu Phe Pro Pro Lys Pro Lys
260 265 270

Asp Thr Leu Met Ile Ser Arg Thr Pro Glu Val Thr Cys Val Val Val
275 280 285

Asp Val Ser His Glu Asp Pro Glu Val Lys Phe Asn Trp Tyr Val Asp
290 295 300

Gly Val Glu Val His Asn Ala Lys Thr Lys Pro Arg Glu Glu Gln Tyr
305 310 315 320

Asn Ser Thr Tyr Arg Val Val Ser Val Leu Thr Val Leu His Gln Asp
325 330 335

Trp Leu Asn Gly Lys Glu Tyr Lys Cys Lys Val Ser Asn Lys Ala Leu
340 345 350

Pro Ala Pro Ile Glu Lys Thr Ile Ser Lys Ala Lys Gly Gln Pro Arg
355 360 365

Glu Pro Gln Val Tyr Thr Leu Pro Pro Ser Arg Asp Glu Leu Thr Lys
370 375 380

Asn Gln Val Ser Leu Thr Cys Leu Val Lys Gly Phe Tyr Pro Ser Asp
385 390 395 400

Ile Ala Val Glu Trp Glu Ser Asn Gly Gln Pro Glu Asn Asn Tyr Lys
405 410 415

Thr Thr Pro Pro Val Leu Asp Ser Asp Gly Ser Phe Phe Leu Tyr Ser
420 425 430

Lys Leu Thr Val Asp Lys Ser Arg Trp Gln Gln Gly Asn Val Phe Ser
435 440 445

Cys Ser Val Met His Glu Ala Leu His Asn His Tyr Thr Gln Lys Ser
450 455 460

Leu Ser Leu Ser Pro Gly Lys
465 470

<210> 42
<211> 122
<212> PRT
<213> Mus musculus

<400> 42
Glu Val Gln Leu Val Glu Ser Gly Gly Asp Leu Val Lys Pro Gly Gly
1 5 10 15

Ser Leu Lys Leu Ser Cys Ala Ala Ser Gly Phe Thr Phe Ser Ser Tyr

20 25 30

Ala Met Ser Trp Val Arg Gln Thr Pro Glu Lys Arg Leu Glu Trp Val

35 40 45

Ala Ala Ile Asn Ser Asn Gly Gly Thr Thr Tyr Tyr Pro Asp Thr Met

50 55 60

Lys Asp Arg Phe Thr Ile Ser Arg Asp Asn Ala Lys Asn Thr Leu Tyr

65 70 75 80

Leu Gln Met Ser Ser Leu Arg Ser Glu Asp Ser Ala Leu Tyr Tyr Cys

85 90 95

Thr Arg His Asn Gly Gly Tyr Glu Asn Tyr Gly Trp Phe Ala Tyr Trp

100 105 110

Gly Gln Gly Thr Leu Val Thr Val Ser Ala

115 120

<210> 43

<211> 470

<212> PRT

<213> Mus musculus

<400> 43

Met Glu Ser Asn Trp Ile Leu Pro Phe Ile Leu Ser Val Ala Ser Gly

1 5 10 15

Val Tyr Ser Glu Val Gln Leu Gln Gln Ser Gly Thr Val Leu Ala Arg
20 25 30

Pro Gly Ala Ser Val Lys Met Ser Cys Lys Ala Ser Gly Tyr Thr Phe
35 40 45

Thr Gly Tyr Trp Met Arg Trp Val Lys Gln Arg Pro Gly Gln Gly Leu
50 55 60

Glu Trp Ile Gly Ala Ile Tyr Pro Gly Asn Ser Asp Thr Thr Tyr Asn
65 70 75 80

Gln Lys Phe Lys Gly Lys Ala Lys Leu Thr Ala Val Thr Ser Val Ser
85 90 95

Thr Ala Tyr Met Glu Leu Ser Ser Leu Thr Asn Glu Asp Ser Ala Val
100 105 110

Tyr Tyr Cys Ser Arg Ser Gly Asp Leu Thr Gly Gly Phe Ala Tyr Trp
115 120 125

Gly Gln Gly Thr Leu Val Thr Val Ser Thr Ala Lys Ala Ser Thr Lys
130 135 140

Gly Pro Ser Val Phe Pro Leu Ala Pro Ser Ser Lys Ser Thr Ser Gly
145 150 155 160

Gly Thr Ala Ala Leu Gly Cys Leu Val Lys Asp Tyr Phe Pro Glu Pro

165 170 175

Val Thr Val Ser Trp Asn Ser Gly Ala Leu Thr Ser Gly Val His Thr
180 185 190

Phe Pro Ala Val Leu Gln Ser Ser Gly Leu Tyr Ser Leu Ser Ser Val
195 200 205

Val Thr Val Pro Ser Ser Ser Leu Gly Thr Gln Thr Tyr Ile Cys Asn
210 215 220

Val Asn His Lys Pro Ser Asn Thr Lys Val Asp Lys Lys Val Glu Pro
225 230 235 240

Lys Ser Cys Asp Lys Thr His Thr Cys Pro Pro Cys Pro Ala Pro Glu
245 250 255

Leu Leu Gly Gly Pro Ser Val Phe Leu Phe Pro Pro Lys Pro Lys Asp
260 265 270

Thr Leu Met Ile Ser Arg Thr Pro Glu Val Thr Cys Val Val Val Asp
275 280 285

Val Ser His Glu Asp Pro Glu Val Lys Phe Asn Trp Tyr Val Asp Gly
290 295 300

Val Glu Val His Asn Ala Lys Thr Lys Pro Arg Glu Glu Gln Tyr Asn
305 310 315 320

Ser Thr Tyr Arg Val Val Ser Val Leu Thr Val Leu His Gln Asp Trp
325 330 335

Leu Asn Gly Lys Glu Tyr Lys Cys Lys Val Ser Asn Lys Ala Leu Pro
340 345 350

Ala Pro Ile Glu Lys Thr Ile Ser Lys Ala Lys Gly Gln Pro Arg Glu
355 360 365

Pro Gln Val Tyr Thr Leu Pro Pro Ser Arg Asp Glu Leu Thr Lys Asn
370 375 380

Gln Val Ser Leu Thr Cys Leu Val Lys Gly Phe Tyr Pro Ser Asp Ile
385 390 395 400

Ala Val Glu Trp Glu Ser Asn Gly Gln Pro Glu Asn Asn Tyr Lys Thr
405 410 415

Thr Pro Pro Val Leu Asp Ser Asp Gly Ser Phe Phe Leu Tyr Ser Lys
420 425 430

Leu Thr Val Asp Lys Ser Arg Trp Gln Gln Gly Asn Val Phe Ser Cys
435 440 445

Ser Val Met His Glu Ala Leu His Asn His Tyr Thr Gln Lys Ser Leu
450 455 460

Ser Leu Ser Pro Gly Lys
465 470

<210> 44

<211> 119

<212> PRT

<213> Mus musculus

<400> 44

Glu Val Gln Leu Gln Gln Ser Gly Thr Val Leu Ala Arg Pro Gly Ala
1 5 10 15

Ser Val Lys Met Ser Cys Lys Ala Ser Gly Tyr Thr Phe Thr Gly Tyr
20 25 30

Trp Met His Trp Val Lys Gln Arg Pro Gly Gln Gly Leu Glu Trp Ile
35 40 45

Gly Ala Ile Tyr Pro Gly Asn Ser Asp Thr Asn Tyr Asn Gln Lys Phe
50 55 60

Lys Gly Lys Ala Lys Leu Thr Ala Val Thr Ser Ala Ser Thr Ala Tyr
65 70 75 80

Met Glu Leu Ser Ser Leu Thr Asn Glu Asp Ala Ala Val Tyr His Cys
85 90 95

Thr Arg Ser Gly Asp Leu Thr Gly Gly Leu Ala Tyr Trp Gly Gln Gly
100 105 110

Thr Leu Val Thr Val Ser Ala
115

<210> 45
<211> 124
<212> PRT
<213> Mus musculus

<400> 45
Gln Val Gln Leu Gln Gln Pro Gly Ala Glu Leu Val Lys Pro Gly Ala
1 5 10 15

Ser Val Lys Leu Ser Cys Lys Ala Ser Gly Tyr Thr Phe Thr Ser Tyr
20 25 30

Trp Met His Trp Val Lys Gln Arg Pro Gly Gln Gly Leu Glu Trp Ile
35 40 45

Gly Glu Ile Asp Pro Ser Asp Ser Tyr Thr Tyr Tyr Asn Gln Lys Phe
50 55 60

Arg Gly Lys Ala Thr Leu Thr Val Asp Lys Ser Ser Asn Thr Ala Tyr
65 70 75 80

Met Gln Leu Ser Ser Leu Thr Ser Glu Asp Ser Ala Val Tyr Tyr Cys
85 90 95

Ser Arg Ser Asn Leu Gly Asp Gly His Tyr Arg Phe Pro Ala Phe Pro

100 105 110

Tyr Trp Gly Gln Gly Thr Leu Val Thr Val Ser Ala

115 120

<210> 46

<211> 124

<212> PRT

<213> Mus musculus

<400> 46

Gln Val Gln Leu Gln Gln Pro Gly Ala Glu Leu Val Lys Pro Gly Ala

1 5 10 15

Ser Val Lys Leu Ser Cys Lys Ala Ser Gly Tyr Thr Phe Thr Ser Tyr

20 25 30

Trp Met His Trp Val Lys Gln Arg Pro Gly Gln Gly Leu Glu Trp Ile

35 40 45

Gly Thr Ile Asp Pro Ser Asp Ser Glu Thr His Tyr Asn Leu Gln Phe

50 55 60

Lys Asp Thr Ala Thr Leu Thr Val Asp Lys Ser Ser Ser Thr Ala Tyr

65 70 75 80

Met Gln Leu Ser Ser Leu Thr Ser Glu Asp Ser Ala Val Tyr Tyr Cys

85 90 95

Ile Arg Gly Ala Phe Tyr Ser Ser Tyr Ser Tyr Trp Ala Trp Phe Ala
100 105 110

Tyr Trp Gly Gln Gly Thr Leu Val Thr Val Ser Ala
115 120

<210> 47
<211> 717
<212> DNA
<213> Mus musculus

<400> 47
atgagtcctg cccagttcct gtttctgtta gtgctctgga ttcgggaaac caacggtgat 60
gttgtgatga cccagactcc actcactttg tcggttacca ttggacaacc agcctccatc 120
tcttgcaagt caagtcagag cctcttagat agtgatggaa agacatattt gaattggttg 180
ttacagaggc caggccagtc tccaaagcgc ctaatctatc tggtgtctaa attggactct 240
ggagcccctg acaggttcac tggcagtgga tcagggacag atttcacact gaaaatcagt 300
agagtggagg ctgaggattt gggaatttat tattgctggc aaggtacaca ttttccgctc 360
acgttcggtg ctgggaccaa gctggagctg aaacgtacgg tggctgcacc atctgtcttc 420
atcttcccgc catctgatga gcagttgaaa tctggaactg cctctgttgt gtgcctgctg 480
aataacttct atcccagaga ggccaaagta cagtggaagg tggataacgc cctccaatcg 540
ggtaactccc aggagagtgt cacagagcag gacagcaagg acagcaccta cagcctcagc 600
agcaccctga cgctgagcaa agcagactac gagaaacaca aagtctacgc ctgcgaagtc 660
acccatcagg gcctgagctc gcccgtcaca aagagcttca acaggggaga gtgttga 717

<210> 48

<211> 336

<212> DNA

<213> Mus musculus

<400> 48

gatgttgtga tgacccagtc tccactcact ttgtcgatta ccattggaca accagcctcc 60

atctcttgca agtcaagtca gagcctctta gatagtgatg gaaagacata tttgaattgg 120

ttgttacaga ggccaggcca gtctccaaag cgcctaatct atctggtgtc taaactggac 180

tctggagtcc ctgacaggtt cactggcagt ggatcaggga cagatttctc actgaaaatc 240

agcagagtgg aggctgagga tttgggaatt tattattgct ggcaaggtac acattttccg 300

ctcacgttcg gtgctgggac caagctggag ctgaaa 336

<210> 49

<211> 717

<212> DNA

<213> Mus musculus

<400> 49

atgagtcctg tccagttcct gtttctgtta atgctctgga ttcaggaaac caacggtgat 60

gttgtgatga cccagactcc actgtctttg tcggttacca ttggacaacc agcctctatc 120

tcttgcaagt caagtcagag cctcttatat agtaatggaa agacatattt gaattggtta 180

caacagaggc ctggccaggc tccaaagcac ctaatgtatc aggtgtccaa actggaccct 240

ggcatccctg acaggttcag tggcagtgga tcagaaacag attttacact taaaatcagc 300

agagtggagg ctgaagattt gggagtttat tactgcttgc aaagtacata ttatccgctc 360

acgttcggtg ctgggaccaa gctggagctg aaacgtacgg tggctgcacc atctgtcttc 420

atcttcccgc catctgatga gcagttgaaa tctggaactg cctctgttgt gtgcctgctg 480

aataacttct atcccagaga ggccaaagta cagtggaagg tggataacgc cctccaatcg 540

ggtaactccc aggagagtgt cacagagcag gacagcaagg acagcaccta cagcctcagc 600

agcaccctga cgctgagcaa agcagactac gagaaacaca aagtctacgc ctgcgaagtc 660

acccatcagg gcctgagctc gcccgtcaca aagagcttca acaggggaga gtgttga 717

<210> 50

<211> 324

<212> DNA

<213> Mus musculus

<400> 50

gacatcaaga tgacccagtc tccatcttcc atgtatgcat ctctaggaga gagagtcact 60

atcacttgca aggcgagtca ggacattaat aactatttaa gctggttcca gcagaaacca 120

gggaaatctc ctaagaccct gatctatcgt gcaaacagat tggtagatgg ggtcccatca 180

aggttcagtg gcagtggatc tgggcaagat tattctctca ccatcagcag cctggagtat 240

gaagatatgg gaattaatta ttgtctacag tgtgatgagt ttcctccgtg gacgttcggt 300

ggaggcacca agctggaaat caaa 324

<210> 51

<211> 336

<212> DNA

<213> Mus musculus

<400> 51

gatgttgtga tgacccaaac tccactctcc ctgcctgtca gtcttggaga tcaagcctcc 60

atctcttgca gatctagtca gagccttgta cacagtaatg gaaacaccta tttacattgg 120

tacctgcaga agccaggcca gtctccaaag ctcctgatct acaaagtttc caaccgattt 180

tctggggtcc cagacaggtt cagtggcagt ggatcaggga cagatttcac actcaagatc 240

agcagagtgg aggctgagga tctgggagtt tatttctgct ctcaaagtac acatgttccg 300

tggacgttcg gtggaggcac caagctggaa atcaaa 336

<210> 52

<211> 705

<212> DNA

<213> Mus musculus

<400> 52

atgagaccct ccattcagtt cctggggctc ttgttgttct ggcttcatgg tgttcagtgt 60

gacatccaga tgacacagtc tccatcctca ctgtctgcat ctctgggagg caaagtcacc 120

atcacttgca aggcaagtca ggacattaac aagaatatag tttggtacca acacaagcct 180

ggaaaaggtc ctaggctgct catatggtac acatctacat tacagccagg catcccatca 240

aggttcagtg gaagtgggtc tgggagagat tattccttca gcatcagcaa cctggagcct 300

gaagatattg caacttatta ctgtctacag tatgataatc ttccacggac gttcggtgga 360

ggcaccaaac tggaaatcaa acgtacggtg gctgcaccat ctgtcttcat cttcccgcca 420

tctgatgagc agttgaaatc tggaactgcc tctgttgtgt gcctgctgaa taacttctat 480

cccagagagg ccaaagtaca gtggaaggtg gataacgccc tccaatcggg taactcccag 540

gagagtgtca cagagcagga cagcaaggac agcacctaca gcctcagcag caccctgacg 600

ctgagcaaag cagactacga gaaacacaaa gtctacgcct gcgaagtcac ccatcagggc 660

ctgagctcgc ccgtcacaaa gagcttcaac aggggagagt gttga 705

<210> 53

<211> 321

<212> DNA

<213> Mus musculus

<400> 53

gacatccaga tgacacagtc tccatcctca ctgtctgcat ctctgggagg caaagtcacc 60
atcacttgca aggcaagtca ggacattaac aagaatataa tttggtacca acacaagcct 120
ggaaaaggtc ctaggctgct catatggtac acatctacat tacagccagg catcccatca 180
aggttcagtg gaagtgggtc tgggagagat tattccttca gcatcagcaa cctggagcct 240
gaagatattg caacttatta ctgtctacag tatgataatc ttccacggac gttcggtgga 300
ggcaccaagc tggaaatcaa a 321

<210> 54
<211> 720
<212> DNA
<213> Mus musculus

<400> 54
atgaggttct ctgctcagct tctggggctg cttgtgctct ggatccctgg atccactgca 60
gatattgtga tgacgcaggc tgcattctcc aatccagtca ctcttggaac atcaacttcc 120
atctcctgca ggtctagtaa gagtctccta catagtaatg gcatcactta tttgtattgg 180
tatctgcaga agccaggcca gtctcctcag ctcctgattt atcagatgtc caaccttgcc 240
tcaggagtcc cagacaggtt cagtagcagt gggtcaggaa ctgatttcac actgagaatc 300
agcagagtgg aggctgagga tgtgggtgtt tattactgtg ctcaaaatct agaacttccg 360
tatacgttcg gatcggggac caagctggaa ataaaacgta cggtggctgc accatctgtc 420
ttcatcttcc cgccatctga tgagcagttg aaatctggaa ctgcctctgt tgtgtgcctg 480
ctgaataact tctatcccag agaggccaaa gtacagtgga aggtggataa cgccctccaa 540
tcgggtaact cccaggagag tgtcacagag caggacagca aggacagcac ctacagcctc 600
agcagcaccc tgacgctgag caaagcagac tacgagaaac acaaagtcta cgcctgcgaa 660
gtcacccatc agggcctgag ctcgcccgtc acaaagagct tcaacagggg agagtgttga 720

<210> 55

<211> 336

<212> DNA

<213> Mus musculus

<400> 55

gatattgtga tgacgcaggc tgcattctcc aatccagtca ctcttggaac atcagcttcc 60

atctcctgca ggtctagtaa gagtctccta catagtaatg gcatcactta tttgtattgg 120

tttctgcaga agccaggcca gtctcctcag ctcctgattt atcagatgtc caaccttgcc 180

tcaggagtcc cagacaggtt cagtagcagt gggtcaggaa ctgatttcac actgagaatc 240

agcagagtgg aggctgagga tgtgggtgtt tattactgtg ctcaaaatct agaacttccg 300

tatacgttcg gatcggggac caagctggaa ataaaa 336

<210> 56

<211> 321

<212> DNA

<213> Mus musculus

<400> 56

gatattgtgc taactcagtc tccagccacc ctgtctgtga ctccaggaga cagagtcagt 60

ctttcctgca gggccagcca tagtattagc aacttcctac actggtatcc acaaaaatca 120

catgagtctc caaggcttct catcaagtat gcttcccagt ccatctctgg gatcccctcc 180

aggttcagtg gcaatggatc agggacagat ttcactctca gtatcaacag tgtggagact 240

gaagattttg gaatgtattt ctgtcaacag agtaacatct ggtcgctcac gttcggtgct 300

gggaccaagc tggagctgaa a 321

<210> 57

<211> 333

<212> DNA

<213> Mus musculus

<400> 57

gacattgtgc tcacccaatc tccaacttct ttggctgtgt ctctagggca gagtgtcacc 60

atctcctgca gagccagtga aagtgttgaa tattatggca ctagtttaat gcagtggtac 120

caacagaaac caggacagcc acccaaactc ctcatctatg gtgcatccaa cgtagaatct 180

ggggtccctg ccaggtttag tggcagtggg tctgggacag acttcagcct caacatccat 240

cctgtggagg aggatgatat tgcaatgtat ttctgtcagc aaagtaggaa ggttccgtat 300

acgttcggat cggggaccaa gctggaaata aaa 333

<210> 58

<211> 238

<212> PRT

<213> Mus musculus

<400> 58

Met Ser Pro Ala Gln Phe Leu Phe Leu Leu Val Leu Trp Ile Arg Glu
1 5 10 15

Thr Asn Gly Asp Val Val Met Thr Gln Thr Pro Leu Thr Leu Ser Val
20 25 30

Thr Ile Gly Gln Pro Ala Ser Ile Ser Cys Lys Ser Ser Gln Ser Leu
35 40 45

Leu Asp Ser Asp Gly Lys Thr Tyr Leu Asn Trp Leu Leu Gln Arg Pro
50 55 60

Gly Gln Ser Pro Lys Arg Leu Ile Tyr Leu Val Ser Lys Leu Asp Ser
65 70 75 80

Gly Ala Pro Asp Arg Phe Thr Gly Ser Gly Ser Gly Thr Asp Phe Thr
85 90 95

Leu Lys Ile Ser Arg Val Glu Ala Glu Asp Leu Gly Ile Tyr Tyr Cys
100 105 110

Trp Gln Gly Thr His Phe Pro Leu Thr Phe Gly Ala Gly Thr Lys Leu
115 120 125

Glu Leu Lys Arg Thr Val Ala Ala Pro Ser Val Phe Ile Phe Pro Pro
130 135 140

Ser Asp Glu Gln Leu Lys Ser Gly Thr Ala Ser Val Val Cys Leu Leu
145 150 155 160

Asn Asn Phe Tyr Pro Arg Glu Ala Lys Val Gln Trp Lys Val Asp Asn
165 170 175

Ala Leu Gln Ser Gly Asn Ser Gln Glu Ser Val Thr Glu Gln Asp Ser
180 185 190

Lys Asp Ser Thr Tyr Ser Leu Ser Ser Thr Leu Thr Leu Ser Lys Ala
195 200 205

Asp Tyr Glu Lys His Lys Val Tyr Ala Cys Glu Val Thr His Gln Gly

210 215 220

Leu Ser Ser Pro Val Thr Lys Ser Phe Asn Arg Gly Glu Cys
225 230 235

<210> 59
<211> 112
<212> PRT
<213> Mus musculus

<400> 59
Asp Val Val Met Thr Gln Ser Pro Leu Thr Leu Ser Ile Thr Ile Gly
1 5 10 15

Gln Pro Ala Ser Ile Ser Cys Lys Ser Ser Gln Ser Leu Leu Asp Ser
20 25 30

Asp Gly Lys Thr Tyr Leu Asn Trp Leu Leu Gln Arg Pro Gly Gln Ser
35 40 45

Pro Lys Arg Leu Ile Tyr Leu Val Ser Lys Leu Asp Ser Gly Val Pro
50 55 60

Asp Arg Phe Thr Gly Ser Gly Ser Gly Thr Asp Phe Ser Leu Lys Ile
65 70 75 80

Ser Arg Val Glu Ala Glu Asp Leu Gly Ile Tyr Tyr Cys Trp Gln Gly
85 90 95

Thr His Phe Pro Leu Thr Phe Gly Ala Gly Thr Lys Leu Glu Leu Lys
100 105 110

<210> 60
<211> 238
<212> PRT
<213> Mus musculus

<400> 60
Met Ser Pro Val Gln Phe Leu Phe Leu Leu Met Leu Trp Ile Gln Glu
1 5 10 15

Thr Asn Gly Asp Val Val Met Thr Gln Thr Pro Leu Ser Leu Ser Val
20 25 30

Thr Ile Gly Gln Pro Ala Ser Ile Ser Cys Lys Ser Ser Gln Ser Leu
35 40 45

Leu Tyr Ser Asn Gly Lys Thr Tyr Leu Asn Trp Leu Gln Gln Arg Pro
50 55 60

Gly Gln Ala Pro Lys His Leu Met Tyr Gln Val Ser Lys Leu Asp Pro
65 70 75 80

Gly Ile Pro Asp Arg Phe Ser Gly Ser Gly Ser Glu Thr Asp Phe Thr
85 90 95

Leu Lys Ile Ser Arg Val Glu Ala Glu Asp Leu Gly Val Tyr Tyr Cys
100 105 110

Leu Gln Ser Thr Tyr Tyr Pro Leu Thr Phe Gly Ala Gly Thr Lys Leu
115 120 125

Glu Leu Lys Arg Thr Val Ala Ala Pro Ser Val Phe Ile Phe Pro Pro
130 135 140

Ser Asp Glu Gln Leu Lys Ser Gly Thr Ala Ser Val Val Cys Leu Leu
145 150 155 160

Asn Asn Phe Tyr Pro Arg Glu Ala Lys Val Gln Trp Lys Val Asp Asn
165 170 175

Ala Leu Gln Ser Gly Asn Ser Gln Glu Ser Val Thr Glu Gln Asp Ser
180 185 190

Lys Asp Ser Thr Tyr Ser Leu Ser Ser Thr Leu Thr Leu Ser Lys Ala
195 200 205

Asp Tyr Glu Lys His Lys Val Tyr Ala Cys Glu Val Thr His Gln Gly
210 215 220

Leu Ser Ser Pro Val Thr Lys Ser Phe Asn Arg Gly Glu Cys
225 230 235

<210> 61

<211> 108

<212> PRT

<213> Mus musculus

<400> 61

Asp Ile Lys Met Thr Gln Ser Pro Ser Ser Met Tyr Ala Ser Leu Gly
1 5 10 15

Glu Arg Val Thr Ile Thr Cys Lys Ala Ser Gln Asp Ile Asn Asn Tyr
20 25 30

Leu Ser Trp Phe Gln Gln Lys Pro Gly Lys Ser Pro Lys Thr Leu Ile
35 40 45

Tyr Arg Ala Asn Arg Leu Val Asp Gly Val Pro Ser Arg Phe Ser Gly
50 55 60

Ser Gly Ser Gly Gln Asp Tyr Ser Leu Thr Ile Ser Ser Leu Glu Tyr
65 70 75 80

Glu Asp Met Gly Ile Asn Tyr Cys Leu Gln Cys Asp Glu Phe Pro Pro
85 90 95

Trp Thr Phe Gly Gly Gly Thr Lys Leu Glu Ile Lys
100 105

<210> 62

<211> 112

<212> PRT

<213> Mus musculus

<400> 62

Asp Val Val Met Thr Gln Thr Pro Leu Ser Leu Pro Val Ser Leu Gly
1               5                   10                  15

Asp Gln Ala Ser Ile Ser Cys Arg Ser Ser Gln Ser Leu Val His Ser
            20                  25                  30

Asn Gly Asn Thr Tyr Leu His Trp Tyr Leu Gln Lys Pro Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Phe Cys Ser Gln Ser
                85                  90                  95

Thr His Val Pro Trp Thr Phe Gly Gly Gly Thr Lys Leu Glu Ile Lys
            100                 105                 110

<210> 63

<211> 234

<212> PRT

<213> Mus musculus

<400> 63

Met Arg Pro Ser Ile Gln Phe Leu Gly Leu Leu Leu Phe Trp Leu His
1               5                   10                  15

Gly Val Gln Cys Asp Ile Gln Met Thr Gln Ser Pro Ser Ser Leu Ser
            20                  25                  30

Ala Ser Leu Gly Gly Lys Val Thr Ile Thr Cys Lys Ala Ser Gln Asp
        35                  40                  45

Ile Asn Lys Asn Ile Val Trp Tyr Gln His Lys Pro Gly Lys Gly Pro
    50                  55                  60

Arg Leu Leu Ile Trp Tyr Thr Ser Thr Leu Gln Pro Gly Ile Pro Ser
65                  70                  75                  80

Arg Phe Ser Gly Ser Gly Ser Gly Arg Asp Tyr Ser Phe Ser Ile Ser
                85                  90                  95

Asn Leu Glu Pro Glu Asp Ile Ala Thr Tyr Tyr Cys Leu Gln Tyr Asp
            100                 105                 110

Asn Leu Pro Arg Thr Phe Gly Gly Gly Thr Lys Leu Glu Ile Lys Arg
115 120 125

Thr Val Ala Ala Pro Ser Val Phe Ile Phe Pro Pro Ser Asp Glu Gln
130 135 140

Leu Lys Ser Gly Thr Ala Ser Val Val Cys Leu Leu Asn Asn Phe Tyr
145 150 155 160

Pro Arg Glu Ala Lys Val Gln Trp Lys Val Asp Asn Ala Leu Gln Ser
165 170 175

Gly Asn Ser Gln Glu Ser Val Thr Glu Gln Asp Ser Lys Asp Ser Thr
180 185 190

Tyr Ser Leu Ser Ser Thr Leu Thr Leu Ser Lys Ala Asp Tyr Glu Lys
195 200 205

His Lys Val Tyr Ala Cys Glu Val Thr His Gln Gly Leu Ser Ser Pro
210 215 220

Val Thr Lys Ser Phe Asn Arg Gly Glu Cys
225 230

<210> 64

<211> 107

<212> PRT

<213> Mus musculus

<400> 64

Asp Ile Gln Met Thr Gln Ser Pro Ser Ser Leu Ser Ala Ser Leu Gly
1 5 10 15

Gly Lys Val Thr Ile Thr Cys Lys Ala Ser Gln Asp Ile Asn Lys Asn
20 25 30

Ile Ile Trp Tyr Gln His Lys Pro Gly Lys Gly Pro Arg Leu Leu Ile
35 40 45

Trp Tyr Thr Ser Thr Leu Gln Pro Gly Ile Pro Ser Arg Phe Ser Gly
50 55 60

Ser Gly Ser Gly Arg Asp Tyr Ser Phe Ser Ile Ser Asn Leu Glu Pro
65 70 75 80

Glu Asp Ile Ala Thr Tyr Tyr Cys Leu Gln Tyr Asp Asn Leu Pro Arg
85 90 95

Thr Phe Gly Gly Gly Thr Lys Leu Glu Ile Lys
100 105

<210> 65

<211> 239

<212> PRT

<213> Mus musculus

<400> 65

Met Arg Phe Ser Ala Gln Leu Leu Gly Leu Leu Val Leu Trp Ile Pro
1 5 10 15

Gly Ser Thr Ala Asp Ile Val Met Thr Gln Ala Ala Phe Ser Asn Pro
20 25 30

Val Thr Leu Gly Thr Ser Thr Ser Ile Ser Cys Arg Ser Ser Lys Ser
35 40 45

Leu Leu His Ser Asn Gly Ile Thr Tyr Leu Tyr Trp Tyr Leu Gln Lys
50 55 60

Pro Gly Gln Ser Pro Gln Leu Leu Ile Tyr Gln Met Ser Asn Leu Ala
65 70 75 80

Ser Gly Val Pro Asp Arg Phe Ser Ser Ser Gly Ser Gly Thr Asp Phe
85 90 95

Thr Leu Arg Ile Ser Arg Val Glu Ala Glu Asp Val Gly Val Tyr Tyr
100 105 110

Cys Ala Gln Asn Leu Glu Leu Pro Tyr Thr Phe Gly Ser Gly Thr Lys
115 120 125

Leu Glu Ile Lys Arg Thr Val Ala Ala Pro Ser Val Phe Ile Phe Pro
130 135 140

Pro Ser Asp Glu Gln Leu Lys Ser Gly Thr Ala Ser Val Val Cys Leu
145 150 155 160

Leu Asn Asn Phe Tyr Pro Arg Glu Ala Lys Val Gln Trp Lys Val Asp
165 170 175

Asn Ala Leu Gln Ser Gly Asn Ser Gln Glu Ser Val Thr Glu Gln Asp
180 185 190

Ser Lys Asp Ser Thr Tyr Ser Leu Ser Ser Thr Leu Thr Leu Ser Lys
195 200 205

Ala Asp Tyr Glu Lys His Lys Val Tyr Ala Cys Glu Val Thr His Gln
210 215 220

Gly Leu Ser Ser Pro Val Thr Lys Ser Phe Asn Arg Gly Glu Cys
225 230 235

<210> 66
<211> 112
<212> PRT
<213> Mus musculus

<400> 66
Asp Ile Val Met Thr Gln Ala Ala Phe Ser Asn Pro Val Thr Leu Gly
1 5 10 15

Thr Ser Ala Ser Ile Ser Cys Arg Ser Ser Lys Ser Leu Leu His Ser

20 25 30

Asn Gly Ile Thr Tyr Leu Tyr Trp Phe Leu Gln Lys Pro Gly Gln Ser
35 40 45

Pro Gln Leu Leu Ile Tyr Gln Met Ser Asn Leu Ala Ser Gly Val Pro
50 55 60

Asp Arg Phe Ser Ser Ser Gly Ser Gly Thr Asp Phe Thr Leu Arg Ile
65 70 75 80

Ser Arg Val Glu Ala Glu Asp Val Gly Val Tyr Tyr Cys Ala Gln Asn
85 90 95

Leu Glu Leu Pro Tyr Thr Phe Gly Ser Gly Thr Lys Leu Glu Ile Lys
100 105 110

<210> 67

<211> 107

<212> PRT

<213> Mus musculus

<400> 67

Asp Ile Val Leu Thr Gln Ser Pro Ala Thr Leu Ser Val Thr Pro Gly
1 5 10 15

Asp Arg Val Ser Leu Ser Cys Arg Ala Ser His Ser Ile Ser Asn Phe
20 25 30

Leu His Trp Tyr Pro Gln Lys Ser His Glu Ser Pro Arg Leu Leu Ile
35 40 45

Lys Tyr Ala Ser Gln Ser Ile Ser Gly Ile Pro Ser Arg Phe Ser Gly
50 55 60

Asn Gly Ser Gly Thr Asp Phe Thr Leu Ser Ile Asn Ser Val Glu Thr
65 70 75 80

Glu Asp Phe Gly Met Tyr Phe Cys Gln Gln Ser Asn Ile Trp Ser Leu
85 90 95

Thr Phe Gly Ala Gly Thr Lys Leu Glu Leu Lys
100 105

<210> 68
<211> 111
<212> PRT
<213> Mus musculus

<400> 68
Asp Ile Val Leu Thr Gln Ser Pro Thr Ser Leu Ala Val Ser Leu Gly
1 5 10 15

Gln Ser Val Thr Ile Ser Cys Arg Ala Ser Glu Ser Val Glu Tyr Tyr
20 25 30

Gly Thr Ser Leu Met Gln Trp Tyr Gln Gln Lys Pro Gly Gln Pro Pro
35 40 45

Lys Leu Leu Ile Tyr Gly Ala Ser Asn Val Glu Ser Gly Val Pro Ala
50 55 60

Arg Phe Ser Gly Ser Gly Ser Gly Thr Asp Phe Ser Leu Asn Ile His
65 70 75 80

Pro Val Glu Glu Asp Asp Ile Ala Met Tyr Phe Cys Gln Gln Ser Arg
85 90 95

Lys Val Pro Tyr Thr Phe Gly Ser Gly Thr Lys Leu Glu Ile Lys
100 105 110

<210> 69
<211> 333
<212> DNA
<213> Mus musculus

<400> 69
cagatccagt tggagcagtc tggacctgag ctgaagaagc ctggagagac agtcaagatc 60
tcctgcaagg cttctggtta tattttcaga gactattcaa tgcactgggt gaagcaggct 120
ccaggaaagg gtttaaagtg gatgggctgg ataaacactg agacgggtga gccaacatat 180
gcagatgact tcaagggacg gtttgccttc tctttggaaa cctctgccag cactgcctat 240

ttgcagatca acaacctcaa aaatgaggac acggctacat atttctgtac tagcctttac 300

tggggccaag ggactctggt cactgtctct gca 333

<210> 70

<211> 372

<212> DNA

<213> Mus musculus

<400> 70

caggtcactc tgaaagagtc tggccctggg atattgcagc cctcccagac cctcagtctg 60

acttgttctt tctctgggtt ttcactgagc acttatggta tgggtgtagg ttggattcgt 120

cagccttcag ggaagggtct ggagtggctg gccaacattt ggtggcatga tgataagtac 180

tataactcag ccctgaagag ccggctcaca atctccaagg atatctccaa caaccaggta 240

ttcctcaaga tctccagtgt ggacactgca gatactgcca catactactg tgctcaaata 300

gcccctcgat ataataagta cgaaggcttt tttgctttct ggggccaagg gactctggtc 360

actgtctctg ca 372

<210> 71

<211> 345

<212> DNA

<213> Mus musculus

<400> 71

caggttcaac tgcagcagtc tggggctgag ctggtgaggc ctggggcttc agtgaagctg 60

tcctgcaagg cttcgggcta cacatttact gactatgaaa tgcactgggt gaagcagaca 120

cctgtgcatg gcctaaaatg gattggagct cttgatccta aaactggtga tactgcctac 180

agtcagaagt tcaagggcaa ggccacactg actgcagaca aatcctccag cacagcctac 240

atggagctcc gcagcctgac atctgaggac tctgccgtct attactgtac aagattctac 300

tcctatactt actggggcca agggactctg gtcactgtct ctgca 345

<210> 72

<211> 357

<212> DNA

<213> Mus musculus

<400> 72

gaggtgcagc ttgttgagac tggtggagga ctggtgcagc ctgaagggtc attgaaactc 60

tcatgtgcag cttctggatt cagcttcaat atcaatgcca tgaactgggt ccgccaggct 120

ccaggaaagg gtttggaatg ggttgctcgc ataagaagtg aaagtaataa ttatgcaaca 180

tattatggcg attcagtgaa agacaggttc accatctcca gagatgattc acaaaacatg 240

ctctatctac aaatgaacaa cttgaaaact gaggacacag ccatatatta ctgtgtgaga 300

gaggtaacta catcgtttgc ttattggggc caagggactc tggtcactgt ctctgca 357

<210> 73

<211> 369

<212> DNA

<213> Mus musculus

<400> 73

gaggtgcagc ttgttgagac tggtggagga ttggtgcagc ctaaagggtc attgaaactc 60

tcatgtgcag cctctggatt caccttcaat gccagtgcca tgaactgggt ccgccaggct 120

ccaggaaagg gtttggaatg ggttgctcgc ataagaagta aaagtaataa ttatgcaata 180

tattatgccg attcagtgaa agacaggttc accatctcca gagatgattc acaaagcatg 240

ctctatctgc aaatgaacaa cttgaaaact gaggacacag ccatgtatta ctgtgtgaga 300

gatccgggct actatggtaa cccctggttt gcttactggg gccaagggac tctggtcact 360

gtctctgca 369

<210> 74

<211> 111

<212> PRT

<213> Mus musculus

<400> 74

Gln Ile Gln Leu Glu Gln Ser Gly Pro Glu Leu Lys Lys Pro Gly Glu
1 5 10 15

Thr Val Lys Ile Ser Cys Lys Ala Ser Gly Tyr Ile Phe Arg Asp Tyr
20 25 30

Ser Met His Trp Val Lys Gln Ala Pro Gly Lys Gly Leu Lys Trp Met
35 40 45

Gly Trp Ile Asn Thr Glu Thr Gly Glu Pro Thr Tyr Ala Asp Asp Phe
50 55 60

Lys Gly Arg Phe Ala Phe Ser Leu Glu Thr Ser Ala Ser Thr Ala Tyr
65 70 75 80

Leu Gln Ile Asn Asn Leu Lys Asn Glu Asp Thr Ala Thr Tyr Phe Cys
85 90 95

Thr Ser Leu Tyr Trp Gly Gln Gly Thr Leu Val Thr Val Ser Ala

100 105 110

<210> 75

<211> 124

<212> PRT

<213> Mus musculus

<400> 75

Gln Val Thr Leu Lys Glu Ser Gly Pro Gly Ile Leu Gln Pro Ser Gln
1 5 10 15

Thr Leu Ser Leu Thr Cys Ser Phe Ser Gly Phe Ser Leu Ser Thr Tyr
20 25 30

Gly Met Gly Val Gly Trp Ile Arg Gln Pro Ser Gly Lys Gly Leu Glu
35 40 45

Trp Leu Ala Asn Ile Trp Trp His Asp Asp Lys Tyr Tyr Asn Ser Ala
50 55 60

Leu Lys Ser Arg Leu Thr Ile Ser Lys Asp Ile Ser Asn Asn Gln Val
65 70 75 80

Phe Leu Lys Ile Ser Ser Val Asp Thr Ala Asp Thr Ala Thr Tyr Tyr
85 90 95

Cys Ala Gln Ile Ala Pro Arg Tyr Asn Lys Tyr Glu Gly Phe Phe Ala
100 105 110

Phe Trp Gly Gln Gly Thr Leu Val Thr Val Ser Ala
115 120

<210> 76
<211> 115
<212> PRT
<213> Mus musculus

<400> 76
Gln Val Gln Leu Gln Gln Ser Gly Ala Glu Leu Val Arg Pro Gly Ala
1 5 10 15

Ser Val Lys Leu Ser Cys Lys Ala Ser Gly Tyr Thr Phe Thr Asp Tyr
20 25 30

Glu Met His Trp Val Lys Gln Thr Pro Val His Gly Leu Lys Trp Ile
35 40 45

Gly Ala Leu Asp Pro Lys Thr Gly Asp Thr Ala Tyr Ser Gln Lys Phe
50 55 60

Lys Gly Lys Ala Thr Leu Thr Ala Asp Lys Ser Ser Ser Thr Ala Tyr
65 70 75 80

Met Glu Leu Arg Ser Leu Thr Ser Glu Asp Ser Ala Val Tyr Tyr Cys
85 90 95

Thr Arg Phe Tyr Ser Tyr Thr Tyr Trp Gly Gln Gly Thr Leu Val Thr
100 105 110

Val Ser Ala
115

<210> 77
<211> 119
<212> PRT
<213> Mus musculus

<400> 77
Glu Val Gln Leu Val Glu Thr Gly Gly Gly Leu Val Gln Pro Glu Gly
1 5 10 15

Ser Leu Lys Leu Ser Cys Ala Ala Ser Gly Phe Ser Phe Asn Ile Asn
20 25 30

Ala Met Asn Trp Val Arg Gln Ala Pro Gly Lys Gly Leu Glu Trp Val
35 40 45

Ala Arg Ile Arg Ser Glu Ser Asn Asn Tyr Ala Thr Tyr Tyr Gly Asp
50 55 60

Ser Val Lys Asp Arg Phe Thr Ile Ser Arg Asp Asp Ser Gln Asn Met
65 70 75 80

Leu Tyr Leu Gln Met Asn Asn Leu Lys Thr Glu Asp Thr Ala Ile Tyr

85 90 95

Tyr Cys Val Arg Glu Val Thr Thr Ser Phe Ala Tyr Trp Gly Gln Gly
100 105 110

Thr Leu Val Thr Val Ser Ala
115

<210> 78
<211> 123
<212> PRT
<213> Mus musculus

<400> 78
Glu Val Gln Leu Val Glu Thr Gly Gly Gly Leu Val Gln Pro Lys Gly
1 5 10 15

Ser Leu Lys Leu Ser Cys Ala Ala Ser Gly Phe Thr Phe Asn Ala Ser
20 25 30

Ala Met Asn Trp Val Arg Gln Ala Pro Gly Lys Gly Leu Glu Trp Val
35 40 45

Ala Arg Ile Arg Ser Lys Ser Asn Asn Tyr Ala Ile Tyr Tyr Ala Asp
50 55 60

Ser Val Lys Asp Arg Phe Thr Ile Ser Arg Asp Asp Ser Gln Ser Met
65 70 75 80

Leu Tyr Leu Gln Met Asn Asn Leu Lys Thr Glu Asp Thr Ala Met Tyr
85 90 95

Tyr Cys Val Arg Asp Pro Gly Tyr Tyr Gly Asn Pro Trp Phe Ala Tyr
100 105 110

Trp Gly Gln Gly Thr Leu Val Thr Val Ser Ala
115 120

<210> 79
<211> 336
<212> DNA
<213> Mus musculus

<400> 79
gatgttgtga tgacccagac tccactcact ttgtcggtta cccttggaca accagcctcc 60
atctcttgca agtcaagtca gagcctctta catagtgatg gaaagacatt tttgaattgg 120
ttattacaga ggccaggcca gtctccaaag cgcctaatct atctggtgtc tagactggac 180
tctggagtcc ctgacaggtt cactggcagt ggatcaggga cagatttcac actgaaaatc 240
agcagagtgg aggctgagga tttgggagtt tattattgct gccaaggtac acattttcct 300
cggacgttcg gtggaggcac caggctggaa atcaaa 336

<210> 80
<211> 336
<212> DNA
<213> Mus musculus

<400> 80

gatgttttga tgacccaaac tccactctcc ctgcctgtca gtcttggaga tcaagcctcc 60

atctcttgca gatctagtca gagcattgta catagtaatg gaaacaccta tttagaatgg 120

tacctgcaga aaccaggcca gtctccaaag ctcctgatct acaaagtttc caaccgattt 180

tctggggtcc cagacaggtt cagtggcagt ggatcaggga cagatttcac actcaagatc 240

agcagagtgg aggctgagga tctgggagtt tattactgct ttcaaggttc acatgttccg 300

tggacgttcg gtggaggcac caagctggaa atcaaa 336

<210> 81

<211> 336

<212> DNA

<213> Mus musculus

<400> 81

gatgttgtga tgacccaaac tccactctcc ctgcctgtca gtcttggaga tcaagcctcc 60

atctcttgca gatctagtca gagccttgta cacagtaatg gaaacaccta tttacattgg 120

tacctgcaga agccaggcca gtctccaaag ctcctgatct acaaagtttc caaccgattt 180

tctggggtcc cagacaggtt cagtggcagt ggatcaggga cagatttcac actcaagatc 240

agcagagtgg aggctgagga tctgggagtt tatttctgct ctcaaaatac acatgttcct 300

cctacgttcg gatcggggac caagctggaa ataaaa 336

<210> 82

<211> 336

<212> DNA

<213> Mus musculus

<400> 82

gatattgtga tgactcagtc tgcaccctct gtacctgtca ctcctggaga gtcagtatcc 60

atctcctgca agtctagtaa gagtctcctg catagtaatg gcaacactta cttgaattgg 120

ttcctgcaga ggccaggcca gtctcctcaa ctcctgattt attggatgtc caaccttgcc 180

tcaggagtcc cagacaggtt cagtggcagt gggtcaggaa ctgctttcac actgagaatc 240

agtagagtgg aggctgagga tgtgggtgtt tattactgta tgcaacatat agaataccct 300

ttcacgttcg gcacggggac aaaattggaa ataaaa 336

<210> 83

<211> 336

<212> DNA

<213> Mus musculus

<400> 83

gatattgtga tgacgcaggc tgcattctcc aatccagtca ctcttggaac atcagcttcc 60

atctcctgca ggtctagtaa gagtctccta catagttatg acatcactta tttgtattgg 120

tatctgcaga agccaggcca gtctcctcag ctcctgattt atcagatgtc caaccttgcc 180

tcaggagtcc cagacaggtt cagtagcagt gggtcaggaa ctgatttcac actgagaatc 240

agcagagtgg aggctgagga tgtgggtgtt tattactgtg ctcaaaatct agaacttcct 300

ccgacgttcg gtggaggcac caagctggaa atcaaa 336

<210> 84

<211> 318

<212> DNA

<213> Mus musculus

<400> 84

caaattgttc tcacccagtc tccagcaatc atgtctgcat ttccagggga gaaggtcacc 60
atgacctgca gtgccagctc aagtgttagt tacatgtact ggtaccagca gaagtcagga 120
tcctccccca gactcctgat ttatgacaca tccaacctgg cttctggagt ccctgttcgc 180
ttcagtggca gtgggtctgg gacctcttac tctctcacaa tcagccgaat ggaggctgaa 240
gatgctgcca cttattactg ccagcagtgg agtagttacc cgctcacgtt cggtggtggg 300
accgagctgg agctgaaa 318

<210> 85
<211> 112
<212> PRT
<213> Mus musculus

<400> 85
Asp Val Val Met Thr Gln Thr Pro Leu Thr Leu Ser Val Thr Leu Gly
1               5                   10                  15

Gln Pro Ala Ser Ile Ser Cys Lys Ser Ser Gln Ser Leu Leu His Ser
            20                  25                  30

Asp Gly Lys Thr Phe Leu Asn Trp Leu Leu Gln Arg Pro Gly Gln Ser
        35                  40                  45

Pro Lys Arg Leu Ile Tyr Leu Val Ser Arg Leu Asp Ser Gly Val Pro
    50                  55                  60

Asp Arg Phe Thr Gly Ser Gly Ser Gly Thr Asp Phe Thr Leu Lys Ile
65                  70                  75                  80

Ser Arg Val Glu Ala Glu Asp Leu Gly Val Tyr Tyr Cys Cys Gln Gly
85 90 95

Thr His Phe Pro Arg Thr Phe Gly Gly Gly Thr Arg Leu Glu Ile Lys
100 105 110

<210> 86
<211> 112
<212> PRT
<213> Mus musculus

<400> 86
Asp Val Leu Met Thr Gln Thr Pro Leu Ser Leu Pro Val Ser Leu Gly
1 5 10 15

Asp Gln Ala Ser Ile Ser Cys Arg Ser Ser Gln Ser Ile Val His Ser
20 25 30

Asn Gly Asn Thr Tyr Leu Glu Trp Tyr Leu Gln Lys Pro Gly Gln Ser
35 40 45

Pro Lys Leu Leu Ile Tyr Lys Val Ser Asn Arg Phe Ser Gly Val Pro
50 55 60

Asp Arg Phe Ser Gly Ser Gly Ser Gly Thr Asp Phe Thr Leu Lys Ile

65 70 75 80

Ser Arg Val Glu Ala Glu Asp Leu Gly Val Tyr Tyr Cys Phe Gln Gly

85 90 95

Ser His Val Pro Trp Thr Phe Gly Gly Gly Thr Lys Leu Glu Ile Lys

100 105 110

<210> 87

<211> 112

<212> PRT

<213> Mus musculus

<400> 87

Asp Val Val Met Thr Gln Thr Pro Leu Ser Leu Pro Val Ser Leu Gly

1 5 10 15

Asp Gln Ala Ser Ile Ser Cys Arg Ser Ser Gln Ser Leu Val His Ser

20 25 30

Asn Gly Asn Thr Tyr Leu His Trp Tyr Leu Gln Lys Pro Gly Gln Ser

35 40 45

Pro Lys Leu Leu Ile Tyr Lys Val Ser Asn Arg Phe Ser Gly Val Pro

50 55 60

Asp Arg Phe Ser Gly Ser Gly Ser Gly Thr Asp Phe Thr Leu Lys Ile
65 70 75 80

Ser Arg Val Glu Ala Glu Asp Leu Gly Val Tyr Phe Cys Ser Gln Asn
85 90 95

Thr His Val Pro Pro Thr Phe Gly Ser Gly Thr Lys Leu Glu Ile Lys
100 105 110

<210> 88
<211> 112
<212> PRT
<213> Mus musculus

<400> 88
Asp Ile Val Met Thr Gln Ser Ala Pro Ser Val Pro Val Thr Pro Gly
1 5 10 15

Glu Ser Val Ser Ile Ser Cys Lys Ser Ser Lys Ser Leu Leu His Ser
20 25 30

Asn Gly Asn Thr Tyr Leu Asn Trp Phe Leu Gln Arg Pro Gly Gln Ser
35 40 45

Pro Gln Leu Leu Ile Tyr Trp Met Ser Asn Leu Ala Ser Gly Val Pro
50 55 60

Asp Arg Phe Ser Gly Ser Gly Ser Gly Thr Ala Phe Thr Leu Arg Ile
65 70 75 80

Ser Arg Val Glu Ala Glu Asp Val Gly Val Tyr Tyr Cys Met Gln His
85 90 95

Ile Glu Tyr Pro Phe Thr Phe Gly Thr Gly Thr Lys Leu Glu Ile Lys
100 105 110

<210> 89
<211> 112
<212> PRT
<213> Mus musculus

<400> 89
Asp Ile Val Met Thr Gln Ala Ala Phe Ser Asn Pro Val Thr Leu Gly
1 5 10 15

Thr Ser Ala Ser Ile Ser Cys Arg Ser Ser Lys Ser Leu Leu His Ser
20 25 30

Tyr Asp Ile Thr Tyr Leu Tyr Trp Tyr Leu Gln Lys Pro Gly Gln Ser

35 40 45

Pro Gln Leu Leu Ile Tyr Gln Met Ser Asn Leu Ala Ser Gly Val Pro
50 55 60

Asp Arg Phe Ser Ser Ser Gly Ser Gly Thr Asp Phe Thr Leu Arg Ile
65 70 75 80

Ser Arg Val Glu Ala Glu Asp Val Gly Val Tyr Tyr Cys Ala Gln Asn
85 90 95

Leu Glu Leu Pro Pro Thr Phe Gly Gly Gly Thr Lys Leu Glu Ile Lys
100 105 110

<210> 90

<211> 106

<212> PRT

<213> Mus musculus

<400> 90

Gln Ile Val Leu Thr Gln Ser Pro Ala Ile Met Ser Ala Phe Pro Gly
1 5 10 15

Glu Lys Val Thr Met Thr Cys Ser Ala Ser Ser Ser Val Ser Tyr Met
20 25 30

Tyr Trp Tyr Gln Gln Lys Ser Gly Ser Ser Pro Arg Leu Leu Ile Tyr
35 40 45

Asp Thr Ser Asn Leu Ala Ser Gly Val Pro Val Arg Phe Ser Gly Ser
50 55 60

Gly Ser Gly Thr Ser Tyr Ser Leu Thr Ile Ser Arg Met Glu Ala Glu
65 70 75 80

Asp Ala Ala Thr Tyr Tyr Cys Gln Gln Trp Ser Ser Tyr Pro Leu Thr
85 90 95

Phe Gly Gly Gly Thr Glu Leu Glu Leu Lys
100 105

<210> 91
<211> 345
<212> DNA
<213> Artificial Sequence

<220>
<223> Description of Artificial Sequence: Mouse-human chimeric antibody H chain

<400> 91
caggtgcagc tggtggagtc tggagctgag gtgaagaagc ctggggcctc agtgaaggtc 60
tcctgcaagg cttctggata caccttcacc gactatgaaa tgcactgggt gcgacaggcc 120

cctggacaag ggcttgagtg gatgggagct cttgatccta aaactggtga tactgcctac 180
agtcagaagt tcaagggcag agtcacgatt accgcggacg aatccacgag cacagcctac 240
atggagctga gcagcctgag atctgaggac acggccgtgt attactgtgc gagattctac 300
tcctatactt actggggcca gggaaccctg gtcaccgtct cctca 345

<210> 92
<211> 345
<212> DNA
<213> Artificial Sequence

<220>
<223> Description of Artificial Sequence: Mouse-human chimeric antibody H chain

<400> 92
caggtgcagc tggtggagtc tggagctgag gtgaagaagc ctggggcctc agtgaaggtc 60
tcctgcaagg cttctggata caccttcacc gactatgaaa tgcactgggt gcgacaggcc 120
cctggacaag ggcttgagtg gatgggagct cttgatccta aaactggtga tactgcctac 180
agtcagaagt tcaagggcag agtcacgctg accgcggacg aatccacgag cacagcctac 240
atggagctga gcagcctgag atctgaggac acggccgtgt attactgtac aagattctac 300
tcctatactt actggggcca gggaaccctg gtcaccgtct cctca 345

<210> 93
<211> 345
<212> DNA
<213> Artificial Sequence

<220>

<223> Description of Artificial Sequence: Mouse-human chimeric antibody H chain

<400> 93

caggtgcagc tggtggagtc tggagctgag gtgaagaagc ctggggcctc agtgaaggtc 60

tcctgcaagg cttctggata caccttcacc gactatgaaa tgcactgggt gcgacaggcc 120

cctggacaag ggcttgagtg gatgggagct cttgatccta aaactggtga tactgcctac 180

agtcagaagt tcaagggcag agtcacgctg accgcggaca aatccacgag cacagcctac 240

atggagctga gcagcctgag atctgaggac acggccgtgt attactgtac aagattctac 300

tcctatactt actggggcca gggaaccctg gtcaccgtct cctca 345

<210> 94

<211> 345

<212> DNA

<213> Artificial Sequence

<220>

<223> Description of Artificial Sequence: Mouse-human chimeric antibody H chain

<400> 94

caggtgcagc tggtggagtc tggagctgag gtgaagaagc ctggggcctc agtgaaggtc 60

tcctgcaagg cttctggata caccttcacc gactatgaaa tgcactgggt gcgacaggcc 120

cctggacaag ggcttgagtg gatgggagct cttgatccta aaactggtga tactgcctac 180

agtcagaagt tcaagggcag agtcacgctg accgcggaca aatccacgag cacagcctac 240

atggagctga gcagcctgac atctgaggac acggccgtgt attactgtac aagattctac 300

tcctatactt actggggcca gggaaccctg gtcaccgtct cctca 345

<210> 95
<211> 345
<212> DNA
<213> Artificial Sequence

<220>
<223> Description of Artificial Sequence: Mouse-human chimeric antibody H chain

<400> 95
caggtgcagc tggtgcagtc tggagctgag gtgaagaagc ctggggcctc agtgaaggtc 60
tcctgcaagg cttctggata caccttcacc gactatgaaa tgcactgggt gcgacaggcc 120
cctggacaag ggcttgagtg gatgggagct cttgatccta aaactggtga tactgcctac 180
agtcagaagt tcaagggcag agtcacgctg accgcggacg aatccacgag cacagcctac 240
atggagctga gcagcctgag atctgaggac acggccgtgt attactgtac aagattctac 300
tcctatactt actggggcca gggaaccctg gtcaccgtct cctca 345

<210> 96
<211> 345
<212> DNA
<213> Artificial Sequence

<220>
<223> Description of Artificial Sequence: Mouse-human chimeric antibody H chain

<400> 96

caggtgcagc tggtgcagtc tggagctgag gtgaagaagc ctggggcctc agtgaaggtc 60

tcctgcaagg cttctggata caccttcacc gactatgaaa tgcactgggt gcgacaggcc 120

cctggacaag ggcttgagtg gatgggagct cttgatccta aaactggtga tactgcctac 180

agtcagaagt tcaagggcag agtcacgctg accgcggaca aatccacgag cacagcctac 240

atggagctga gcagcctgag atctgaggac acggccgtgt attactgtac aagattctac 300

tcctatactt actggggcca gggaaccctg gtcaccgtct cctca 345

<210> 97

<211> 345

<212> DNA

<213> Artificial Sequence

<220>

<223> Description of Artificial Sequence: Mouse-human chimeric antibody H chain

<400> 97

caggtgcagc tggtgcagtc tggagctgag gtgaagaagc ctggggcctc agtgaaggtc 60

tcctgcaagg cttctggata caccttcacc gactatgaaa tgcactgggt gcgacaggcc 120

cctggacaag ggcttgagtg gatgggagct cttgatccta aaactggtga tactgcctac 180

agtcagaagt tcaagggcag agtcacgctg accgcggaca aatccacgag cacagcctac 240

atggagctga gcagcctgac atctgaggac acggccgtgt attactgtac aagattctac 300

tcctatactt actggggcca gggaaccctg gtcaccgtct cctca 345

<210> 98

<211> 115

<212> PRT

<213> Artificial Sequence

<220>

<223> Description of Artificial Sequence: Mouse-human chimeric antibody H chain

<400> 98

Gln Val Gln Leu Val Glu Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Glu Met His Trp Val Arg Gln Ala Pro Gly Gln Gly Leu Glu Trp Met
        35                  40                  45

Gly Ala Leu Asp Pro Lys Thr Gly Asp Thr Ala Tyr Ser Gln Lys Phe
    50                  55                  60

Lys Gly Arg Val Thr Ile Thr Ala Asp Glu Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Phe Tyr Ser Tyr Thr Tyr Trp Gly Gln Gly Thr Leu Val Thr
            100                 105                 110

Val Ser Ser

115

<210> 99

<211> 115

<212> PRT

<213> Artificial Sequence

<220>

<223> Description of Artificial Sequence: Mouse-human chimeric antibody H chain

<400> 99

Gln Val Gln Leu Val Glu Ser Gly Ala Glu Val Lys Lys Pro Gly Ala

1 5 10 15

Ser Val Lys Val Ser Cys Lys Ala Ser Gly Tyr Thr Phe Thr Asp Tyr

20 25 30

Glu Met His Trp Val Arg Gln Ala Pro Gly Gln Gly Leu Glu Trp Met

35 40 45

Gly Ala Leu Asp Pro Lys Thr Gly Asp Thr Ala Tyr Ser Gln Lys Phe

50 55 60

Lys Gly Arg Val Thr Leu Thr Ala Asp Glu Ser Thr Ser Thr Ala Tyr

65 70 75 80

Met Glu Leu Ser Ser Leu Arg Ser Glu Asp Thr Ala Val Tyr Tyr Cys
85 90 95

Thr Arg Phe Tyr Ser Tyr Thr Tyr Trp Gly Gln Gly Thr Leu Val Thr
100 105 110

Val Ser Ser
115

<210> 100
<211> 115
<212> PRT
<213> Artificial Sequence

<220>
<223> Description of Artificial Sequence: Mouse-human chimeric antibody H chain

<400> 100
Gln Val Gln Leu Val Glu Ser Gly Ala Glu Val Lys Lys Pro Gly Ala
1 5 10 15

Ser Val Lys Val Ser Cys Lys Ala Ser Gly Tyr Thr Phe Thr Asp Tyr
20 25 30

Glu Met His Trp Val Arg Gln Ala Pro Gly Gln Gly Leu Glu Trp Met
35 40 45

Gly Ala Leu Asp Pro Lys Thr Gly Asp Thr Ala Tyr Ser Gln Lys Phe
50 55 60

Lys Gly Arg Val Thr Leu Thr Ala Asp Lys Ser Thr Ser Thr Ala Tyr
65 70 75 80

Met Glu Leu Ser Ser Leu Arg Ser Glu Asp Thr Ala Val Tyr Tyr Cys
85 90 95

Thr Arg Phe Tyr Ser Tyr Thr Tyr Trp Gly Gln Gly Thr Leu Val Thr
100 105 110

Val Ser Ser
115

<210> 101
<211> 115
<212> PRT
<213> Artificial Sequence

<220>
<223> Description of Artificial Sequence: Mouse-human chimeric antibody H chain

<400> 101
Gln Val Gln Leu Val Glu Ser Gly Ala Glu Val Lys Lys Pro Gly Ala
1 5 10 15

Ser Val Lys Val Ser Cys Lys Ala Ser Gly Tyr Thr Phe Thr Asp Tyr
20 25 30

Glu Met His Trp Val Arg Gln Ala Pro Gly Gln Gly Leu Glu Trp Met
35 40 45

Gly Ala Leu Asp Pro Lys Thr Gly Asp Thr Ala Tyr Ser Gln Lys Phe
50 55 60

Lys Gly Arg Val Thr Leu Thr Ala Asp Lys Ser Thr Ser Thr Ala Tyr
65 70 75 80

Met Glu Leu Ser Ser Leu Thr Ser Glu Asp Thr Ala Val Tyr Tyr Cys
85 90 95

Thr Arg Phe Tyr Ser Tyr Thr Tyr Trp Gly Gln Gly Thr Leu Val Thr
100 105 110

Val Ser Ser
115

<210> 102
<211> 115
<212> PRT
<213> Artificial Sequence

<220>
<223> Description of Artificial Sequence: Mouse-human chimeric antibody

H chain

<400> 102

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Asp Tyr
20 25 30

Glu Met His Trp Val Arg Gln Ala Pro Gly Gln Gly Leu Glu Trp Met
35 40 45

Gly Ala Leu Asp Pro Lys Thr Gly Asp Thr Ala Tyr Ser Gln Lys Phe
50 55 60

Lys Gly Arg Val Thr Leu Thr Ala Asp Glu Ser Thr Ser Thr Ala Tyr
65 70 75 80

Met Glu Leu Ser Ser Leu Arg Ser Glu Asp Thr Ala Val Tyr Tyr Cys
85 90 95

Thr Arg Phe Tyr Ser Tyr Thr Tyr Trp Gly Gln Gly Thr Leu Val Thr
100 105 110

Val Ser Ser
115

<210> 103

<211> 115

<212> PRT

<213> Artificial Sequence

<220>

<223> Description of Artificial Sequence: Mouse-human chimeric antibody H chain

<400> 103

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Asp Tyr
20 25 30

Glu Met His Trp Val Arg Gln Ala Pro Gly Gln Gly Leu Glu Trp Met
35 40 45

Gly Ala Leu Asp Pro Lys Thr Gly Asp Thr Ala Tyr Ser Gln Lys Phe
50 55 60

Lys Gly Arg Val Thr Leu Thr Ala Asp Lys Ser Thr Ser Thr Ala Tyr
65 70 75 80

Met Glu Leu Ser Ser Leu Arg Ser Glu Asp Thr Ala Val Tyr Tyr Cys
85 90 95

Thr Arg Phe Tyr Ser Tyr Thr Tyr Trp Gly Gln Gly Thr Leu Val Thr
100 105 110

Val Ser Ser
115

<210> 104
<211> 115
<212> PRT
<213> Artificial Sequence

<220>
<223> Description of Artificial Sequence: Mouse-human chimeric antibody H chain

<400> 104
Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Asp Tyr
20 25 30

Glu Met His Trp Val Arg Gln Ala Pro Gly Gln Gly Leu Glu Trp Met
35 40 45

Gly Ala Leu Asp Pro Lys Thr Gly Asp Thr Ala Tyr Ser Gln Lys Phe
50 55 60

Lys Gly Arg Val Thr Leu Thr Ala Asp Lys Ser Thr Ser Thr Ala Tyr
65 70 75 80

Met Glu Leu Ser Ser Leu Thr Ser Glu Asp Thr Ala Val Tyr Tyr Cys
85 90 95

Thr Arg Phe Tyr Ser Tyr Thr Tyr Trp Gly Gln Gly Thr Leu Val Thr
100 105 110

Val Ser Ser
115

<210> 105
<211> 336
<212> DNA
<213> Artificial Sequence

<220>
<223> Description of Artificial Sequence: Mouse-human chimeric antibody L chain

<400> 105
gatgttgtga tgactcagtc tccactctcc ctgcccgtca cccctggaga gccggcctcc 60
atctcctgca gatctagtca gagccttgta cacagtaatg gaaacaccta tttacattgg 120
tacctgcaga agccagggca gtctccacag ctcctgatct ataaagtttc caaccgattt 180
tctggggtcc ctgacaggtt cagtggcagt ggatcaggca cagattttac actgaaaatc 240
agcagagtgg aggctgagga tgttggggtt tattactgct ctcaaaatac acatgttcct 300
cctacgtttg gccaggggac caagctggag atcaaa 336

<210> 106

<211> 112

<212> PRT

<213> Artificial Sequence

<220>

<223> Description of Artificial Sequence: Mouse-human chimeric antibody L chain

<400> 106

Asp Val Val Met Thr Gln Ser Pro Leu Ser Leu Pro Val Thr Pro Gly
1 5 10 15

Glu Pro Ala Ser Ile Ser Cys Arg Ser Ser Gln Ser Leu Val His Ser
20 25 30

Asn Gly Asn Thr Tyr Leu His Trp Tyr Leu Gln Lys Pro Gly Gln Ser
35 40 45

Pro Gln Leu Leu Ile Tyr Lys Val Ser Asn Arg Phe Ser Gly Val Pro
50 55 60

Asp Arg Phe Ser Gly Ser Gly Ser Gly Thr Asp Phe Thr Leu Lys Ile
65 70 75 80

Ser Arg Val Glu Ala Glu Asp Val Gly Val Tyr Tyr Cys Ser Gln Asn
85 90 95

Thr His Val Pro Pro Thr Phe Gly Gln Gly Thr Lys Leu Glu Ile Lys

100 105 110

<210> 107
<211> 27
<212> DNA
<213> Artificial Sequence

<220>
<223> Description of Artificial Sequence: Primer

<400> 107
cttgtacaca gtgacggaaa cacctat 27

<210> 108
<211> 27
<212> DNA
<213> Artificial Sequence

<220>
<223> Description of Artificial Sequence: Primer

<400> 108
ataggtgttt ccgtcactgt gtacaag 27

<210> 109

<211> 16

<212> PRT

<213> Artificial Sequence

<220>

<223> Description of Artificial Sequence: Mutant antibody L chain

<400> 109

Arg Ser Ser Gln Ser Leu Val His Ser Asn Ala Asn Thr Tyr Leu His

1 5 10 15

<210> 110

<211> 16

<212> PRT

<213> Artificial Sequence

<220>

<223> Description of Artificial Sequence: Mutant antibody L chain

<400> 110

Arg Ser Ser Gln Ser Leu Val His Ser Asn Asp Asn Thr Tyr Leu His

1 5 10 15

<210> 111

<211> 16

<212> PRT

<213> Artificial Sequence

<220>

<223> Description of Artificial Sequence: Mutant antibody L chain

<400> 111

Arg Ser Ser Gln Ser Leu Val His Ser Asn Glu Asn Thr Tyr Leu His
1 5 10 15

<210> 112

<211> 16

<212> PRT

<213> Artificial Sequence

<220>

<223> Description of Artificial Sequence: Mutant antibody L chain

<400> 112

Arg Ser Ser Gln Ser Leu Val His Ser Asn Phe Asn Thr Tyr Leu His
1 5 10 15

<210> 113

<211> 16

<212> PRT

<213> Artificial Sequence

<220>

<223> Description of Artificial Sequence: Mutant antibody L chain

<400> 113

Arg Ser Ser Gln Ser Leu Val His Ser Asn His Asn Thr Tyr Leu His
1               5                   10                  15

<210> 114

<211> 16

<212> PRT

<213> Artificial Sequence

<220>

<223> Description of Artificial Sequence: Mutant antibody L chain

<400> 114

Arg Ser Ser Gln Ser Leu Val His Ser Asn Asn Asn Thr Tyr Leu His
1               5                   10                  15

<210> 115

<211> 16

<212> PRT

<213> Artificial Sequence

<220>

<223> Description of Artificial Sequence: Mutant antibody L chain

<400> 115

Arg Ser Ser Gln Ser Leu Val His Ser Asn Thr Asn Thr Tyr Leu His
1 5 10 15

<210> 116

<211> 16

<212> PRT

<213> Artificial Sequence

<220>

<223> Description of Artificial Sequence: Mutant antibody L chain

<400> 116

Arg Ser Ser Gln Ser Leu Val His Ser Asn Gln Asn Thr Tyr Leu His
1 5 10 15

<210> 117

<211> 17

<212> PRT

<213> Artificial Sequence

<220>

<223> Description of Artificial Sequence: Mutant antibody L chain

<400> 117

Arg Ser Ser Gln Ser Leu Val His Ser Asn Gly Ile Asn Thr Tyr Leu
1 5 10 15

His

<210> 118

<211> 16

<212> PRT

<213> Artificial Sequence

<220>

<223> Description of Artificial Sequence: Mutant antibody L chain

<400> 118

Arg Ser Ser Gln Ser Leu Val His Ser Asn Lys Asn Thr Tyr Leu His
1 5 10 15

<210> 119

<211> 16

<212> PRT

<213> Artificial Sequence

<220>

<223> Description of Artificial Sequence: Mutant antibody L chain

<400> 119

Arg Ser Ser Gln Ser Leu Val His Ser Asn Leu Asn Thr Tyr Leu His
1 5 10 15

<210> 120

<211> 16

<212> PRT

<213> Artificial Sequence

<220>

<223> Description of Artificial Sequence: Mutant antibody L chain

<400> 120

Arg Ser Ser Gln Ser Leu Val His Ser Asn Ser Asn Thr Tyr Leu His
1 5 10 15

<210> 121

<211> 16

<212> PRT

<213> Artificial Sequence

<220>

<223> Description of Artificial Sequence: Mutant antibody L chain

<400> 121

Arg Ser Ser Gln Ser Leu Val His Ser Asn Trp Asn Thr Tyr Leu His
1 5 10 15

<210> 122

<211> 16

<212> PRT

<213> Artificial Sequence

<220>

<223> Description of Artificial Sequence: Mutant antibody L chain

<400> 122

Arg Ser Ser Gln Ser Leu Val His Ser Asn Tyr Asn Thr Tyr Leu His
1 5 10 15

<210> 123

<211> 16

<212> PRT

<213> Artificial Sequence

<220>

<223> Description of Artificial Sequence: Mutant antibody L chain

<400> 123

Arg Ser Ser Gln Ser Leu Val His Ser Asn Arg Asn Thr Tyr Leu His
1 5 10 15

<210> 124

<211> 16

<212> PRT

<213> Artificial Sequence

<220>

<223> Description of Artificial Sequence: Mutant antibody L chain

<400> 124

Arg Ser Ser Gln Ser Leu Val His Ser Asn Val Asn Thr Tyr Leu His
1               5                   10                  15

<210> 125

<211> 16

<212> PRT

<213> Artificial Sequence

<220>

<223> Description of Artificial Sequence: Mutant antibody L chain

<400> 125

Arg Ser Ser Gln Ser Leu Val His Ser Asn Pro Asn Thr Tyr Leu His
1               5                   10                  15

<210> 126

<211> 112

<212> PRT

<213> Artificial Sequence

<220>

<223> Description of Artificial Sequence: Mutant antibody L chain

<400> 126

Asp Val Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Val His Ser
            20                  25                  30

Asn Ala Asn Thr Tyr Leu His Trp Tyr Leu Gln Lys Pro Gly Gln Ser
        35                  40                  45

Pro Gln Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Asn
                85                  90                  95

Thr His Val Pro Pro Thr Phe Gly Gln Gly Thr Lys Leu Glu Ile Lys
            100                 105                 110

<210> 127

<211> 112

<212> PRT

<213> Artificial Sequence

<220>

<223> Description of Artificial Sequence: Mutant antibody L chain

<400> 127

Asp Val Val Met Thr Gln Ser Pro Leu Ser Leu Pro Val Thr Pro Gly
1 5 10 15

Glu Pro Ala Ser Ile Ser Cys Arg Ser Ser Gln Ser Leu Val His Ser
20 25 30

Asn Asp Asn Thr Tyr Leu His Trp Tyr Leu Gln Lys Pro Gly Gln Ser
35 40 45

Pro Gln Leu Leu Ile Tyr Lys Val Ser Asn Arg Phe Ser Gly Val Pro
50 55 60

Asp Arg Phe Ser Gly Ser Gly Ser Gly Thr Asp Phe Thr Leu Lys Ile
65 70 75 80

Ser Arg Val Glu Ala Glu Asp Val Gly Val Tyr Tyr Cys Ser Gln Asn
85 90 95

Thr His Val Pro Pro Thr Phe Gly Gln Gly Thr Lys Leu Glu Ile Lys
100 105 110

<210> 128

<211> 112

<212> PRT

<213> Artificial Sequence

<220>

<223> Description of Artificial Sequence: Mutant antibody L chain

<400> 128

Asp Val Val Met Thr Gln Ser Pro Leu Ser Leu Pro Val Thr Pro Gly
1 5 10 15

Glu Pro Ala Ser Ile Ser Cys Arg Ser Ser Gln Ser Leu Val His Ser
20 25 30

Asn Glu Asn Thr Tyr Leu His Trp Tyr Leu Gln Lys Pro Gly Gln Ser
35 40 45

Pro Gln Leu Leu Ile Tyr Lys Val Ser Asn Arg Phe Ser Gly Val Pro
50 55 60

Asp Arg Phe Ser Gly Ser Gly Ser Gly Thr Asp Phe Thr Leu Lys Ile
65 70 75 80

Ser Arg Val Glu Ala Glu Asp Val Gly Val Tyr Tyr Cys Ser Gln Asn
85 90 95

Thr His Val Pro Pro Thr Phe Gly Gln Gly Thr Lys Leu Glu Ile Lys
100 105 110

<210> 129
<211> 112
<212> PRT
<213> Artificial Sequence

<220>
<223> Description of Artificial Sequence: Mutant antibody L chain

<400> 129
Asp Val Val Met Thr Gln Ser Pro Leu Ser Leu Pro Val Thr Pro Gly
1 5 10 15

Glu Pro Ala Ser Ile Ser Cys Arg Ser Ser Gln Ser Leu Val His Ser
20 25 30

Asn Phe Asn Thr Tyr Leu His Trp Tyr Leu Gln Lys Pro Gly Gln Ser
35 40 45

Pro Gln Leu Leu Ile Tyr Lys Val Ser Asn Arg Phe Ser Gly Val Pro
50 55 60

Asp Arg Phe Ser Gly Ser Gly Ser Gly Thr Asp Phe Thr Leu Lys Ile
65 70 75 80

Ser Arg Val Glu Ala Glu Asp Val Gly Val Tyr Tyr Cys Ser Gln Asn
85 90 95

Thr His Val Pro Pro Thr Phe Gly Gln Gly Thr Lys Leu Glu Ile Lys
100 105 110

<210> 130
<211> 112
<212> PRT
<213> Artificial Sequence

<220>
<223> Description of Artificial Sequence: Mutant antibody L chain

<400> 130
Asp Val Val Met Thr Gln Ser Pro Leu Ser Leu Pro Val Thr Pro Gly
1 5 10 15

Glu Pro Ala Ser Ile Ser Cys Arg Ser Ser Gln Ser Leu Val His Ser
20 25 30

Asn His Asn Thr Tyr Leu His Trp Tyr Leu Gln Lys Pro Gly Gln Ser

35 40 45

Pro Gln Leu Leu Ile Tyr Lys Val Ser Asn Arg Phe Ser Gly Val Pro
50 55 60

Asp Arg Phe Ser Gly Ser Gly Ser Gly Thr Asp Phe Thr Leu Lys Ile
65 70 75 80

Ser Arg Val Glu Ala Glu Asp Val Gly Val Tyr Tyr Cys Ser Gln Asn
85 90 95

Thr His Val Pro Pro Thr Phe Gly Gln Gly Thr Lys Leu Glu Ile Lys
100 105 110

<210> 131
<211> 112
<212> PRT
<213> Artificial Sequence

<220>
<223> Description of Artificial Sequence: Mutant antibody L chai

<400> 131
Asp Val Val Met Thr Gln Ser Pro Leu Ser Leu Pro Val Thr Pro Gly
1 5 10 15

Glu Pro Ala Ser Ile Ser Cys Arg Ser Ser Gln Ser Leu Val His Ser
20 25 30

Asn Asn Asn Thr Tyr Leu His Trp Tyr Leu Gln Lys Pro Gly Gln Ser
35 40 45

Pro Gln Leu Leu Ile Tyr Lys Val Ser Asn Arg Phe Ser Gly Val Pro
50 55 60

Asp Arg Phe Ser Gly Ser Gly Ser Gly Thr Asp Phe Thr Leu Lys Ile
65 70 75 80

Ser Arg Val Glu Ala Glu Asp Val Gly Val Tyr Tyr Cys Ser Gln Asn
85 90 95

Thr His Val Pro Pro Thr Phe Gly Gln Gly Thr Lys Leu Glu Ile Lys
100 105 110

<210> 132

<211> 112

<212> PRT

<213> Artificial Sequence

<220>

<223> Description of Artificial Sequence: Mutant antibody L chain

<400> 132

Asp Val Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Val His Ser
            20                  25                  30

Asn Thr Asn Thr Tyr Leu His Trp Tyr Leu Gln Lys Pro Gly Gln Ser
        35                  40                  45

Pro Gln Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ser Gln Asn
                85                  90                  95

Thr His Val Pro Pro Thr Phe Gly Gln Gly Thr Lys Leu Glu Ile Lys
            100                 105                 110

<210> 133

<211> 112

<212> PRT

<213> Artificial Sequence

<220>

<223> Description of Artificial Sequence: Mutant antibody L chain

<400> 133

Asp Val Val Met Thr Gln Ser Pro Leu Ser Leu Pro Val Thr Pro Gly
1 5 10 15

Glu Pro Ala Ser Ile Ser Cys Arg Ser Ser Gln Ser Leu Val His Ser
20 25 30

Asn Gln Asn Thr Tyr Leu His Trp Tyr Leu Gln Lys Pro Gly Gln Ser
35 40 45

Pro Gln Leu Leu Ile Tyr Lys Val Ser Asn Arg Phe Ser Gly Val Pro
50 55 60

Asp Arg Phe Ser Gly Ser Gly Ser Gly Thr Asp Phe Thr Leu Lys Ile
65 70 75 80

Ser Arg Val Glu Ala Glu Asp Val Gly Val Tyr Tyr Cys Ser Gln Asn
85 90 95

Thr His Val Pro Pro Thr Phe Gly Gln Gly Thr Lys Leu Glu Ile Lys
100 105 110

<210> 134

<211> 112

<212> PRT

<213> Artificial Sequence

<220>

<223> Description of Artificial Sequence: Mutant antibody L chain

<400> 134

Asp Val Val Met Thr Gln Ser Pro Leu Ser Leu Pro Val Thr Pro Gly
1 5 10 15

Glu Pro Ala Ser Ile Ser Cys Arg Ser Ser Gln Ser Leu Val His Ser
20 25 30

Asn Ile Asn Thr Tyr Leu His Trp Tyr Leu Gln Lys Pro Gly Gln Ser
35 40 45

Pro Gln Leu Leu Ile Tyr Lys Val Ser Asn Arg Phe Ser Gly Val Pro
50 55 60

Asp Arg Phe Ser Gly Ser Gly Ser Gly Thr Asp Phe Thr Leu Lys Ile
65 70 75 80

Ser Arg Val Glu Ala Glu Asp Val Gly Val Tyr Tyr Cys Ser Gln Asn

85 90 95

Thr His Val Pro Pro Thr Phe Gly Gln Gly Thr Lys Leu Glu Ile Lys

100 105 110

<210> 135

<211> 112

<212> PRT

<213> Artificial Sequence

<220>

<223> Description of Artificial Sequence: Mutant antibody L chain

<400> 135

Asp Val Val Met Thr Gln Ser Pro Leu Ser Leu Pro Val Thr Pro Gly

1 5 10 15

Glu Pro Ala Ser Ile Ser Cys Arg Ser Ser Gln Ser Leu Val His Ser

20 25 30

Asn Lys Asn Thr Tyr Leu His Trp Tyr Leu Gln Lys Pro Gly Gln Ser

35 40 45

Pro Gln Leu Leu Ile Tyr Lys Val Ser Asn Arg Phe Ser Gly Val Pro

50 55 60

Asp Arg Phe Ser Gly Ser Gly Ser Gly Thr Asp Phe Thr Leu Lys Ile
65 70 75 80

Ser Arg Val Glu Ala Glu Asp Val Gly Val Tyr Tyr Cys Ser Gln Asn
85 90 95

Thr His Val Pro Pro Thr Phe Gly Gln Gly Thr Lys Leu Glu Ile Lys
100 105 110

<210> 136
<211> 112
<212> PRT
<213> Artificial Sequence

<220>
<223> Description of Artificial Sequence: Mutant antibody L chain

<400> 136
Asp Val Val Met Thr Gln Ser Pro Leu Ser Leu Pro Val Thr Pro Gly
1 5 10 15

Glu Pro Ala Ser Ile Ser Cys Arg Ser Ser Gln Ser Leu Val His Ser
20 25 30

Asn Leu Asn Thr Tyr Leu His Trp Tyr Leu Gln Lys Pro Gly Gln Ser
35 40 45

Pro Gln Leu Leu Ile Tyr Lys Val Ser Asn Arg Phe Ser Gly Val Pro
50 55 60

Asp Arg Phe Ser Gly Ser Gly Ser Gly Thr Asp Phe Thr Leu Lys Ile
65 70 75 80

Ser Arg Val Glu Ala Glu Asp Val Gly Val Tyr Tyr Cys Ser Gln Asn
85 90 95

Thr His Val Pro Pro Thr Phe Gly Gln Gly Thr Lys Leu Glu Ile Lys
100 105 110

<210> 137
<211> 112
<212> PRT
<213> Artificial Sequence

<220>
<223> Description of Artificial Sequence: Mutant antibody L chain

<400> 137
Asp Val Val Met Thr Gln Ser Pro Leu Ser Leu Pro Val Thr Pro Gly

1 5 10 15

Glu Pro Ala Ser Ile Ser Cys Arg Ser Ser Gln Ser Leu Val His Ser

20 25 30

Asn Ser Asn Thr Tyr Leu His Trp Tyr Leu Gln Lys Pro Gly Gln Ser

35 40 45

Pro Gln Leu Leu Ile Tyr Lys Val Ser Asn Arg Phe Ser Gly Val Pro

50 55 60

Asp Arg Phe Ser Gly Ser Gly Ser Gly Thr Asp Phe Thr Leu Lys Ile

65 70 75 80

Ser Arg Val Glu Ala Glu Asp Val Gly Val Tyr Tyr Cys Ser Gln Asn

85 90 95

Thr His Val Pro Pro Thr Phe Gly Gln Gly Thr Lys Leu Glu Ile Lys

100 105 110

<210> 138

<211> 112

<212> PRT

<213> Artificial Sequence

<220>

<223> Description of Artificial Sequence: Mutant antibody L chain

<400> 138

Asp Val Val Met Thr Gln Ser Pro Leu Ser Leu Pro Val Thr Pro Gly
1 5 10 15

Glu Pro Ala Ser Ile Ser Cys Arg Ser Ser Gln Ser Leu Val His Ser
20 25 30

Asn Trp Asn Thr Tyr Leu His Trp Tyr Leu Gln Lys Pro Gly Gln Ser
35 40 45

Pro Gln Leu Leu Ile Tyr Lys Val Ser Asn Arg Phe Ser Gly Val Pro
50 55 60

Asp Arg Phe Ser Gly Ser Gly Ser Gly Thr Asp Phe Thr Leu Lys Ile
65 70 75 80

Ser Arg Val Glu Ala Glu Asp Val Gly Val Tyr Tyr Cys Ser Gln Asn
85 90 95

Thr His Val Pro Pro Thr Phe Gly Gln Gly Thr Lys Leu Glu Ile Lys
100 105 110

<210> 139

<211> 112

<212> PRT

<213> Artificial Sequence

<220>

<223> Description of Artificial Sequence: Mutant antibody L chain

<400> 139

Asp Val Val Met Thr Gln Ser Pro Leu Ser Leu Pro Val Thr Pro Gly
1 5 10 15

Glu Pro Ala Ser Ile Ser Cys Arg Ser Ser Gln Ser Leu Val His Ser
20 25 30

Asn Tyr Asn Thr Tyr Leu His Trp Tyr Leu Gln Lys Pro Gly Gln Ser
35 40 45

Pro Gln Leu Leu Ile Tyr Lys Val Ser Asn Arg Phe Ser Gly Val Pro
50 55 60

Asp Arg Phe Ser Gly Ser Gly Ser Gly Thr Asp Phe Thr Leu Lys Ile
65 70 75 80

Ser Arg Val Glu Ala Glu Asp Val Gly Val Tyr Tyr Cys Ser Gln Asn
85 90 95

Thr His Val Pro Pro Thr Phe Gly Gln Gly Thr Lys Leu Glu Ile Lys
100 105 110

<210> 140

<211> 112

<212> PRT

<213> Artificial Sequence

<220>

<223> Description of Artificial Sequence: Mutant antibody L chain

<400> 140

Asp Val Val Met Thr Gln Ser Pro Leu Ser Leu Pro Val Thr Pro Gly
1 5 10 15

Glu Pro Ala Ser Ile Ser Cys Arg Ser Ser Gln Ser Leu Val His Ser
20 25 30

Asn Arg Asn Thr Tyr Leu His Trp Tyr Leu Gln Lys Pro Gly Gln Ser
35 40 45

Pro Gln Leu Leu Ile Tyr Lys Val Ser Asn Arg Phe Ser Gly Val Pro
50 55 60

Asp Arg Phe Ser Gly Ser Gly Ser Gly Thr Asp Phe Thr Leu Lys Ile
65 70 75 80

Ser Arg Val Glu Ala Glu Asp Val Gly Val Tyr Tyr Cys Ser Gln Asn
85 90 95

Thr His Val Pro Pro Thr Phe Gly Gln Gly Thr Lys Leu Glu Ile Lys
100 105 110

<210> 141
<211> 112
<212> PRT
<213> Artificial Sequence

<220>
<223> Description of Artificial Sequence: Mutant antibody L chain

<400> 141
Asp Val Val Met Thr Gln Ser Pro Leu Ser Leu Pro Val Thr Pro Gly
1 5 10 15

Glu Pro Ala Ser Ile Ser Cys Arg Ser Ser Gln Ser Leu Val His Ser
20 25 30

Asn Val Asn Thr Tyr Leu His Trp Tyr Leu Gln Lys Pro Gly Gln Ser
35 40 45

Pro Gln Leu Leu Ile Tyr Lys Val Ser Asn Arg Phe Ser Gly Val Pro

50 55 60

Asp Arg Phe Ser Gly Ser Gly Ser Gly Thr Asp Phe Thr Leu Lys Ile
65 70 75 80

Ser Arg Val Glu Ala Glu Asp Val Gly Val Tyr Tyr Cys Ser Gln Asn
85 90 95

Thr His Val Pro Pro Thr Phe Gly Gln Gly Thr Lys Leu Glu Ile Lys
100 105 110

<210> 142
<211> 112
<212> PRT
<213> Artificial Sequence

<220>
<223> Description of Artificial Sequence: Mutant antibody L chain

<400> 142
Asp Val Val Met Thr Gln Ser Pro Leu Ser Leu Pro Val Thr Pro Gly
1 5 10 15

Glu Pro Ala Ser Ile Ser Cys Arg Ser Ser Gln Ser Leu Val His Ser
20 25 30

Asn Pro Asn Thr Tyr Leu His Trp Tyr Leu Gln Lys Pro Gly Gln Ser
35 40 45

Pro Gln Leu Leu Ile Tyr Lys Val Ser Asn Arg Phe Ser Gly Val Pro
50 55 60

Asp Arg Phe Ser Gly Ser Gly Ser Gly Thr Asp Phe Thr Leu Lys Ile
65 70 75 80

Ser Arg Val Glu Ala Glu Asp Val Gly Val Tyr Tyr Cys Ser Gln Asn
85 90 95

Thr His Val Pro Pro Thr Phe Gly Gln Gly Thr Lys Leu Glu Ile Lys
100 105 110

## Description

### Technical Field

The present invention relates to a method of producing a recombinant protein, particularly an antibody, using a cell in which the function of a fucose transporter is inhibited.

### Background Art

Antibodies can exert anti-tumor effects via their ADCC (antibody-dependent cell-mediated cytotoxicity) activity or CDC (complement dependent cytotoxicity) activity. Antibodies are sugar chain-bound glycoproteins. It is known that an antibody's cytotoxic activity level can vary depending on the types and amounts of sugar chains that bind to the antibody. In particular, it has been reported that the amount of fucose binding to an antibody is strongly involved in the cytotoxic activity level (Shields et al., J Biol Chem-, 277(30), 26733-26740, 2002). Furthermore, a method of producing a recombinant antibody not having fucose has been reported, Such method involves preventing an enzyme that catalyzes the binding of fucose to a sugar chain from being expressed upon antibody production in order to obtain an antibody with enhanced cytotoxic activity (International Patent Publication No, WO00/61739).

### Disclosure of the Invention

An object of the present invention is to provide a method for easily and reliably producing a recombinant protein wherein the binding of fucose is eliminated or decreases. In particular, an object of the present invention is to provide a method of producing an antibody wherein the binding of fucose is eliminated or decreases and whose cytotoxic activity is enhanced. Furthermore, another object of the present invention is to provide a host cell for producing such protein

### Means to Solve the Problems

In a mechanism by which fucose binds to an antibody within an antibody-producing cell, it is known that GDP binds to fucose that has been incorporated into a cell. GDP-fucose is then incorporated into the Golgi apparatus and then the fucose of the GDP-fucose is transferred to N-acetylglucosamine that has been added as a sugar chain to protein within the Golgi apparatus. Specifically, the Fc region of an antibody molecule has two sites to which an N-glycoside-bound sugar chain binds. Fucose binds to the N-acetylglucosainiiie portion of an N-glycoside-bound sugar chain (Pate L. Smith et al., J. Cell Biol. 2002, 158, 801-815).

The present inventors have considered that disruption of fucose transporter genes on both chromosomes leads to inhibition of the incorporation of fucose into the Golgi apparatus, so that addition of fucose to an antibody can be inhibited The present inventors have prepared a cell wherein fucose transporter genes on both chromosomes are disrupted and thus completed the present invention.

In the present invention, to satisfy conditions where the addition of fucose to an antibody is inhibited, it is not necessary that all the produced antibodies do not experience the addition of fucose thereto, but the proportion of protein to which fucose has been added should be decreased among antibody compositions

The present invention will be described as follows

[1] A cell, in which the expression of fucose transporter genes on both chromosomes is artificially suppressed.
[2] The cell according to [1], in which the fucose transporter genes are disrupted.
[3] The cell according to [1] or [2], which is an animal cell.
[4] The cell according to [3], in which the animal cell is a Chinese hamster cell
[5] The cell according to [4], in which the animal cell is a CHO cell
[6] The cell according to any one of [2] to [5], in which gene disruption is carried out by homologous recombination using a gene targeting vector.
[7] The cell according to any one of [1] to [6], in which a gene encoding a foreign protein is introduced.
[8] The cell according to [7], in which the gene encoding the foreign protein is a gene encoding an antibody
[9] A method of producing a protein, comprising culturing the cell according to any one of [1] to [8].
[10] The production method according to [9], in which the protein is an antibody.
[11] The production method according to [10], comprising producing a protein to which fucose is not added
[12] A method for inhibiting the addition of fucose to a protein, comprising artificially suppressing the expression of fucose transporter genes on both chromosomes upon production of a recombinant protein using a cell.
[13] The method for inhibiting the addition of fucose to a protein according to [12], comprising artificially suppressing gene expression through deletion of a gene.

[14] The method for inhibiting the addition of fucose to a protein according to [12] or [13], in which the protein is an antibody.
[15] The method for inhibiting the addition of fucose to a protein according to any one of [12] to [14], in which the cell is a CHO cell.

This description includes part or all of the contents as disclosed in the description and/or drawings of PCT International Patent Application No. PCT/IP2004/019261, which is apriority document of the present application.

Brief Description of the Drawings

Fig. 1 shows the structures of 3 types of targeting vector.
Fig. 2 shows the structures of bands that appeared after knockout of the first step and the following cleavage with *Bgl*II.
Fig. 3 shows the results of southern blot analysis in the case of the knockout of the first step.
Fig. 4 shows homologous recombination efficiencies in the case of the knockout of the second step.
Fig. 5 is a photograph showing the results of Southern blot analysis of fucose transporter gene-deficient cell lines.
Fig. 6 shows the results of fucose expression analysis of a fucose transporter gene-deficient cell line (wild/KO).
Fig. 7 shows the results of fucose expression analysis of a fucose transporter gene-deficient cell line (KO/KO).
Fig. 8 shows the ADCC activity of each anti-HM1.24 antibody as determined using human PBMC and ARH-77 as a target cell.
Fig. 9 shows the ADCC activity of each anti-GPC3 antibody as determined using human PBMC and HuH-7 as a target cell.
Fig. 10 shows the normal phase HPLC chromatograms of 2-AB-labeled sugar chains prepared from a humanized anti-HM1.24 antibody (a) produced by FT-KO cells and a humanized anti-HM1.24 antibody (b) produced by CHO cells.
Fig. 11 shows the normal phase HPLC chromatograms of agalactosyl 2-AB-labeled sugar chains prepared from humanized anti-GPC3 antibodies (a, b, and c) produced by FT-KO cells and a humanized anti-GPC3 antibody produced by CHO cells.
Fig. 12 shows inferred structures of peak G(0) and G(0)-Fuc shown in Fig. 11.
Fig 13 shows a DSC (differential scanning calorimeter) measurement chart showing the results of measuring an antibody (a) produced by FT-KO cells and an antibody (b) produced by CHO cells.

Preferred Embodiments of the Invention

"Fucose transporter" in the present invention means a polypeptide having fucose transport activity. For example, when a fucose transporter is expressed on the cell membrane, it generally incorporates fucose into the cells. When a fucose transporter is expressed on the Golgi membrane, it generally incorporates fucose into the Golgi apparatus. In the present invention, a preferable fucose transporter is a Chinese hamster fucose transporter, and a more preferable example is a fucose transporter having the amino acid sequence represented by SEQ ID NO: 2. SEQ ID NO: 1 shows the nucleotide sequence of the Chinese hamster fucose transporter gene.
The Golgi apparatus incorporates fucose into itself mainly via fucose transporters existing on the Golgi membrane. Through inhibition of the fucose transporter function, incorporation of fucose into the Golgi apparatus can be inhibited and the amount of fucose to be incorporated into the Golgi apparatus can be decreased.
To inhibit the fucose transporter function of cells means to cause a decrease or disappearance of the fucose transport activity of a fucose transporter.
The fucose transporter function of cells may be inhibited by any method. A method for inhibiting fucose transporter expression is preferable.
A method for inhibiting fucose transporter expression is not specifically limited, as long as the number of fucose transporters having normal transport ability decreases. A method that involves deleting (knockout) a gene (fucose transporter gene) encoding a fucose transporter through the use of a targeting vector targeting the fucose transporter or the like is preferable.
Generally a cell has fucose transporter genes on both chromosomes. The cell of the present invention, in which the function of a fucose transporter is inhibited, lacks fucose transporter genes on both chromosomes. Alternatively, the cell of the present invention, in which the function of a fucose transporter is inhibited, is not particularly limited, as long as it lacks two fucose transporter genes on both chromosomes Preferably, the cell lacks whole fucose transporter genes existing on chromosomes For example, when 3 fucose transporter genes in total exist on chromosomes, deletion of 3 fucose transporter genes is preferable. Whether or not all the fucose transporter genes on chromosomes have been deleted can be examined using Southern blot analysis as described in Examples below or the FISH method, for example.

Protein produced by the production method of the present invention may be any protein. In general, such protein is a glycoprotein and preferably an antibody.

Types of antibody that are produced by the method of the present invention are not specifically limited. For example, mouse antibodies, rat antibodies, rabbit antibodies, sheep antibodies, camel antibodies, human antibodies, and artificially altered (for the purpose of, for example, lowering heterologous antigenicity against humans) gene recombinant antibody such as a chimeric antibody or a humanized antibody can be appropriately used. Such gene recombinant antibodies can be produced using a known method. A chimeric antibody comprises the variable region of the heavy and light chains of an antibody of a non-human mammal such as a mouse and the constant region of the heavy and light chains of a human antibody, DNA encoding the variable region of a mouse antibody is ligated to DNA encoding the constant region of a human antibody. The resultant is incorporated into an expression vector, and then the vector is introduced into a host to cause the host to produce the gene product. Thus a gene recombinant antibody can be obtained. A humanized antibody is also referred to as a reshaped human antibody. A humanized antibody is obtained by transplanting the complementarity determining region (CDR) of an antibody of a non-human mammal such as a mouse into the complementarity determining region of a human antibody. General gene recombination techniques therefor are also known. Specifically, DNA sequences designed to have the CDR of a mouse antibody ligated to the framework region (FR) of a human antibody are synthesized by the PCR method from several oligonucleotides, adjacent oligonucleotides of which have an overlap region at their terminal portions. The thus obtained DNA is ligated to DNA encoding the constant region of a human antibody, the resultant is incorporated into an expression vector, and then the vector is introduced into a host to cause the host to produce the gene product, so that a gene recombinant antibody can be obtained (see European Patent Application Publication No. EP 239400 and International Patent Application Publication No. WO 96/02576). As the FR of a human antibody, which is ligated via CDR, FR that allows the formation of an antigen-binding site with a good complementarity determining region is selected. If necessary, for the formation of an antigen-binding site having the appropriate complementarity determining region of a reshaped human antibody, the amino acids of the framework region of an antibody variable region may be substituted (Sato, K. et al., Cancer Res, 1993, 53, 851-856). Furthermore, methods for obtaining human antibodies are also known. For example, a desired human antibody having activity of binding to an antigen can also be obtained by sensitizing a human lymphocyte *in vitro* with a desired antigen or a cell that expresses a desired antigen and then fusing the sensitized lymphocyte to a human myeloma cell such as U266 (see JP Patent Publication (Kokoku) No. 1-59878 B (1989)) Moreover, a desired human antibody can be obtained by immunizing a transgenic animal that has all the repertories of a human antibody gene with a desired antigen (see International Patent Application Publication Nos. WO93/12227, WO92/03918, WO94/02602, WO94/25585, WO96/34096, and WO96/33735). Furthermore, a technique is also known by which a human antibody is obtained by panning using a human antibody library. For example, the variable region of a human antibody is expressed as a single chain antibody (scFv) on the surface of a phage by a phage display method. A phage that binds to an antigen can be selected. A DNA sequence encoding the variable region of a human antibody that binds to the antigen can be determined by analyzing the gene of the thus selected phage. When the

DNA sequence of scFv that binds to an antigen is revealed, an appropriate expression vector is constructed based on the sequence, and then a human antibody can be obtained. These methods are already known. Concerning these methods, WO92/01047, WO92/20791, WO93/06213, WO93/11236, WO93/19172, WO95/01438, and WO95/15388 can be referred to

Furthermore, the antibody of the present invention may be a lower molecular weight antibody such as an antibody fragment or a modified product of the antibody, as long as such antibody can bind to an antigen. Examples of such antibody fragment include Fab, F(ab')2, Fv, or single chain Fv(scFv) wherein Fv of the H chain and Fv of the L chain are linked using an appropriate linker (Huston, J. S. et al., Proc. Natl Acad. Sci. U.S.A. (1988) 85, 5879-5883), and a diabody. To obtain such antibody fragment, a gene encoding such antibody fragment is constructed, the gene is introduced into an expression vector, and then the gene is expressed in an appropriate host cell (e.g., see Co, M. S.. et al., J. Immunol. (1994) 152, 2968-2976; Better, M and Horwitz, A. H., Methods Enzymol. (1989) 178, 476-496; Pluckthun, A. and Skerra, A., Methods Enzymol. (1989) 178, 497-515; Lamoyi, E., Methods Enzymol. (1986) 121, 652-663; Rousseaux, J. et al., Methods Enzymol (1986) 121, 663-669; and Bird, R. E. and Walker, B. W., Trends Biotechnol. (1991) 9, 132-137). A diabody is prepared by dimerization; specifically, by linking two fragments (e.g., scFv), each of which is prepared by linking two variable regions using a linker or the like (hereinafter, referred to as a fragment composing a diabody). Generally, a diabody contains two VLs and two VHs (P. Hoiliger et al., Proc Natl. Acad. Sci. U.S.A. 90, 6444-6448 (1993); EP404097; WO93/11161; Johnson et al., Methods in Enzymology, 203, 88-98, (1991); Holliger et al., Protein Engineering, 9, 299-305, (1996); Perisic et al., Structure, 2, 1217-1226, (1994); John et al., Protein Engineering, 12(7), 597-604, (1999); Holliger et al., Proc Natl. Acad. Sci. U.S.A 90, 6444-6448, (1993); and Atwell et al., Mol. Immunol. 33, 1301-1312, (1996)).

As a modified product of an antibody, antibodies to which various molecules such as polyethylene glycol (PEG) have been bound can also be used Furthermore, a radioactive isotope, a chemical therapeutic agent, a cytotoxic substance such as toxin derived from bacteria, or the like can be bound to an antibody. In particular, a radiolabeled antibody is useful. Such modified product of an antibody can be obtained by chemically modifying an obtained antibody. In addition, methods for modifying antibodies have already been established in the field.

<Protein production method according to the method of the present invention>

A recombinant polypeptide can be produced by a method known by persons skilled in the art. In general, such recombinant polypeptide can be purified and prepared as follows, DNA encoding a polypeptide is incorporated into an appropriate expression vector, a transformant that has been obtained by introducing the vector into an appropriate host cell is collected, and then an extract is obtained. Subsequently, the resultant is subjected to chromatography such as ion exchange chromatography, reverse phase chromatography, or gel filtration, affinity chromatography using a column (to which an antibody against the polypeptide of the present invention has been immobilized), or chromatography using combination of a plurality of such columns.

Furthermore, when protein is expressed as a fusion polypeptide with a glutathione-S-transferase protein or as a recombinant polypeptide to which a plurality of histidines have been added in host cells (e.g., animal cells or *Escherichia coli*), the expressed recombinant polypeptide can be purified using a glutathione column or a nickel column. After purification of the fusion polypeptide, if necessary, regions other than the target polypeptide can also be cleaved and removed from the fusion polypeptide using thrombin, factor Xa or the like.

Protein to be produced by the production method of the present invention is preferably an antibody with cytotoxic activity that is affected by fucose binding thereto.

A method of producing an antibody using genetic recombination techniques, which is well known by persons skilled in the art, involves incorporating an antibody gene into an appropriate vector, introducing the vector into a host, and thus causing the production of the antibody using genetic recombination techniques (e.g., see Carl, A. K. Borrebaeck, James, W. Larrick, THERAPEUTIC MONOCLONAL ANTIBODIES, Published in the United Kingdom by MACMILLAN PUBLISHERS LTD, 1990).

<Cells to be used in the protein production method of the present invention and protein production using the cells>

Furthermore, the present invention encompasses a host cell that can produce a foreign protein, wherein the expression of fucose transporter genes existing on both chromosomes is artificially suppressed.

A protein having no fucose binding thereto can be obtained by expressing a foreign protein using the aforementioned cells wherein the expression of fucose transporter genes on both chromosomes is artificially suppressed as host cells. Here, a foreign protein means a protein not derived from the cell itself Host cells are not specifically limited. For example, cells wherein sugar is added to a recombinant protein when the protein is expressed can be used. More specifically, various animal cells or the like can be used. Preferably CHO cells can be used. In the present invention, in particular, CHO cells wherein a fucose transporter gene has been knocked out can be appropriately used. As animal cells, mammalian cells such as CHO (J. Exp. Med. (1995) 108, 945), COS, 3I3, myeloma, BHK (baby hamster kidney), HeLa, and Vero are known. As amphibian cells, Xenopus oocytes (Valle, et al., Nature (1981) 291, 358-340), for example, are known. As insect cells Sf9, Sf21, and Tn5, for example, are known. Examples of CHO cells include dhfr-CHO cells (Proc Natl. Acad. Sci. U.S.A. (1980) 77, 4216-4220) and CHO K-1 cells (Proc. Natl. Acad. Sci. U.S.A. (1968) 60, 1275), which are deficient in a DHFR gene. For the purpose of mass-expression in animal cells, CHO cells are particularly preferable.

Protein having no fucose binding thereto can be obtained by incorporating a gene encoding a foreign protein such as an antibody to be produced into an expression vector and then incorporating the expression vector into host cells capable of producing such foreign protein; that is, cells having an inhibited fucose transporter function. Examples of vectors include expression vectors derived from mammals (e.g., pcDNA3 (produced by Invitrogen Corporation) and pEGF-BOS (Nucleic Acids. Res.1990, 19(17), p. 5322), pEF, and pCDM8), expression vectors derived from insect cells (e g., the "Bac-to-BAC baculovirus expression system" (produced by GIBCO-BRL Life Technologies Inc) and pBacPAK8), expression vectors derived from plants (e.g., pMH1 and pMH2), expression vectors derived from animal viruses (e.g., pHSV, pMV, and pAdexLcw), expression vectors derived from retroviruses (e.g., pZIPneo), expression vectors derived from yeast (e.g., the "Pichia Expression Kit" (produced by Invitrogen Coiporation), pNV11, and SP-Q01), and expression vectors derived from *Bacillus subtilis* (e.g, pPL608 and pKTH50). When CHO cells are used as host cells, it is preferable to use a vector derived from a mammal.

For the purpose of expression in animal cells such as CHO cells, COS cells, or NIH3T3 cells, generally a vector used herein has a promoter required for expression within cells, such as an SV40 promoter (Mulligan et al, Nature (1979) 277, 108), an MMLV-LTR promoter, an EF1$\alpha$ promoter (Mizushima et al., Nucleic Acids Res. (1990) 18, 5322), or a CMV promoter. It is further preferable that such vector has a gene for selection of transformed cells (e.g., a drug resistance gene that enables distinguishment by the use of a drug (e.g , neomycin and G418)). Examples of a vector having such properties include pMAM, pDR2, pBK-RSV, pBK-CMV, pOPRSV, and pOP13,

Furthermore, an example of a method for the purpose of stable expression of a gene and amplification of the number of copies of a gene within cells involves introducing a vector (e.g., pCHOI) having a complementary DHFR gene into CHO cells that are deficient in the nucleic acid synthesis pathway, followed by amplification using methotrexate (MIX).. Furthermore, an example of a method for the purpose of transient expression of a gene involves transforming COS cells having a gene that expresses SV40 T antigen on a chromosome with a vector (e.g., pcD) having an SV40 replication origin As a replication initiation site, a site derived from polyoma virus, adenovirus, bovine papilloma virus (BPV), or the like can also be used Furthermore, to amplify the number of copies of a gene in a host cell system, an expression vector may contain as a selection marker an aminoglycoside transferase (APH) gene, thymidine kinase (TK) gene, *Esherichia coli* xanthine-guanine phosphoribosyltransferase (Ecogpt) gene, dihydrofolate reductase (dhfr) gene, or the like.

A vector can be introduced into a host cell by, for example, a calcium phosphate method, a DEAE dextran method, a method using cationic ribosome DOTAP (produced by Boehringer Mannheim, an electroporation method, or lipofection.

Cell culture can be carried out according to a known method As a culture solution for animal cells, DMEM, MEM, RPMI1640, or IMDM, for example, can be used. At this time, a seum fluid such as fetal calf serum (FCS) can be used together therewith. Alternatively serum-free culture may also be carried out. pH during culture is preferably between approximately 6 and 8. Culture is generally carried out at approximately 30°C to 40°C for approximately 15 to 200 hours If necessary, exchange of media, aeration, and agitation are carried out.

An example of such a cell wherein the expression of fucose transporter genes on both chromosomes is artificially suppressed is a cell wherein fucose transporter genes existing on both chromosomes have been disrupted.

"Disruption of a gene" means the suppression of the expression of the gene by partial deletion, substitution, insertion, addition, or the like conducted for the nucleotide sequence of the gene. Here, "suppression of gene expression" may also include a case where the gene itself is expressed, but a gene encoding a protein having normal functions is not expressed.

"Disruption of a gene" of the present invention includes not only a case where gene expression is completely suppressed, but also a case wherein gene expression is partially suppressed. "Deletion (knockout) of a gene" and "inactivation of a gene" are also used so as to have a meaning equivalent to that of "disruption of a gene " Furthermore, cells having a gene disrupted by homologous recombination using gene targeting are referred to as gene-knock out cells A cell wherein a gene encoding a fucose transporter is disrupted is an example of a cell wherein fucose transporter expression is artificially suppressed.

A cell wherein a gene encoding a fucose transporter is disrupted is a cell wherein the amount of fucose existing in the Golgi apparatus is significantly decreased compared with that in a cell wherein a fucose transporter gene is not disrupted, a cell wherein intracellular fucose transport ability is lowered or deleted, or a cell wherein intracellular activity to incorporate fucose into the Golgi apparatus is lowered or eliminated

In particular, cells wherein both fucose transporter genes homologous chromosome pair have been deleted exert the above properties more significantly than cells lacking single fucose transporter gene.

The amount of fucose in the Golgi apparatus can be measured by isolating the Golgi apparatus from a cell, extracting sugar, and then carrying out an antigen antibody reaction, a binding reaction between sugar and lectin, liquid chromatography, electrophoresis, or the like. Moreover, intracellular fucose transport ability and intracellular activity to incorporate fucose into the Golgi apparatus can be determined by, for example, using fucose labeled with a fluorescent substance, a radioisotope, or the like.

A gene can be disrupted by, for example, a homologous recombination method.

Such homologous recombination method means a method by which only the target gene is arbitrarily altered by homologous gene recombination between a gene on a chromosome and a foreign DNA Another DNA sequence is inserted into an exon of a gene for the purpose of dividing a sequence encoding a protein. To facilitate identification of a cell having a gene targeting vector, a selection marker such as a neomycin resistance gene derived from a bacterium is generally used as a sequence to divide the gene. A targeting vector is designed and produced based on the sequence information of the fucose transporter gene described in this specification and the fucose transporter gene to be disrupted is then subjected to homologous recombination using the targeting vector. For example, a substitution vector can contain a homologous region that has been ligated to the 5' and the 3' side of mutation to be introduced, a positive selection marker, a restriction enzyme site for linearizing the vector outside the homologous region, a negative selection marker arranged outside the homologous region, a restriction enzyme cleavage site for detecting mutation, and the like. Targeting vectors can be produced according to methods described in, for example. edited by Kenichi Yamamura et al., Transgenic Animal, KYORITSU SHUPPAN CO., LTD., March 1, 1997; Shinichi Aizawa, Gene Targeting, Production of Mutant Mice using ES cells, Bio Manual Series 8, YODOSHA CO., LTD., 1995; Hogan et al, Manipulating the Mouse Embryo, Cold Spring Harbor Laboratory Press (1944); Joyner, A.L., Gene Targeting, A Practical Approach Series, IRL Press (1993); and edited by Masami Matsumura et al., Experimental Medicine, Separate Volume, New Genetic Engineering Handbook (3rd revised version), YODOSHA CO., LTD., 1999. Both insertion and substitution targeting vectors may be used Furthermore, recombination can also be caused by targeting using a Cre-lox system. Targeting using the Cre-lox system can be carried out according to a method describe in, for example, JP Patent Publication (Kohyo) NO. 11-503015 A (1999). As a method for selecting homologous recombinants that have experienced homologous recombination, a known selection method such as positive selection, promoter selection, negative selection, or polyA selection may be used. For identification of a homologous recombinant, both the PCR method and the Southern blotting method can be used..

Furthermore, a cell wherein the fucose transporter gene of the present invention is disrupted can also be obtained by randomly introducing a mutation into a cell, as long as both fructose transporters on chromosome pair are deleted Examples of a method for randomly introducing mutation into a cell include a method that involves randomly introducing a gene disruption vector containing a marker into the genome of a cell and then screening for a cell having a disrupted fucose transporter gene, and a method that involves randomly introducing mutation using an chemical mutagen such as ENU (N-ethyl-N-nitrosourea) and then screening for such cell having a disrupted fucose transporter gene. Screening for cells that have become unable to produce any fucose transporter may be carried out using fucose transporter activity as an index. Alternatively, such screening can also be carried out by Western blotting or Northern blotting using fucose transporter gene transcription or expression as an index.

Furthermore, the cell of the present invention having a disrupted fucose transporter gene can also be obtained from an animal having a knocked-out fucose transporter gene. Such animal having a knocked-out fucose transporter gene can be produced by disrupting a fucose transporter of an ES cell by the above method and then producing from the ES cell according to, for example, a method disclosed in WO02/33054 Publication. Examples of animals that are used in this case include, but are not limited to, goats, pigs, sheep, cattle, mice, hamsters, and rats. Established cells having no fucose transporter genes can be obtained by producing such established cells from animals having a knocked-out fucose transporter gene.

When a foreign recombinant protein is produced according to the present invention in host cells wherein two fucose transporter genes on both chromosomes have been disrupted, addition of fucose to a protein is inhibited This is because fucose within each of the cells is not transported into the Golgi apparatus. In the case of such recombinant protein produced in host cells having disrupted fucose transporter genes, the amount of bound fucose is significantly lower or preferably unable to be detected, compared with the case of recombinant protein produced in host cells having an undisrupted fucose transporter gene When a foreign protein is an antibody, a product can be obtained wherein no fucose is binding to an N-glycoside-boutid sugar chain binding to 2 sugar-chain-binding sites existing in 1 molecule of an antibody; that is, existing in the Fe region composed of 2 H chains. Such antibody having no fucose binding thereto has enhanced cytotoxic activity In addition, when an antibody for which the addition of fucose thereto is inhibited is produced using the cell of the present invention, it is not necessary that all the produced antibodies have not experienced the addition of fucose thereto. The proportion of protein to which fucose has been added in antibody compositions should be reduced.

For example, in the case of an antibody that is produced using the animal cell of the present invention having disrupted fucose transporter genes, 50% or more, preferably 70% or more, further preferably 85% or more, and particularly preferably 90% or more of all the sugar chains has not experienced the addition of fucose thereto.

Furthermore, the present invention also encompasses animals (excluding humans) wherein fucose transporter genes on both chromosomes are deleted. A recombinant polypeptide can be produced *in vivo* using such animals.

DNA encoding target protein is introduced into these animals, polypeptides are produced *in vivo* within the animals, and then the polypeptides are collected. The term "host" in the present invention encompasses these animals and the like When animals are used, there are production systems using mammals and production systems using insects. As mammal, goats, pigs, sheep, mice, or cattle can be used (Vicki Glaser, SPECTRUM Biotechnology Applications, 1993).

For example, a target DNA is prepared in the form of a fusion gene with a gene encoding a polypeptide such as goat β casein that is uniquely produced in milk. Subsequently, a DNA fragment comprising the fusion gene is injected into a goat embryo and then the embryo is transplanted into a female goat. A target polypeptide can be obtained from milk that is produced by transgenic goats born from goats that have accepted such embryos or from the progenies of such transgenic goats. To increase the amount of milk containing polypeptides, which is produced by transgenic goats, an appropriate hormone may also be used for such transgenic goats (Ebert, K.M. et al., Bio/Technology (1994) 12, 699-702).

The thus obtained polypeptide can be isolated from within host cells or outside the cells (e g., media) and then purified as a substantially pure and uniform polypeptide. To separate and purify polypeptide, separation and purification methods that are generally used for polypeptide purification may be employed and are not specifically limited. For example, polypeptides can be separated and purified by the use of appropriate selection and combination of a chromatography column, a filter, ultrafiltration, salting-out, solvent precipitation, solvent extraction, distillation, immunoprecipitation, SDS-polyacrylamide gel electrophoresis, an isoelectric focusing method, dialysis, recrystallization, and the like.

Examples of chromatography include affinity chromatography, ion exchange chromatograph, hydrophobic chromatography, gel filtration, reverse phase chromatography, and adsorption chromatography (Strategies for Protein Purification and Characterization: A Laboratory Course Manual, Ed Daniel R. Marshak et al., Cold Spring Harbor Laboratory Press, 1996). These types of chromatography can be carried out using liquid-phase chromatography such as HPLC or FPLC.

In addition, when a proper protein modification enzyme is caused to act on polypeptides before or after purification, modification can be arbitrarily carried out or peptides can be partially removed. As protein modification enzymes, trypsin, chymotrypsin, lysylendopeptidase, protein kinase, and glucosidase, for example, are used

A known sequence can also be used for a gene encoding the H chain or the L chain of an antibody that is produced by the production method of the present invention. Furthermore, such gene can also be obtained by a method known by persons skilled in the art. For example, a gene encoding an antibody can be cloned and obtained from a hybridoma producing a monoclonal antibody or can also be obtained from an antibody library. Hybridomas can be produced basically using a known technique as described below. Specifically, a hybridoma can be produced by using as a sensitizing antigen a desired antigen or a cell that expresses a desired antigen, carrying out immunization according to a general immunization method using such antigen, fusing the thus obtained immunocyte with a known parent cell by a general cell fusion method, and then screening for a monoclonal antibody-producing cell (hybridoma) by a general screening method. The cDNA of an antibody variable region (V region) is synthesized from the mRNA of the thus obtained hybridoma using reverse transcriptase The cDNA is ligated to DNA encoding a desired antibody constant region (C region), so that a gene encoding the H chain or the L chain can be obtained A sensitizing antigen upon immunization is not specifically limited. For example, a target full-length protein or partial peptide can be used, Antigens can be prepared by a method known by persons skilled in the art. For example, antigens can be prepared according to a method using baculovirus (e.g., WO98/46777). Hybridomas can be produced according to, for example, Milstein et al's method (Kohler, G, and Milstein, C., Methods Enzymol. 1981, 73, 3-46) or the like. When the immunogenicity of an antigen is low, such antigen is bound to a macromolecule having immunogenicity, such as albumin, and then immunization is carried out.

Regarding an antibody library, many antibody libraries are already known. In addition, a production method for an antibody library is known Hence, persons skilled in the art can appropriately obtain an antibody library,

Antibodies that are expressed and produced as described above can be purified by a known method that is used for general protein purification. Antibodies can be separated and purified by appropriate selection or combination of, for example, an affinity column (eg, protein A column), a chromatography column, a filter, ultrafiltration, salting-cut, and dialysis (Antibodies: A Laboratory Manual, Ed Harlow and David Lane, Cold Spring Harbor Laboratory, 1988).

A known means can be used for measuring the antigen-binding activity of an antibody (Antibodies A Laboratory Manual, Ed Harlow, David Lane, Cold Spring Harbor Laboratory, 1988) For example, ELISA (enzyme-linked immunosorbent assay), EIA (enzyme-linked immunoassay), RIA (radioimmunoassay), or a fluorescent immunoassay can be used.

The sugar chain structure of protein produced using the cell of the present invention can be analyzed by a method described in a 2-dimensional sugar chain mapping method (Anal Biochem, 171, 73 (1988); Biochemical Experimental Methods 23-Methods for Studying Glycoprotein Sugar Chains, edited by Reiko Takahashi, Center for Academic Publications Japan (1989)). Moreover, sugar chains can also be analyzed by mass spectrometry such as MALDI-TOF-MS.

Furthermore, the stability, such as the thermostability, of a protein having no fucose added thereto that is produced using a cell of the present invention is equivalent to that of a protein to which fucose has been added.

Cytotoxic activity of antibody

Antibodies produced by the method of the present invention have enhanced cytotoxic activity.

Examples of cytotoxic activity in the present invention include antibody-dependent cell-mediated cytotoxicity (ADCC) activity and complement-dependent cytotoxicity (CDC) activity. In the present invention, CDC activity means cytotoxic activity of a complement system. ADCC activity means activity to damage a target cell. Specifically, when a specific antibody attaches to a cell surface antigen of a target cell, an Fcy receptor-retaining cell (e.g., an immunocyte) binds to the Fc portion of the antibody via an Fcγ receptor, damaging the target cell.

Whether or not an antibody has ADCC activity or has CDC activity can be determined by a known method (e.g., Current protocols in Immunology, Chapter 7, Immunologic studies in humans, Editor John E. Coligan et al., John Wiley & Sons, Inc., (1993)).

Specifically, effector cells, a complement solution, and target cells are prepared.

(1) Preparation of effector cells

A spleen is extracted from a CBA/N mouse or the like, and then spleen cells are separated in an RPMI1640 medium (produced by GIBCO). After washing with the same medium containing 10% fetal bovine serum (FBS, produced by HyClone), the cells are prepared to a concentration of $5 \times 10^6$/ml, thereby preparing effector cells..

(2) Preparation of a complement solution

Baby Rabbit Complement (produced by CEDARLANE LABORATORIES LIMITED) is diluted 10-fold in a 10% FBS-containing medium (produced by GIBCO), thereby preparing a complement solution.

(3) Preparation of target cells

Pancreatic cancer cell lines (e.g., AsPC-1 and Capan-2) are radiolabeled by culturing the cell lines with 0.2 mCi $^{51}$Cr-sodium chromate (produced by Amersham Pharmacia Biotech) in a 10% FBS-containing DMEM medium at 37°C for 1 hour After radiolabeling, the cells are washed three times in a 10% FBS-containing RPMI1640 medium and then prepared to a cell concentration of $2 \times 10^5$/ml, thereby preparing target cells

Subsequently, ADCC activity or CDC activity are determined. To determine ADCC activity, target cells and antibodies are added in amounts of 50 ml each to a 96-well U-bottomed plate (produced by Becton, Dickinson and Company) and are then allowed to react on ice for 15 minutes. Next, 100 μl of effector cells is added, followed by 4 hours of culture in a carbon dioxide gas incubator. The final antibody concentration is 0 or 10 μg/ml. After culture, 100 μl of the supernatant is collected, and then radioactivity is measured using a gamma counter (COBRAIIAUTO-GMMA, MODEL D5005, produced by Packard Instrument Company) Cytotoxic activity (%) can be calculated by $(A-C)/(B-C) \times 100$. "A" denotes radioactivity (cpm) in each sample, "B" denotes radioactivity (cpm) in a sample supplemented with 1% NP-40 (produced by NACALAI TESQUE, INC.), and "C" denotes radioactivity (cpm) in a sample containing only target cells

Furthermore, to determine CDC activity, target cells and anti-PepT antibodies are added in amounts of 50 μl each to a 96-well flat-bottomed plate (produced by Becton, Dickinson and Company) and are then allowed to react on ice for 15 minutes. Subsequently, 100 μl of a complement solution is added, followed by 4 hours of culture in a carbon dioxide gas incubator. The final antibody concentration is 0 or 3 gl/ml. After culture, 100 μl of the supernatant is collected, and then radioactivity is measured using a gamma counter. Cytotoxic activity can be calculated in a manner similar to that used for determination of ADCC activity.

For example, the ADCC activity of an antibody produced using an animal cell of the present invention having a disrupted fucose transporter gene is compared with that of an antibody produced using an animal cell of the same species having an undisrupted fucose transporter gene. Comparison is made by finding antibody concentrations that lead to 50% cytotoxic activity from an antibody concentration-cytotoxic activity (%) curve. An antibody concentration that leads to 50% cytotoxic activity of an antibody that is produced using an animal cell having a disrupted fucose transporter gene is 1/2, preferably 1/5, and further preferably 1/10 of the antibody concentration that leads to 50% cytotoxicity of an antibody that is produced using an animal cell having an undisrupted fucose transporter gene. That is, the ADCC activity of an antibody produced using an animal cell of the present invention having a disrupted fucose transporter gene is enhanced to a level 2 or more, preferably 5 or more, and further preferably 10 or more times greater than that of an antibody produced using an animal cell having an undisrupted fucose transporter gene

The present invention will be described in more detail below with reference to example However, the present invention is not limited to these examples.

Example 1 Disruption of a fucose transporter gene in CHO cells

1. Construction of targeting vectors

(1) Construction of a KO1 vector

A hygromycin resistance gene ($Hyg^r$) was prepared by PCR using pcDNA3.1/Hygro (Invitrogen Corporation) as a template and Hyg5-BH and Hyg3-NT as primers. A *Bam*H I and a TGCGC sequence were added to the 5' side of the initiation codon of $Hyg^r$, thereby preparing a sequence that was the same as that on the 5' region of the initiation codon of a fucose transporter genes. A *Not* I sequence was added to the 3' region comprising a SV40 polyA addition signal, thereby extracting $Hyg^r$.
Forward primer
Hyg5-BH 5'- GGA TCC TGC GCA TGA AAA AGC CTG AAC TCA CC -3' (SEQ ID NO: 3)
Reverse primer
Hyg3-NT 5'- GAG GCC GCC TAT TCC TTT GCC CTC GGA CG-3'(SEQ ID NO: 4)

A targeting vector ver 1 (hereinafter referred to as KO1 vector) for a fucose transporter knockout was constructed by inserting separately a 5' fragment (ranging from No. 2,780 *Sma* I to No 4,232 *Bam*H I of the nucleotide sequence shown in SEQ ID NO: 1) of a fucose transporter, a 3' fragment (ranging from No.4,284 to No.10,934 *Sac* I) of the fucose transporter, and a Hyg' fragment into a pMCIDT-A vector (Yagi T, Proc. Natl. Acad. Sci. U.S.A. vol. 87, pp. 9918-9922, 1990) (Fig, 1). The vector is charaterized by carrying promoter-less $Hyg^r$ unit. Thus, $Hyg^r$ is expressed by a promoter of the fucose transporter following homologous recombination. However, introduction of only one copy of a vector into cells by homologous recombination does not always result in the enough expression of $Hyg^r$ that provide resistance against hygromycin B in the cells. In addition, the KO1 vector was linealized by *Not* I and transfected into cells. With the use of the KO1 vector, the fucose transporter gene will lack 41 base pairs of exon 1 comprising the initiation codon and lose the relevant function.

(2) Preparation of pBSK-pgk-1-$Hyg^r$

A mouse pgk-1 gene promoter was prepated from pKJ2 vector (Popo H, Biochemical Genetics vol. 28, pp 299-308, 1990) by *Eco*R I-*Pst* I digestion and then cloned into the *Eco*R I-*Pst* I site of pBluescript (Stratagene Corporation), thereby preparing pBSK-pgk-1. A Hygromycin resistance gene ($Hyg^r$) was prepared by PCR using pcDNA3 1/Hygro (Invitrogen Corporation) as a template and Hyg5-AV and Hyg3-BH as primers. Thus, an Eco T221 and a Kozak sequence were added to the 5' region of $Hyg^r$. Furthermore, a *Bartr*H I sequence was added to the 3' region comprising a SV40 polyA addition signal, thereby extracting $Hyg^r$.
Forward primer
Hyg5-AV 5'- ATG CAT GCC ACC ATG AAA AAG CCT GAA CTC ACC -3' (SEQ ID NO: 5)
Reverse primer
Hyg3-BH 5'- GGA TCC CAG GCT TTA CAG TTT ATG CTT C -3' (SEQ ID NO: 6)
The $Hyg^r$ (EcoT22 I-*Bam*H I) fragment was inserted into the *Pst* I-*Bam*H I site of pBSK-pgk-1, thereby preparing pBSK-pgk-1-$Hyg^r$. (3) Preparation of KO2 vector
A targeting vector ver.2 (hereinafter referred to as the KO2 vector) for a fucose transporter knockout was constructed by inserting separately a 5'region (ranging from No. 2,780 *Sma* I to No. 4,232 *Bam*H I in the nucleotide sequence shown in SEQ ID NO: 1), a 3'region (ranging from No. 4,284 to No 10,934 *Sac* I) of the fucose transporter, and pgk-1-$Hyg^r$ fragments into a pMCIDT-A vector (Fig. 1). Unlike the KO1 vector, a pgk-1 gene promoter was added to $Hyg^r$ of the KO2 vector Introduction of even only one copy of a vector into cells via homologous recombination can cause the cells to acquire resistance to hygromycin B. In addition, the KO2 vector was linealized by *Not* I and then transfected into cells. With the use of the KO2 vector, the fucose transporter may lack 46 base pairs of exon 1 comprising the initiation codon and lose the relevant function. (4) Preparation of pBSK-pgk-1-$Puro^r$
A pPUR vector (BD Biosciences) was digested with *Pst* I and *Bam*H I. The $Puro^r$ fragment was inserted into the *Pst* I-*Bam*H I site of pBSK-pgk-1, thereby preparing pBSK-pgk-1-$Puro^r$. (5) Preparation of KO3 vector
A targeting vector ver. 3 (hereinafter referred to as the KO3 vector) for a fucose transporter knockout was constructed by separately inserting a 5' region (ranging from No. 2,780 *Sma* I to No. 4,232 *Bam*H I of the nucleotide sequence shown in SEQ ID NO: 1), a 3' region (ranging from No. 4,284 to No.. 10,934 *Sac* I) of the fucose transporter, and pgk-1-$Puro^r$ fragments into a pMCIDT-A vector (Fig. 1). In addition, a sequence, to which the following primer for screening binds had been previously added to the 3' end of pgk-1-$Puro^r$. In addition, the KO3 vector was linealized with *Not* I and then transfected into cells. With the use of the KO3 vector, the fucose transporter may lack 46 base pairs of exon 1 comprising the initiation codon and lose the relevant function.
Reverse primer
RSGR. A 5'-GCT GTC TGG AGT ACT GTG CAT CTG C -3' (SEQ ID NO: 7)
Knockout of the fucose transporter gene was attempted using the above 3 types of targeting vector

2. Vector transfection into CHO cells

HT Supplement (100 ×) (Invitrogen Corporation cat 11067-030) and penicillin streptomycin (Invitrogen Corporation cat. 15140-122) were each added to CHO-S-SFMII HT-(Invitrogen Corporation cat 12052-098) in a volume one-hundredth of the volume of CHO-S-SFMII HT- The solution was used as medium for culture (hereinafter referred to as SFMII (+)). The DXBII cell line of CHO cells was maintained and cells after gene transfer were also cultured in SFMII (+) $8 \times 10^6$ CHO cells were suspended in 0.8 mL of Dulbecco's phosphate buffer (hereinafter abbreviated as "PBS"; Invitrogen Corporation cat. 14190-144). 30 μg of the targeting vector was added to the cell suspension. The cell suspension was transferred to a Gene Pulser Cuvette (4 mm) (Bio-Rad Laboratories Inc. cat. 1652088). After the suspension had been allowed to stand on ice for 10 minutes, the cells were electroporated using GENE-PULSER. II (Bio-Rad Laboratories Inc. code No. 340BR) at setting of 1.5 kV, 25 μFD. After vector transfection, the cells were suspended in 200 ml of SFMII(+) medium. The cell suspension was then dispensed into twenty 96-well flat-bottomed plates (IWAKI cat-1860-096) at 100 μl/well The cells in the plates were cultured in a $CO_2$ incubator for 24 hours at 37°C, followed by addition of a drug.

3. First step of knockout

The KO1 vector or the KO2 vector was transfected into CHO cells. At 24 hours after vector transfection, selection was performed using hygromycin B (Invitrogen Corporation cat. 10687-010). Hygromycin B was added to SPMII(+) to a concentration of 0.3 mg/ml and then added to transfected cells at 100 μl/well

4. Screening of homologous recombinants by PCR

(1) Preparation of samples for PCR

Homologous recombinants were screened by the PCR method. CHO cells to be used in screening were cultured in a 96-well flat-bottomed plate. After removal of culture supernatant, a buffer for cell lysis was added at 50 μl/well. After incubation at 55°C for 2 hours, proteinase K was inactivated by subsequent heating at 95°C for 15 minutes, so as to prepare a template for PCR. I he buffer for cell lysis was composed of 5 μl of 10 × LA buffer II (attached to TaKaRa LA Taq), 2 5 μl of 10% NP-40 (Roche cat. 1 332 473), 4 μl of proteinase K (20 mg/ml and TaKaRa cat. 9033), and 38.5 μl of distilled water (Nacalai Tesque Inc cat. 36421-35) per well.

(2) PCR conditions

A PCR reaction mixture was determined to contain 1 μl of each of the above PCR samples, 5 μl of 10 × LA buffer II, 5 μl of $MgCl_2$ (25 mM), 5 μl of dNTP (25 mM), 2 μl of each primer (10 μM each), 0.5 μl of LA Taq (5 IU/μl and cat. RR002B), and 29.5 μl of distilled water (total 50 μl). For screening of the cells in which the KO1 vector had been transected, TP-F4 and THygro-R1 were used as PCR primers. For screening of the cells in which the KO2 vector had been introduced, TP-F4 and THygro-F1 were used as PCR primer.

Moreover, PCR conditions employed for the cells into which the KO1 vector had been transfected consisted of pre-heating at 95°C four 1 minute, 40 amplification cycles (each cycle consisting of 95°C for 30 seconds, 60°C for 30 seconds, and 60°C for 2 minutes), and additional heating at 72°C for 7 minutes. PCR conditions employed for screening of the cells into which the KO2 vector had been transfected consisted of preheating at 95°C for 1 minute, 40 amplification cycles (each cycle consisting of 95°C for 30 seconds and 70°C for 3 minutes), and additional heating at 70°C for 7 minutes.

Primers are as shown below. In cell samples of homologous recombinants, an approximately 1.6 kb DNA in the case of the KO1 vector and an approximately 2.0-kb DNA in the case of the KO2 vector were amplified, Regarding the primers, TP-F4 was set in the 5' region of fucose transporter genome outside the vector. THygro-F1 and THygro-R1 were arranged within $Hyg^r$ in the vector.
Forward primer (KO1, KO2)
TP-F4 5'-GGAATG CAG CTT CCT CAA GGG ACT CGC-3' (SEQ ID NO: 8)
Reverse primer (KO1)
THygro-R1 5'-TGC ATC AGG TCG GAG ACG CTG TCG AAC-3'(SEQ ID NO: 9)
Reverse primer (KO2)
THygro-F1 5'-GCA CTC GTC CGA GGG CAA AGG AAT AGC-3' (SEQ ID NO: 10)

5. PCR screening results

918 cells into which the KO1 vector had been transfected were analyzed. Of these cells, 1 cell was considered to be a homologous recombinants (with homologous recombination efficiency of approximately 0.1%). Furthermore, 537 cells into which the KO2 vector had been transfected were analyzed. Of these cells, 17 cells were considered to be homologous recombinants (with homologous recombination efficiency of approximately 3.2%)

6. Southern blot analysis

Such confirmation was also performed by the Southern blot method, 10 μg of genomic DNA was prepared from the cultured cells according to a standard method, and then Southern blot was performed. A 387-bp probe was prepared from the region ranging from No 2,113 to No. 2,500 of the nucleotide sequence shown in SEQ ID NO: 1 by the PCR method using the following 2 primers. The thus prepared probe was used for confirmation by the Southern blot method. The genomic DNA was digested with *Bgl* II.
Forward primer
Bgl-F: 5'-TGT GCT GGG AAT TGAACC CAG GAC-3'(SEQ ID NO: 11)
Reverse primer
Bgl-R: 5'-CTA CTT GTC TGT GCT TTC TTC C-3' (SEQ ID NO: 12)

As a result of digestion with *Bgl* II, an approximately 30-kb band was detected from the chromosome of the fucose transporter, an approximately 4.6-kb band was detected from the chromosome into which the KO1 vector had been inserted after homologous recombination, and an approximately 5.0-kb band was detected from the chromosome into which the KO2 vector had been inserted after homologous recombination. Fig. 2 shows the band patterns after digestion with Bgl II following the knockout of the first step. Fig 3 shows data of Southern blot 1 cell line of homologous recombinants using the KO1 vector and 7 cell lines of homologous recombinants using the KO2 vector were used for this experiment The only one cell line obtained from the KO1-vector transfection was named 5C1. Subsequent analysis revealed that this cell line is composed of multiple cell populations. Cloning was performed by the limiting dilution method for use in the subsequent experiments. Furthermore, the cell line obtained from the KO2-vector transfection was named 6E2.

7. Second step of knock out

3 types of vector were used for homologous recombinants obtained from transfection, of the KO1 vector or the KO2 vector, so as to attempt to establish cell lines completely lacking the fucose transporter gene. Combinations of such vectors with cells are as follows: method 1 (KO2 vector and 5C1 cells (KO1)); method 2 (KO2 vector and 6E2 cells (KO2)); and method 3 (KO3 vector and 6E2 cells (KO2)). The vectors were separately transfected into each cell type At 24 hours after vector introduction, selection was initiated using hygromycin B or puromycin (Nacalai Tesque Inc., cat. 29455-12). Hygromycin B was used in the case of method 1 at a final concentration of 1 mg/ml and in the case of method 2 at a final concentration of 7 mg/ml. Further, in the case of method 3, hygromycin B was added to a final concentration of 0.15 mg/ml and puromycin was added to a final concentration of 8 μg/ml.

8. Screening of homologous recombinants by PCR

Samples were prepared as described above. Screening in the case of method 1 was performed by PCR to detect signals of homologous recombination by both KO1 vector and KO2 vector. For screening in the case of method 2, the following PCR primers were designed TPS-F1 was set in the Nos 3,924-to-3,950 legion and SHygro-R1 was set in the Nos. 4,248-to-4,274 region of the nucleotide sequence shown in SEQ ID NO: 1. With the use of these PCR primers, a gene region of 350 bp of the fucose transporter, which is deficient due to the KO2 vector, is amplified. Therefore, for PCR screening in the case of method 2, cells in which such 350-bp region had not been amplified were determined to be cells completely lacking the fucose transporter gene. PCR. conditions consisted of preheating at 95°C for I minute, 35 amplification cycles (each cycle consisting of 95°C for 30 seconds and 70°C for I mimite), and additional heating at 70°C for 7 minutes.

Forward primer

TPS-F1: 5'-CTC GAC TCG TCC CTA TTA GGC AAC AGC-3' (SEQ ID NO: 13)

Reverse primer

SHygro-R1: 5'-TCA GAG GCA GTG GAG CCT CCA GTC AGC-3' (SEQ ID NO: 14)

In the case of method 3, TP-F4 was used as a forward primer and RSGR-A was used as a reverse primer. PCR conditions consisted of preheating at 95°C for I minute, 35 amplification cycles (each cycle consisting of 95°C for 30 seconds, 60°C for 30 seconds, and 72°C for 2 minutes), and additional heating at 72°C for 7 minutes. In the cell samples of homologous recombinants in the case of the KO3 vector, an approximately 1.6-kb DNA was amplified As a result of the PCR, homologous recombination by the KO3 vector was confirmed. It was also confirmed that the region of homologous recombination by the KO2 vector was remained.

9. Results of PCR screening

In the case of method 1, 616 cells were analyzed. Of these cells, 18 cells were thought to be homologous recombinants (homologous recombination efficiency: 2.9%) (Fig 4). In the case of method 2, 524 cells were analyzed Of these cells, 2 cells were thought to be homologous recombinants (homologous recombination efficiency: approximately 0.4%) Furthermore, in the case of method 3, 382 cells were analyzed. Of these cells, 7 cells were thought to be homologous recombinant (homologous recombination efficiency: approximately 1.8%).

10. Southern blot analysis

Southern blot analysis was performed according to the above methods. As a result, among the analyzed cells, one cell completely lacking the fucose transporter gene was discovered In the case of knockout of the first step, PCR analysis results were consistent with Southern blot analysis results. Regarding knockout of the second step, the two were not consistent with each other. Possible causes of such results are: 1. a mixture of homologous recombinants of either KO1 alone or KO2 alone in the case of method 1; 2 presence of not a pair of fucose transporter genes (2 fucose transporter genes), but a plural number of pairs of fucose transporter genes (or 3 or more fucose transporter genes); and 3. increased copy number of the fucose transporter gene that had remained after subculture of cell lines in which the knockout of the first step had been successfully performed

11. Fucose expression analysis

Furthermore, fucose expression in 26 cells that were determined as homologous recombinant by PCR was analyzed. $1\times10^6$ cells were stained with 100 μl of PBS (hereinafter, referred to as FACS solution) containing 5 μg/mL Lens culinaris Agglutinin, FITC Conjugate (Vector Laboratories cat. FL-1041), 25% FBS, and 0.02% sodium azide for 1 hour during cooling with ice. Subsequently, the cells were washed three times with FACS solution and then analyzed by FACS Calibur (Becton, Dickinson and Company). As a result, it was revealed that only the cells completely lacking the fucose transporter gene as determined by Southern blot analysis exerted decreased fucose expression levels Figs 5 to 7 show the results of Southern blot analysis performed for the obtained clones (Fig 5) and the results of determining fucose expression levels Fig 6 shows the results for wild/KO. Fig. 7 shows the results for KO/KO.

The following was demonstrated by the above results.

Only one cell line completely lacked the fucose transporter gene and the number of such cells screened for was 616 Specifically, homologous recombination frequency was approximately as low as 0.16%. Regarding the knockout of the second step, there are several possible reasons for the inconsistency between PCR analysis results and Southern blot analysis results, as described above. However, in the case of method 3, since selection was performed using 2 types of drug, it was unlikely that the obtained cell lines would contain homologous recombinants by both the KO2 vector alone and the KO3 vector alone.. Moreover, it was unlikely that any of the other cell lines determined as homologous recombinants by PCR would be composed of a plurality of cell populations The presence of 3 or more fucose transporter genes as described above makes targeting in cells significantly difficult. In such case, it was concluded that homologous recombinants may be impossible to obtain unless the KO1 vector, with which $Hyg^r$ is weakly expressed, is used and screening would be performed many times.

Example 2 Construction of antibody expression vectors

A humanized anti-HM1.24 antibody expression vector, AHM(I)/N5KG1 (WO2005/014651), was digested with *Ssp* I. pCXND3/hGC33/K7Arg that is a vector for expression of an altered antibody gene (reference examples 1 to 3 described later), in which humanized amti-GPC3 antibody H chain is of version K. and Asn33 of the L chain had been substituted with Arg, was digested with *Pvu* I. These vectors were then used for experiments.

Example 3 Establishment of antibody-producing cells

Hygromycin B was prepared in SFMII (+) medium at a final concentration of 1 mg/ml and the fucose transporter-deficient cell line (FT-KO cell, clone name of 3F2) obtained in Example 1 was maintained $8 \times 10^6$ 3F2 cells were suspended in 0.8 mL of a Dulbecco's phosphate buffer 25 μg of each antibody expression vector was added to the cell suspension and then the cell suspension was transferred to a Gene Pulser Cuvette. After the cubette was allowed to stand on ice for 10 minutes, each vector was introduced into the cells by an electroporation method using GENE-PULSER II under conditions of 1.5 kV and 25 μFD. After introduction of the vector, the cells were suspended in 40 mL of SFMII (+) medium and then the suspended cells were inoculated into a 96-well flat bottomed plate (IWAKI) at 100 μl/well. The cells in the plate were cultured within a $CO_2$ incubator for 24 hours at 37°C, and then Geneticin (Invitrogen Corporation, cat 10131-027) was added at a final concentration of 0.5 mg/mL The amounts of antibodies produced by cells that had become resistant to the drug were determined. Thus, a humanized anti-HM1 24 antibody-producing cell line and a humanized anti-GPC3 antibody-producing cell line were each established.

Example 4 Antibody purification

The culture supernatant of each antibody-expressing cell line was collected and then Hitrap rProtein A (Pharmacia CAT# 17-5080-01) was charged with the culture supernatant using a P-1 pump (Pharmacia) The resultant was washed using a binding buffer (20 mM Sodium phosphate (pH 7.0)) and then eluted with an elution buffer (0.1M Glycin-HCl (pH 2.7)). The eluate was immediately neutralized with a neutralizing buffer (1M Tris-HCl (pH 9.0)). Eluted fractions of the antibodies were selected using DC protein assay (BIO-RAD CAT# 500-0111) and then pooled Each resultant was concentrated to approximately 2 mL using Centriprep-YM10 (Millipore CAT# 4304) Next, the resultants were separated by gel filtration using Superdex 200 26/60 (Pharmacia) that had been equilibrated with 20 mM acetate buffer and 150 mM NaCl (pH6.0) Monomer fraction peaks were collected, concentrated using Centriprep-YM10, subjected to filtration using a MILLEX-GW 022 μm Filter Unit (Millipore CAT# SLGV 013SL), and then stored at 4°C. Concentrations of the thus purified antibodies were obtained through measurement of absorbances at 280 nm and the following conversion based on molar extinction coefficients

Example 5 *In vitro* ADCC activity of a humanized anti-HM1.24 antibody produced by FT-KO cells

1. Preparation of a human PBMC solution

A peripheral blood sample (heparinized blood sample) was obtained from a healthy subject, diluted two-fold using PBS(-), and then layered on Ficoll-PaqueTM PLUS (Amersham Biosciences). The resultant was centrifuged (500×g, 30 minutes, 20°C) and then the intermediate layer that was a mononuclear cell fraction was isolated. The fraction was washed 3 times and then suspended in 10% FBS/RPMI, thereby preparing a human PBMC solution.

2. Preparation of target cells

ARH-77 cells (ATCC) cultured in 10% FBS/RPMI1640 medium were removed by pipetting from the dish, centrifuged within a tube, and then suspended in 100 μL of medium, 5.55 MBq of Cr-51 was added to the medium and then cultured in a 5% carbon dioxide gas incubator at 37°C for 1 hour The cells were washed twice with medium and then 10% FBS/RPMI1640 medium was added to the washed cells. The cells were dispensed into a 96-well U-bottomed plate (Falcon) at $1\times10^4$ cells/well, thereby preparing target cells.

3. Chromium release test (ADCC activity)

50 μL of an antibody solution prepared at various concentrations was added to the target cells.. After 15 minutes of reaction at room temperature, 100 μL of a human PBMC solution was added ($5\times10^5$ cells/well) The cells were cultured in a 5% carbon dioxide gas incubator at 37°C for 4 hours After culture, the plates were subjected to centrifugation and then radioactivity in 100 μL of each culture supernatant was measured using a gamma counter. A specific chromium release percentage was found via the following formula.

$$\text{Specific chromium release percentage (\%)} = (A - C) \times 100 / (B-C)$$

"A" indicates the mean radioactivity (cpm) in each well, "B" indicates the mean radioactivity (cpm) in a well wherein 100 μL of a 2% NP-40 aqueous solution (Nonidet P-40, Code No. 252-23, NACALAI TESQUE, INC.) and 50 μL of 10% FBS/RPMI medium were added to target cells, and "C" indicates the mean radioactivity (cpm) in a well wherein 150 μL of 10% FBS/RPMI medium was added to target cells. The test was performed in tripricate and then means and standard deviations were calculated for ADCC activity (%).

Fig. 8 shows the ADCC activity of the anti-HM1.24 antibodies determined using human PBMC Open squares indicate the activity of the humanized anti-HM1.24 antibody produced by wild type CHO cells and closed circles indicate the activity of the humanized anti-HM1.24 antibody produced by FT-KO cells. The humanized anti-HM1.24 antibody produced by FT-KO cells exerted stronger ADCC activity than that of the humanized amti-HM1.24 antibody produced by the wild type CHO cells. Thus, it was revealed that the ADCC activity of such antibody produced by FT-KO cells is enhanced.

Example 6 *In vitro* ADCC activity of humanized anti-GPC3 antibody produced by FT-KO cells

1 Preparation of human PBMC solution

A peripheral blood sample (heparinized blood sample) was obtained from a healthy subject, diluted 2-fold with PBS(-), and then layered on Frcoll-PaqueTM PLUS (Amersham Biosciences). The resultant was centrifuged (500 × g, 30 minutes, 20°C) and then an intermediate layer that was a mononuclear cell fraction was isolated After 3 times of washing, the resultant was suspended in 10% FBS/RPMI, thereby preparing a human PBMC solution.

2. Preparation of target cells

HuH-7 cells (Health Science Research Resources Bank) cultured in 10% FBS/D-MEM medium were removed from a dish using a Cell Dissociation Buffer (Invitrogen Corporation). The cells were dispensed into a 96-well U-bottomed plate (Falcon) at 1×$10^4$ cells/well and then cultured for I day. After culture, 5.55 MBq of Cr-51 was added to the cells, followed by I hour of culture in a 5% carbon dioxide gas incubator at 37°C. The cells were washed once with medium and then 50 μL of 10% FBS/D-MEM medium was added to the cells, thereby preparing target cells.

3. Chromium release test (ADCC activity)

Preparation of a human PBMC solution and a chromium release test were performed according to the methods described in Example 1. Fig. 9 shows the ADCC activity of humanized anti-GPC3 antibodies determined using human PBMC. Open circles indicate the activity of the humanized anti-GPC3 antibody produced by wild type CHO cells and closed triangles indicate the activity of the humanized anti-GPC3 antibody produced by FT-KO cells. The humanized anti-GPC3 antibody produced by FT-KO cells exerted stronger ADCC activity than that of the humanized anti-GPC3 antibody produced by wild type CHO cells. Thus, it was revealed that the ADCC activity of such antibody produced by FT-KO cells is enhanced.

Example 7 Analysis of a humanized anti-HM1.24 antibody sugar chain produced by FT-KO cells

1. Preparation of 2-aminobenzamide-labeled sugar chains (2-AB-labeled sugar chains)

N-Glycosidase F (Roche diagnostics) was caused to act on the antibody of the present invention produced by FT-KO cells and an antibody produced by CHO cells as a control sample. Sugar chains were then liberated from proteins (Weitzhandler M et al.., Journal of Pharmaceutical Sciences 83: 12 (1994), 1670-1675) After removal of proteins using ethanol (Schenk B et al, The Journal of Clinical Investigation 108: 11 (2001), 1687-1695), the resultants were concentrated to dryness and then fluorescence-labeled using 2-aminopyridine (Bigge I C. et al., Analytical Biochemistry 230: 2 (1995), 229-238). The thus obtained 2-AB-labeled sugar chains were subjected to solid phase extraction using a cellulose cartridge for removing the reagent. The resultants were then centrifuged and concentrated, thereby purifying the 2-AB-labeled sugar chains.

2. Analysis of purified 2-AB-labeled sugar chains by normal phase HPLC

With the method in the previous section, 2-AB-labeled sugar chains were prepared using the antibody of the present invention produced by FT-KO cells and the antibody produced by CHO cells as a control sample Normal phase HPLC analysis was performed using an amide column (TSKgel Amide-80 produced by TOSOH Corporation) and then the resulting chromatograms were compared. The antibody produced by CHO cells contained G(0)-Fuc in a small amount. The proportion of G(0)-Fuc in an agalactosyl sugar chain (G(0)+G(0)-Fuc) was approximately 1 5% On the other hand, G(0)-Fuc contained in the antibody produced by FT-KO cells accounted for 90% or more (Fig. 10 and Table 1).

Table 1

| Relative percentage of each sugar chain inferred from analysis of agalactosyl 2-AB-labeled sugar chain by normal phase HPLC | | |
|---|---|---|
| Sugar chain name | CHO | FTKO |
| G(0)-Fuc | 1 5% | 92.4% |
| G(0) | 98.5% | 7.6% |

Example 8 Analysis of humanized anti-GPC3 antibody sugar chains produced by FT-KO cells

1. Preparation of 2-aminobenzmide-labeled sugar chains (2-AB-labeled sugar chains)

N-Glycosidase F (Roche Diagnostics) was caused to act on the antibodies of the present invention produced by FT-KO cells and an antibody produced by CHO cells as a control sample. Sugar chains were liberated from proteins (Weitzhandler M. et al., Journal of Pharmaceutical Sciences 83: 12 (1994), 1670-1675). After removal of proteins using ethanol (Schenk B. et al., The Journal of Clinical Investigation 108: 11 (2001), 1687-1695), the resultants were concentrated to dryness and then fluorescence labeled with 2-ammopyridine (Bigge J. C. et al., Analytical Biochemistry 230: 2 (1995), 229-238). The thus obtained 2-AB-labeled sugar chains were subjected to solid phase extraction using a cellulose cartridge for removing the reagent. The resultants were then centrifuged and concentrated, thereby purifying the 2-AB-labeled sugar chains. Next, β-Galactosidase (SEIKAGAKU Corporation) was caused to act on the purified 2-AB-labeled sugar chains, thereby preparing agalactosyl 2-AB-labeled sugar chains.

2. Analysis of agalactosyl 2-AB-labeled sugar chains by normal phase HPLC

Agalactosyl 2-AB-labeled sugar chains were prepared by the method described in the above section using the antibodies of the present invention produced by FT-KO cells and the antibody produced by CHO cells as a control sample- Normal phase HPLC analysis was performed using an amide column (produced by TOSOH Corporation: TSKgel Amide-80) and then the resulting chromatograms were compared. In the case of the antibody produced by CHO cells, G(0) was contained as a main component; and the peak area proportion of G(0)-Fuc that had not experienced addition of fucose thereto was approximately 4%. On the other hand, in the case of the antibodies produced by FT-KO cells, G (0)-Fuc was contained as a main component; and the peak area proposition of G(0)-Fuc was 90% or more for all the FT-KO cell lints (Fig-11 and Table 2) Fig. 12 shows the inferred structures of peak G(0) and G(0)-Fuc.

Table 2

| Relative percentage of each sugar chain inferred from analysis of agalactosyl 2-AB-labeled sugar chain by normal phase HPLC | | | | |
|---|---|---|---|---|
| Sugar chain name | CHO | FT-KO-a | FT-KO-b | FT-KO-c |
| G(0)-Fuc | 4.0% | 92.4% | 92.5% | 93.2% |
| G(0) | 96.0% | 7.6% | 7.5% | 6.8% |

Example 9 Analysis of thermostability of a humanized anti-GPC3 antibody produced by FT-KO cells

1. Preparation of sample solutions for DSC measurement

A 20 mol/L sodium acetate buffer solution (pH 6.0) containing 200 mmol/L sodium chloride was used as a dialysis external solution. A dialysis membrane containing an antibody solution sealed therein in an amount equivalent to 700 μg was immersed in the solution for 24 hours for dialysis, thereby preparing a sample solution.

2. Thermal denaturation temperature measurement by DSC

The sample solution and a reference solution (dialysis external solution) were sufficiently deaerated. Each of these solutions was then sealed in a calorimeter cell, followed by sufficient thermal equilibration at 20°C. Next, DSC scanning was performed at a temperature between 20°C and 100°C and a scanning speed of approximately 1K/minute The resulting denaturation peaks are shown in the form of temperature functions (Fig 13). In this measurement, the antibody produced by CHO cells and the antibody produced by FT-KO cells were equivalent to each other in terms of thermal denaturation temperature.

Reference example 1 Production of anti-GPC3 antibody

1. Human glypican 3(GPC3) cDNA cloning

A full-length cDNA encoding human GPC3 was amplified by a PCR reaction using 1st strand cDNA as a template prepared from a colon cancer cell line Caco2 by a standard method and an Advantage2 kit (CLONTECH Laboratories, Inc.) Specifically, 50 μl of a reaction solution containing 2 μl of Caco2-derived cDNA, 1 μl of a sense primer (GATATC-ATGGCCGGGACCGTGCGCACCGCGT:SEQ ID NO: 15), 1 μl of an antisense primer (GCTAGC-TCAGTGCACCAGGAAGAAGAAGCAC:SEQ ID NO: 16), 5 μl of an Advantage2 10 × PCR buffer, 8 μl of dNTP mix (1.25 mM), and 1.0 μl of Advantage polymerase Mix was subjected to 35 cycles of the PCR reaction, each cycle consisting of 94°C for 1 minute, 63°C for 30 seconds, and 68°C for 3 minutes An amplified product obtained by the PCR reaction was inserted into TA vector pGEM-T Easy using pGEM-T Easy Vector System I (Promega Corporation). The sequence was confirmed using an ABI3100 DNA sequencer and then cDNA encoding full-length human GPC3 was isolated. The sequence represented by SEQ ID NO: 17 indicates the nucleotide sequence of the human GPC3 gene. The sequence represented by SEQ ID NO: 18 indicates the amino acid sequence of the human GPC3 protein.

2. Production of soluble GPC3

As an immune protein for the production of anti-GPC3 antibodies, a soluble GPC3 protein lacking a C-terminal hydrophobic region (564-580 amino acids) was produced..

PCR was performed using the full-length human GPC3 cDNA as a template, an antisense primer (ATA GAA TTC CAC CAT GGC CGG GAC CGT GCG C: SEQ ID NO: 19) and a sense primer (ATA GGA TCC CTT CAG CGG GGA ATG AAC GTT C: SEQ ID NO: 20) to which an *Eco*R I recognition sequence and a Kozak sequence had been added. The thus obtained PCR fragment (1711 bp) was cloned into pCXND2-Flag pCXND2-Flag was designed so that a DHFR gene expression site of pCHOI (Hirata et al., FEBS letter 1994; 356; 244-248) was inserted into the *Hin*d III site in pCXN2 (Niwa et al., Gene 1991; 108; 193-199) and so that a Flag tag sequence was added downstream of the multicloning site for expression of a Flag-tag-added protein The thus prepared expression plasmid DNA was transfected into a CHO cell line DXB11. Through selection with 500 μg/mL Geneticin, a CHO cell line expressing soluble GPC3 at high levels was obtained. Large-scale culture of the CHO cell line expressing soluble GPC3 at high levels was performed using a 1700-$cm^2$ roller bottle.. The culture supernatant was collected and then purified. DEAE sepharose Fast Flow (Amersham Biosciences) was charged with the culture supernatant. After washing, elution was performed using a buffer containing 500 mM NaCl. Next, affinity purification was performed using Anti-Flag M2 agarose affinity gel (SIGMA). Elution was performed using 200 μg/mL FLAG peptide. After concentration using Centriprep-10 (Millipore corporation), gel filtration was performed using Superdex 200 HR 10/30 (Amersham Biosciences), so as to eliminate the FLAG peptide. Finally, the resultant was concentrated using a DEAE sepharose Fast Flow column and at the same time elution was performed using PBS (containing 500 mM NaCl) not containing Tween20, thereby performing buffer substitution.

3. Preparation of a soluble GPC3 core protein

GPC3 forms a macromolecule as a result of modification by heparan sulfate. To eliminate antibodies against heparan sulfate upon screening for anti-GPC3 antibodies, a soluble GPC3 core protein in which point mutation had been introduced at a heparan sulfate addition site was prepared and then used for screening.

cDNA in which Ser at position 495 and Ser at position 509 had been substituted with Ala was prepared by an assembly PCR method using the above soluble GPC3 (1-563) as a template. At this time, a primer was designed so that an His tag was added to the C-terminus. The thus obtained cDNA was cloned into a pCXND3 vector pCXND3 was constructed by inserting the DHFR gene expression site in pCHOI into the *Hin*d III site in pCXN2 The thus prepared expression plasmid DNA was transfected into a DXB11 cell line. Through selection using 500 μg/mL Geneticin, a CHO cell line expressing the soluble GPC3 core protein at a high level was obtained.

Large-scale culture was performed using a 1700-$cm^2$ roller bottle. The culture supernatant was collected and then purified. Q sepharose Fast Flow (Amersham Biosciences) was charged with the culture supernatant. After washing, elution was performed using phosphate buffer containing 500 mM NaCl. Next, affinity purification was performed using Chelating sepharose Fast Flow (Amersham Biosciences). Gradient elution was performed using 10 mM to 150 mM imidazole. Finally, the resultant was concentrated using Q sepharose Fast Flow and then eluted using a phosphate buffer containing 500 mM NaCl,

As a result of SDS polyacrylamide gel electrophoresis under reduction conditions, 3 bands (70 kDa, 40 kDa, and 30 kDa) were obtained. Amino acid sequencing was performed using an ABI492 protein sequencer (Applied Biosystems). As a result, a 30-kDa band matched an amino acid sequence starting from position 359 or position 375 of GPC3. Thus, it was revealed that GPC3 had been digested between Arg358 and Ser359 or between Lys374 and Va1375 so as to be divided into a 40-kDa, N-terminal fragment and a 30-kDa C-terminal fragment.

4 Preparation of CHO cells expressing full-length human GPC3

To obtain a cell line for evaluation of binding activity using flow cytometry, a CHO cell expressing full-length GPC3 was established.

10 μg of full-length human GPC3 gene expression vector was mixed with 60 μL of StipeiFect (QIAGEN), thereby forming a complex. The complex was added to the CHO cell line DXB11, so that gene transfer was performed After 24 hours of culture using a $CO_2$ incubator, selection was initiated using αMEM (GIBCO BRL) containing Geneticin at a final concentration of 0.5 mg/mL and 10% FBS The thus obtained Geneticin-resistant colonies were collected, and then cloning of the cells was performed by a limiting dilution method. Each cell clone was solubilized, Full-length human GPC3 expression was confirmed by Western blotting using an anti-GPC3 antibody. Thus, a cell line stably expressing full-length human GPC3 was obtained.

5. Evaluation of binding activity by ELISA

The soluble GPC3 core protein was diluted to 1 μg/mL using a coating buffer (0.1 mol/L $NaHCO_3$ (pH 9.6), 0,02% (w/v) $NaN_3$). The diluted protein was added to an immunoplate and then allowed to stand at 4°C overnight for coating. Blocking treatment was performed using a dilution buffer (50 mmol/L Tris-HCl (pH 8.1), 1 mmol/L $MgCl_2$, 150 mmol/L NaCl, 0.05% (v/v) Tween 20, 0.02% (w/v) $NaN_3$, and 1% (w/v) BSA). An anti-GPC3 antibody was added and then the resultant was allowed to stand at room temperature for 1 hour. After washing with a rinse buffer (0.05% (v/v) Tween 20, PBS), an anti-mouse IgG antibody (ZYMED Laboratories) labeled with alkaline phosphatase was added and then the resultant was allowed to stand at room temperature for 1 hour After washing with the rinse buffer, SIGMA104 (SIGMA) diluted to 1 mg/mL in a substrate buffer (50 mmol/L $NaHCO_3$ (pH 9.8) and 10 mmol/L $MgCl_2$) was added, followed by 1 hour of color development at room temperature. Absorbance (405 nm and reference wavelength of 655 nm) was then measured using a Benchmark Plus (BIO-RAD).

6. Immunization with soluble GPC3 and selection of hybridomas

Human GPC3 and mouse GPC3 show homology as high as 94% at the amino acid level. Hence, based on the assumption that anti-GPC3 antibody may be impossible to obtain via immunization of normal mice with GPC3, mice with autoimmune disease, MRL/MpJUmmCrj-lpr/lpr mice (hereinafter referred to as MRL/lpr mice, purchased from CHARLES RIVER LABORATORIES JAPAN, INC.), were used as animals to be immnized. Immunization was initiated for 7-week-old mice, 8-week-old mice, or older mice Upon primary immunization, soluble GPC3 prepared at 100 μg/head and then emulsified using Freund's complete adjuvant (FCA, Becton, Dickinson and Company) was subcutaneously administered. 2 weeks later, soluble GPC3 was prepared at 50 μg/head and then emulsified using Freund's incomplete adjuvant (FIA, Becton, Dickinson and Company) The thus emulsified product was subcutaneously administered. Subsequently, booster immunization was performed a total of 5 times at intervals of 1 week. Final immunization was performed by administering soluble GPC3 diluted at 50 μg/head using PBS to 2 of these mice via the tail vein. At 4 days after final immunization, spleen cells were extracted and then mixed with mouse myeloma cells P3-X63Ag8U1 (P3U1, purchased from ATCC) at 2:1 PEG1500 (Roche Diagnostics) was gradually added, so that cell fusion was performed. RPMI1640 medium (GIBCO BRL) was carefully added to dilute PEG1500 and then PEG 1500 was removed by centrifugation. The resultant was suspended in RPMI1640 containing 10% FBS and then inoculated at 100 μL/well in a 96-well culture plate. On the next day, RPMI1640 (hereinafter, referred to as HAT medium) containing 10% FBS, 1 × HAT media supplement (SIGMA), and 0.5 × BM-Condimed H1 Hybridoma cloning supplement (Roche Diagnostics) was added at 100 μL/well. On days 2, 3, and 5, a half of each culture solution was substituted with HAT medium. Screening was performed using culture supernatants on day 7. Screening was performed by ELISA using an immunoplate on which the soluble GPC3 core protein had been immobilized. Positive clones were mono-cloned by a limiting dilution method. As a result, 11 clones (M3C11, M13B3, M1E7, M3B8, M11F1, L9G11, M19B11, M6B1, M18D4, M5B9, and M10D2) of antibodies having strong activity of binding to GPC3 were obtained

7. Cloning of anti-GPC3 antibody variable regions

Amplification was performed by an RT-PCR method using Total RNA extracted from hybridomas producing anti-GPC3 antibodies. Total RNA was extracted from $1 \times 10^7$ hybridoma cells using RNeasy Plant Mini Kits (QIAGEN). A 5' terminal gene fragment was amplified using 1 μg of Total RNA, a SMART RACE cDNA Amplification Kit (CLONTECH Laboratories, Inc.), and one of the following synthetic oligonucleotides:

a synthetic oligonucleotide MHC-IgG1 complementary to a mouse IgG1 constant region sequence
GGG CCA GTC GAT AGACAG ATG (SEQ ID NO: 21);
a synthetic oligonucleotide MHC-IgG2a complementary to a mouse IgG2a constant region sequence
CAG GGG CCA GTG GAT AGA CCG ATG (SEQ ID NO: 22);
a synthetic oligonucleotide MHC-IgG2b complementary to a mouse IgG2b constant region sequence
CAG GGG CCA GTG GAT AGA CTG ATG (SEQ ID NO: 23); and
a synthetic oligonucleotide kappa complementary to a mouse κ chain constant region nucleotide sequence
GCT CAC TGG ATG GTG GGA AGA TG (SEQ ID NO: 24).

A reverse transcription reaction was performed at 42°C for 1 hour and 30 minutes. 50 μL of a PCR solution contained 5 μL of 10 × Advantage 2 PCR Buffer, 5 μL of 10 × Universal Primer A Mix, 0.2 mM dNTPs (dATP, dGTP, dCTP, and dTTP), 1 μL of Advantage 2 Polymerase Mix (they were produced by CLONTECH Laboratories Inc.), 2.5 μL of the reverse transcription reaction product, and 10 pmole of synthetic oligonucleotide MHC-IgG1, MHC-IgG2a, MHC-IgG2b, or kappa. A reaction cycle of 94°C (initial temperature) for 30 seconds, 94°C for 5 seconds, and 72°C for 3 minutes was repeated 5 times. A reaction cycle of 94°C for 5 seconds, 70°C for 10 seconds, and 72°C for 3 minutes was repeated 5 times. Furthermore, a reaction cycle of 94°C for 5 seconds, 68°C for 10 seconds, and 72°C for 3 minutes was repeated 25 times. Finally the reaction pruducts were heated at 72°C for 7 minutes Each PCR product was purified from agarose gel using a QIAquick Gel Extraction Kit (produced by QIAGEN). The purified product was cloned into a pGEM-T Easy Vector (produced by Promega Corporation) so that each nucleotide sequence was determined.

The nucleotide sequences of the H chain variable regions of M3C11, M13B3, M1E7, M3B8, M11F1, M19B11, M6B1, M18D4, M5B9, M10D2, and L9G11 were represented by SEQ ID NOS: 25, 26, 27, 28, 29, 30, 31, 32, 33, 34, and 35, respectively. The amino acid sequences of the same were represented by SEQ ID NOS: 36, 37, 38, 39, 40, 41, 42, 43, 44, 45, and 46, respectively. The nucleotide sequences of the L chain variable regions of the same were represented by SEQ ID NOS: 47, 48, 49, 50, 51, 52, 53, 54, 55, 56, and 57, respectively The amino acid sequences of the same were represented by SEQ ID NOS: 58, 59, 60, 61, 62, 63, 64, 65, 66, 67, and 68, respectively.

8- Production of anti-GPC3 antibody mouse-human chimeric antibodies

H chain and L chain variable region sequences of anti-GPC3 antibodies were each ligated to human IgG1 and a κ chain constant region sequence. PCR was performed using a synthetic oligonucleotide being complementary to the 5' terminal nucleotide sequence of the H chain variable region of each antibody and having a Kozak sequence and a synthetic oligonucleotide complementary to the 3' terminal nucleotide sequence having an *Nhe* I site. The thus obtained PCR product was cloned into a pB-CH vector constructed by inserting the human IgG1 constant region into a pBluescript KS+ vector (TOYOBO Co., Ltd.). Because of the *Nhe* I site, the mouse H chain variable region had been ligated to the human H chain (γ1 chain) constant region. The thus prepared H chain gene fragment was cloned into an expression vector pCXND3. Furthermore, PCR was performed using a synthetic oligonucleotide being complementary to the 5' terminal nucleotide sequence of the L chain variable region of each antibody and having a Kozak sequence and a synthetic oligonucleotide complementary to the 3' terminal nucleotide sequence having a *Bsi*W I site. The thus obtained PCR product was cloned into a pB-CL vector constructed by inserting the human kappa chain constant region into a pBluescript KS+ vector (TOYOBO Co., Ltd.). Because of the *Bsi*W I site, the human L chain variable region had been ligated to the constant region. The prepared L chain gene figment was cloned into an expression vector pUCAG The vector pUCAG was constructed by ligating a 2.6-kbp fragment (obtained by digestion of pCXN (Niwa et al., Gene 1991; 108: 193-200) with a restriction enzyme *Bam*H I) to the restriction enzyme *Ban*H I site of a pUC19 vector (TOYOBO Co , Ltd.), followed by cloning.

To construct each anti-GPC3 mouse-human chimeric antibody expression vector, a gene fragment obtained by digestion of the pUCAG vector into which the L chain gene fragment had been inserted with a restriction enzyme *Hin*d III (TAKARA SHUZO CO., LTD.) was ligated to the restriction enzyme *Hin*d III cleavage site of pCXND3 into which an H chain gene had been inserted. Cloning was then performed. This plasmid expresses a neomycin resistance gene, a DHFR gene, and an anti-GPC3 mouse-human chimeric antibody gene in animal cells.

Stable expression cell lines were prepared using CHO cells (DG44 cell line) as follows. Gene transfer was performed by an electroporation method using Gene PulserII (produced by Bio-Rad Laboratories, Inc.). 25 μg of each anti-GPC3 mouse-human chimeric antibody expression vector was mixed with 0.75 ml of CHO cells suspended in PBS ($1 \times 10^7$ cells/ml) The mixture was cooled on ice for 10 minutes and then transferred into a cuvette. Pulses were then applied with capacity of 1.5 kV and 25 μFD After 10 minutes of a recovery period at room temperature, electroporated cells were suspended in 40 mL of CHO-S-SFMII medium (Invitrogen Corporation) containing an HT supplement (Invitrogen Corporation) at the same (1-fold) concentration. A 50-fold diluted solution was prepared using similar medium and then dispensed into a 96-well culture plate at 100 μl/well. After 24 houts of culture in a $CO_2$ incubator (5% $CO_2$), Geneticin (Invitrogen Corporation) was added at 0.5 mg/mL, followed by 2 weeks of culture. IgG amounts in the culture supernatants in wells for which colonies of Geneticin-resistant transformed cells had been observed were determined by a concentration determination method as described below. Culture of highly productive cell lines was scaled up in order. Thus, cell lines stably expressing the anti-GPC3 mouse-human chimeric antibodies were obtained and then subjected to large-scale culture, thereby resulting in culture supernatants. Each anti-GPC3 mouse-human chimeric antibody was purified using Hi Trap ProteinG HP (Amersham Biosciences).

9. Immunization with GC-3 and selection of hybridomas

Of the thus obtained anti-GPC3 antibodies, only M11F1 and M3B8 exerted strong CDC activity. Hence, it was revealed that CDC activity has epitope dependency. For the purpose of obtaining an antibody having both ADCC activity and CDC activity, immunization was performed with GC-3, which is a GST fusion protein containing M11F1 and M3BB epitopes. GC-3 was purified by the above method in a large amount. The resultant was subjected to gel filtration using Superdex75 (Amersham Biosciences) and then the buffer was substituted with PBS. The product was used as an immune protein. Three Balb/c (purchased from CHARLES RIVER LABORATORIES JAPAN, INC.) and three MRL/lpr mice were immunized with GC-3 according to the above method. Upon primary immunization, GC-3 prepared at 100 μg/head and emulsified using FCA was subcutaneously administered, 2 weeks later, GC-3 prepared at 50 μg/head and then emulsified using FIA was subcutaneously administered. After 5 times of immunization, final immunization (50 μg/head) was performed for all mice through administration via the tail vein so that cell fusion was performed. Screening was performed by ELISA using an immunoplate on which the soluble GPC3 core protein had been immobilized Positive clones were mono-cloned by a limiting dilution method. As a result, 5 clones (GC199, GC202, GC33, GC179, and GC194) of antibodies having strong activity of binding to GPC3 were obtained.

The H chain and L chain variable regions of GC199, GC202, GC33, GC179, and GC194 were cloned according to the above method and then the sequences thereof were determined. Regarding GC194 L chain, 2 types of sequence were cloned The nucleotide sequences of the H chain variable regions of GC199, GC202, CC33, GC179, and GC194 were represented by SEQ ID NOS: 69, 70, 71, 72, and 73, respectively The amino acid sequences of the same were represented by SEQ ID NOS: 74, 75, 76, 77, and 78, respectively. The nucleotide sequences of the L chain variable regions of GC199, GC202, GC33, GC179, OC194(1), and GC194(2) were represented by SEQ ID NOS: 79, 80, 81, 82, 83, and 84, respectively. The amino acid sequences of the same were represented by SEQ ID NOS: 85, 86, 87, 88, 89, and 90, respectively.

Reference example 2 Humanization of GC33

Antibody sequence data was obtained from Kabat Database (ftp://ftp ebi.ac.ult/pub/databases/kabat/) and ImMunoGeneTics Database (IMGT), which are open to the public. The H chain variable regions and the L chain variable regions were separately subjected to homology search. As a result, it was revealed that the H chain variable regions have high homology with DN13 (Smithson et al.., Mol Immunol. 1999; 36: 113-124). It was also revealed that the L chain variable regions have high homology with accession number AB064105, homo sapiens IGK mRNA for ímmunoglobulin kappa light chain VL1 region, partial cds, clone: K64. Furthermore, the signal sequence of accession Number S40357 having high homology with AB064105 was used for an L chain signal sequence A complementary determining region (hereinafter, referred to as CDR) was transplanted into the framework region (hereinafter, referred to as FR) of each of these antibodies, so that a humanized antibody was prepared.

Specifically, each synthetic oligo DNA comprising approximately 50 bases was designed, so that approximately 20 bases of such 50-base synthetic oligo DNA undergo hybridization. These synthetic oligo DNAs were assembled by the PCR method, thereby preparing genes each encoding a different variable region. Each of these genes was cloned into an expression vector HEFgγ1 (constructed via cleavage at the *Hind* III sequence inserted at the end of 5' terminal synthetic oligo DNA and at the *Bam*H I sequence inserted at the end of 3' terminal synthetic oligo DNA and cloning of a human IgG1 constant legion thereinto) and an expression vector HEFgκ (constructed via cleavage at the same sites and cloning of a human κ chain constant region thereinto) (Sato et al., Mol Immunol. 1994; 371-381). The H chain and the L chain of the thus prepared humanized GC33 were each named "ver a." Humanized GC33 (ver.a/ver.a) in which both H and L chains were "ver a" exerted binding activity lower than that of an antibody (mouse/mouse) having a mouse GC33 variable region. Through a chimeric combination of a mouse GC33 sequence and ver a sequence for H chain and L chain, respectively, antibodies (mouse/vera and ver.a/mouse) were produced. Then the binding activity of the chimeric antibodies were evaluated. As a result, lowered binding activity was confirmed in ver.a/mouse, revealing that lowered binding activity due to amino acid substitution is caused by an H chain. Hence, altered II chains, ver.c, ver.f, ver.h, ver.i, ver.j, and vei.k, were prepared. All types of humanized GC33 exerted binding activity equivalent to that of chimeric antibodies having the mouse GC33 variable regions. The nucleotide sequences of the humanized GC33 H chain variable regions (ver.a, ver.c, ver.f, ver.h, ver.i, ver.j, and ver.k) are represented by SEQ ID NOS: 91, 92, 93, 94, 95, 96, and 97, respectively. The amino acid sequences of the same are represented by SEQ ID NOS: 98, 99, 100, 101, 102, 103, and 104, respectively. The nucleotide sequence of humanized GC33 L chain variable region ver.a is represented by SEQ ID NO: 105 and the amino acid sequence of the same is represented by SEQ ID NO: 106.

In humanized GC33 H chain variable regions (ver.i, ver.j., and ver.k), glutamic acid at position 6 had been substituted with glutamine These antibodies exerted significantly enhanced thermostability

Reference example 3 Alteration of humanized GC33 L chain

Regarding protein deamidation, a primary-sequence-dependent deamidation (reaction) rate constant is known. Asn-Gly is known as a sequence that particularly tends to undergo deamidation (Rocinsan et al., Proc. Natl. Acad. Sci. U.S.A. 2001; 98; 944-949.). Regarding Asn33 within CDR1 of humanized GC33 L chain ver. a variable region represented by SEQ ID NO: 105, it was predicted that the Asn33 sequence would tend to undergo deamidation since the primary sequence is Asn-Gly.

For evaluation of the effect of Asn33 deamidation on binding activity, an altered antibody was produced in which Asn33 had been substituted with Asp. For introduction of point mutation, a Quick Change Site-Directed Mutagenesis Kit (Stratagene Corporation) was used Specifically, 50 μL of a reaction solution containing 125 ng of a sense primer (CTT GTA CAC AGT GAC GGA AAC ACC TAT:SEQ ID NO: 107), 125 ng of an antisense primer (ATA GGT GTT TCC GTC ACT GTG TAC AAG: SEQ ID NO: 108), 5 μL of 10× reaction buffer, 1 μL of dNTP mix, 10 ng of HEFgκ in which humanized GC33 L chain ver.a had been cloned, and 1 μL of Pfu Turbo DNA Polymerase was subjected to 12 reaction cycles, each cycle consisting of 95°C for 30 seconds, 55°C for 1 minute, and 68°C for 9 minutes. Subsequently, restriction enzyme *Dpn* I was added to perform digestion at 37°C for 2 hours. The resultant was introduced into attached XL1-Blue competent cells, thereby obtaining transformants. Clones in which mutation had been introduced successfully were subjected to excision of variable regions and then cloned into HEFgκ again. The resultants were introduced together with HEFgγ1 in which humanized GC33 H chain ver-k had been cloned into COS7 cells using Fugene6 (Roche diagnostics). The culture supernatants of COS7 cells that had been caused to transiently express the antibodies were collected Antibody concentrations were determined by Sandwich ELISA using an anti-human IgG antibody. The binding activity of the altered antibodies was evaluated by ELISA using an immobilized soluble OPC3 core protein. In the case of the altered antibody (N33D) in which Asn33 had been substituted with Asp, binding activity had disappeared. Hence, it was considered that the effect of Asn33 deamidation on binding activity is significant

As a method for suppressing Asn33 deamidation, a method that involves altering Gly34 to result in another amino acid has been reported (WO03057881A1). According to this method, altered antibodies G34A, G34D, G34E, G34F, G34H, G34N, G34P, G34Q, G34I, G34K, G34L, G34V, G34W, G34Y, G34R, G34S, and G34T, which had experienced substitution with 17 amino acids excluding Cys and Met, were produced using a Quick Change Site-Directed Mutagenesis Kit. Binding activity was evaluated using the culture supernatants of COS7 cells that had transiently expressed the antibodies. As a result, it was revealed that amino acid substitution with those other than Pro(G34P) and Val(G34V) did not affect the maintenance of binding activity

The amino acid sequences of the light chain CDR1 of the above altered antibodies are shown in SEQ ID NO: 109 (G34A), SEQ ID NO: 110 (G34D), SEQ ID NO: 111 (G34E), SEQ ID NO: 112 (G34F), SEQ ID NO: 113 (G34H), SEQ ID NO: 114 (G34N), SEQ ID NO: 115 (G34T), SEQ ID NO: 116 (G34Q), SEQ ID NO: 117 (G34I), SEQ ID NO: 118 (G34K), SEQ ID NO: 119 (G34L), SEQ ID NO: 120 (G34S), SEQ ID NO: I21 (G34W), SEQ ID NO: 122 (G34Y), SEQ ID NO: 123 (G34R), SEQ ID NO: 124 (G34V), and SEQ ID NO: 125 (G34P), respectively. Furthermore, the amino acid sequences of the light chain variable regions of the above altered antibodies are shown in SEQ ID NO: 126 (G34A), SEQ ID NO: 127 (G34D), SEQ ID NO: 128 (G34E), SEQ ID NO: 129(G34F), SEQ ID NO: 130 (G34H), SEQ ID NO: 131 (G34N), SEQ ID NO: 132 (G34T), SEQ ID NO: 133 (G34Q), SEQ ID NO: 134 (G34I), SEQ ID NO: 135 (G34K), SEQ ID NO: 136 (G34L), SEQ ID NO: 137 (G34S), SEQ ID NO: 138 (G34W), SEQ ID NO: 133 (G34Y), SEQ ID NO: 140 (G34R), SEQ ID NO: 141 (G34V), and SEQ ID NO: 142(G34P), respectively.

Industrial Applicability

The present invention provides cells in which the expression of fucose transporter genes on both chromosomes is artificially suppressed. When the cells are used for producing a protein, a recombinant protein not having fucose can be produced Such a recombinant protein not having fucose possesses reduced cytotoxic activity compared with that of proteins having fucose Furthermore, stability of such a recombinant protein is equivalent to that of proteins having fucose. The protein of the present invention is particularly advantageous when it is used for a recombinant antibody to be used as an antibody drug.

All publications, patents, and patent applications cited herein are incorporated herein in their entirety A person skilled in the art would easily understand that various modifications and changes of the present invention are feasible within the range of the technical ideas and the scope of the invention as disclosed in the attached claims. The present invention is intended to include such modifications and changes

The invention also relates to the following aspects as defined in the following numbered paragraphs:

1. A cell, in which the expression of fucose transporter genes on both chromosomes is artificially suppressed.
2. The cell according to paragraph 1, in which the fucose transporter genes are disrupted.
3. The cell according to paragraph 1 or 2, which is an animal cell.
4. The cell according to paragraph 3, in which the animal cell is a Chinese hamster cell.
5. The cell according to paragraph 4, in which the animal cell is a CHO cell.
6. The cell according to any one of paragraphs 2 to 5, in which gene disruption is carried out by homologous recombination using a gene targeting vector.
7. The cell according to any one of paragraphs 1 to 6, in which gene encoding a foreign protein is introduced.
8. The cell according to paragraph 7, in which the gene encoding the foreign protein is a gene encoding an antibody.
9. A method of producing a protein, comprising culturing the cell according to any one of paragraphs 1 to 8.
10. The production method according to paragraph 9, in which the protein is an antibody.
11. The production method according to paragraph 10, comprising producing a protein to which fucose is not added,

12. A method for inhibiting the addition of fucose to a protein, comprising artificially suppressing the expression of fucose transporter genes on both chromosomes upon production of a recombinant protein using a cell.

13. The method for inhibiting the addition of fucose to a protein according to paragraph 12, comprising artificially suppressing gene expression through deletion of a gene.

14. The method for inhibiting the addition of fucose to a protein according to paragraph 12 or 13, in which the protein is an antibody.

15. The method for inhibiting the addition of fucose to a protein according to any one of paragraphs 12 to 14, in which the cell is a CHO cell.

## Claims

1. A method of producing an antibody of which the ADCC activity is enhanced to a level 2 or more times greater than that of an antibody produced using an animal cell having an undisrupted fucose transporter gene and in which 90% or more of all the sugar chains has not experienced the addition of fucose thereto, comprising culturing an animal cell in which fucose transporter genes on both chromosomes are disrupted

2. The method according to claim 1, wherein the animal cell is a Chinese hamster cell.

3. The method according to claim 2, wherein the Chinese hamster cell is a CHO cell.

4. The method according to any one of claims 1 to 3, wherein the fucose transporter genes are disrupted by homologous recombination using a target vector.

Fig. 1

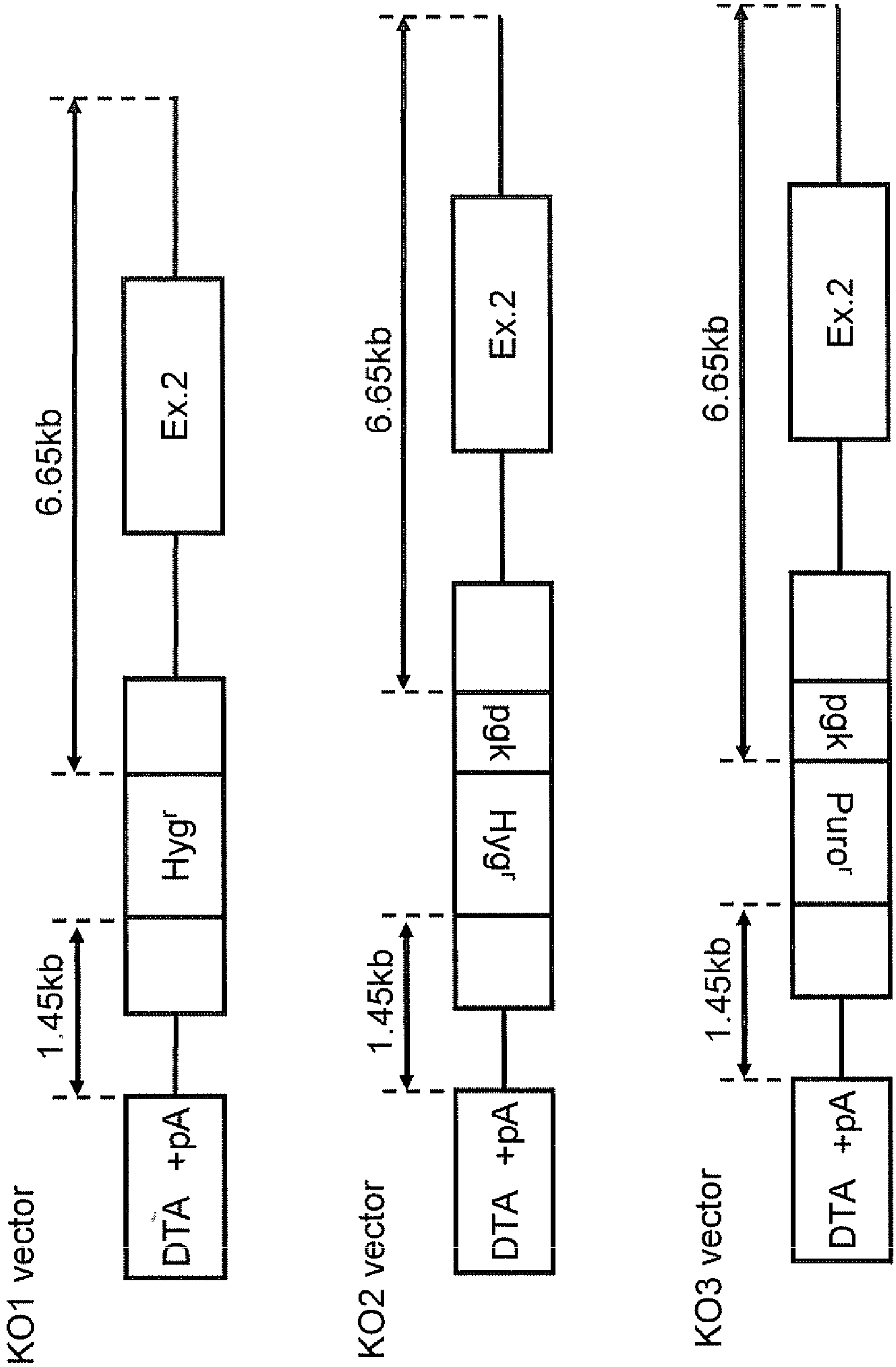
KO1 vector
DTA +pA
1.45kb
Hygr
6.65kb
Ex.2
KO2 vector
DTA +pA
1.45kb
Hygr
pgk
6.65kb
Ex.2
KO3 vector
DTA +pA
1.45kb
Puror
pgk
6.65kb
Ex.2

Fig. 2

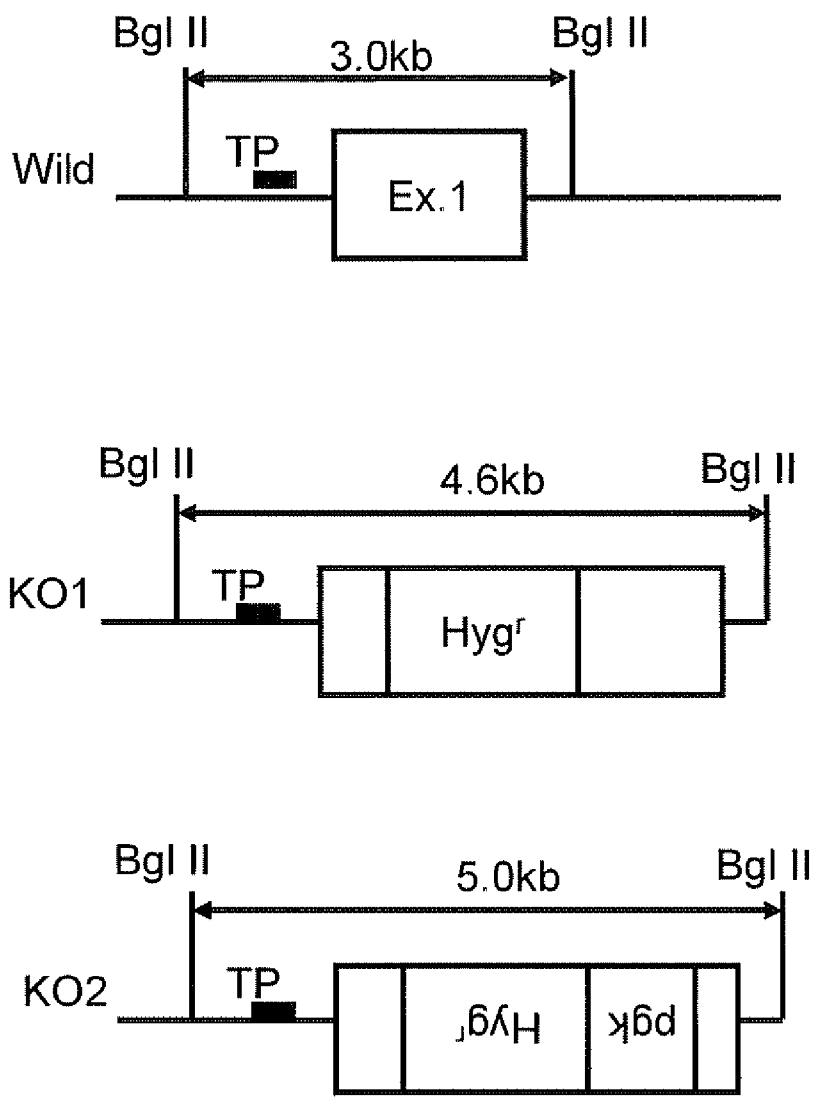
Bgl II
3.0kb
Bgl II
Wild
TP
Ex.1
Bgl II
4.6kb
Bgl II
KO1
TP
Hyg^r
Bgl II
5.0kb
Bgl II
KO2
TP
Hyg^r
pgk

Fig. 3

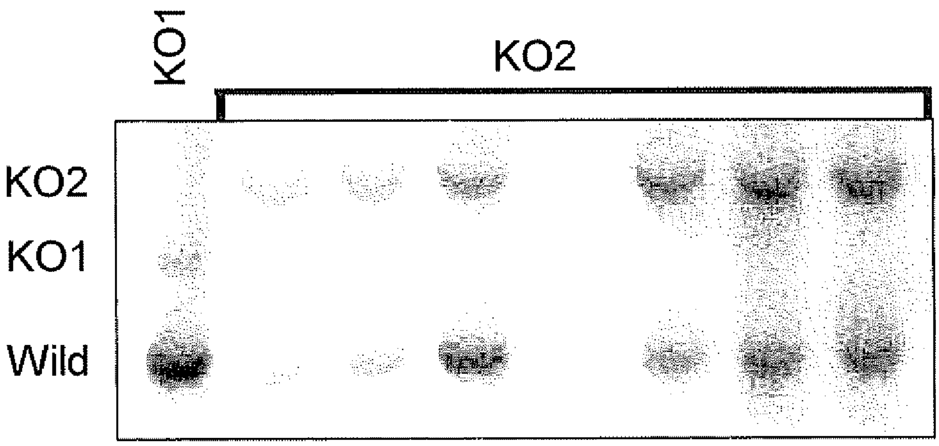

Fig. 4

| Methods | Vectors/Cells | Homologous recombinants/ Tested cells | Efficiency % |
|---|---|---|---|
| 1 | KO2 / KO1 | 18 / 616 | 2.9 |
| 2 | KO2 / KO2 | 2 / 524 | 0.4 |
| 3 | KO3 / KO2 | 7 / 382 | 1.8 |

Fig. 5

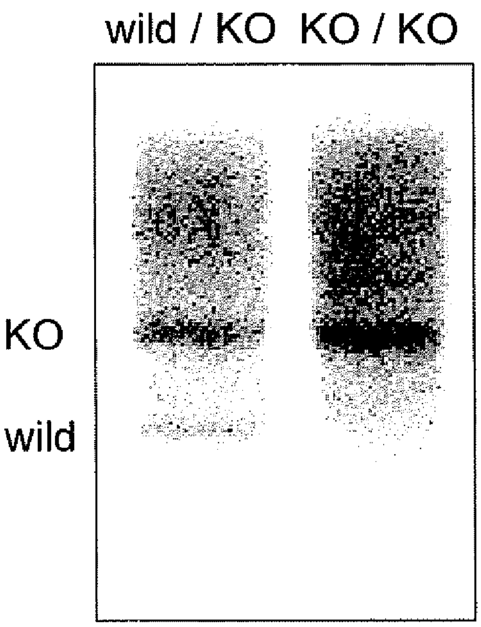

Fig. 6

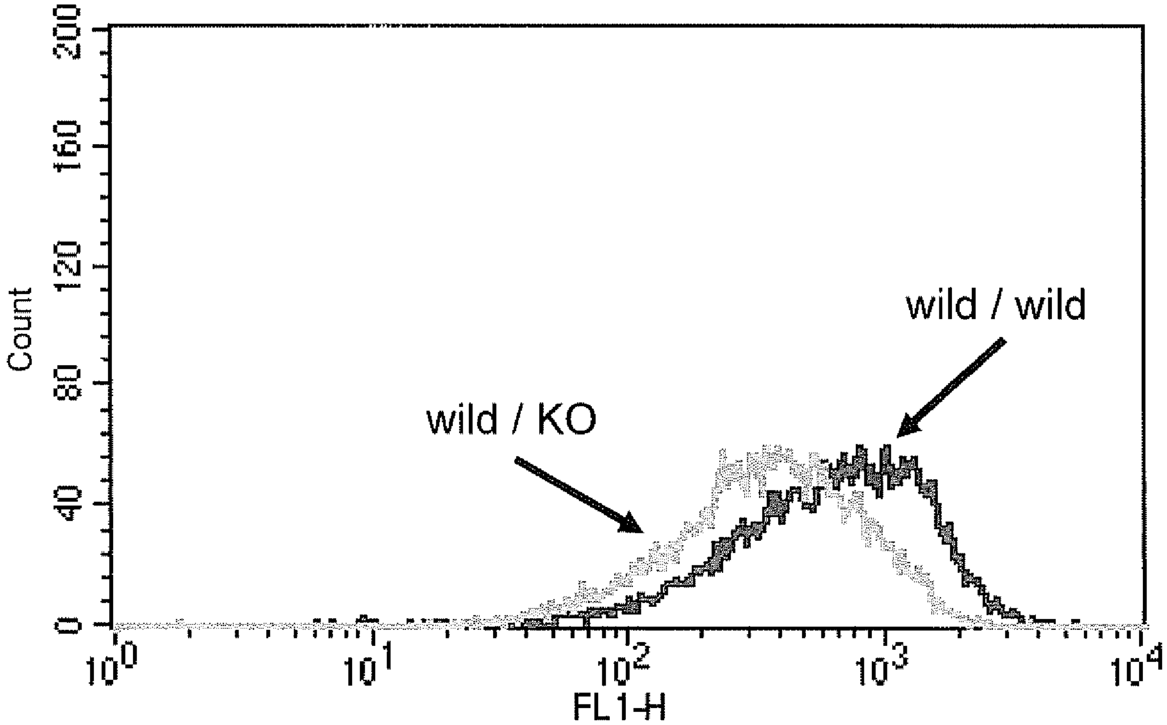
200
160
120
80
40
0
Count
wild / wild
wild / KO
10^0
10^1
10^2
10^3
10^4
FL1-H

Fig. 7

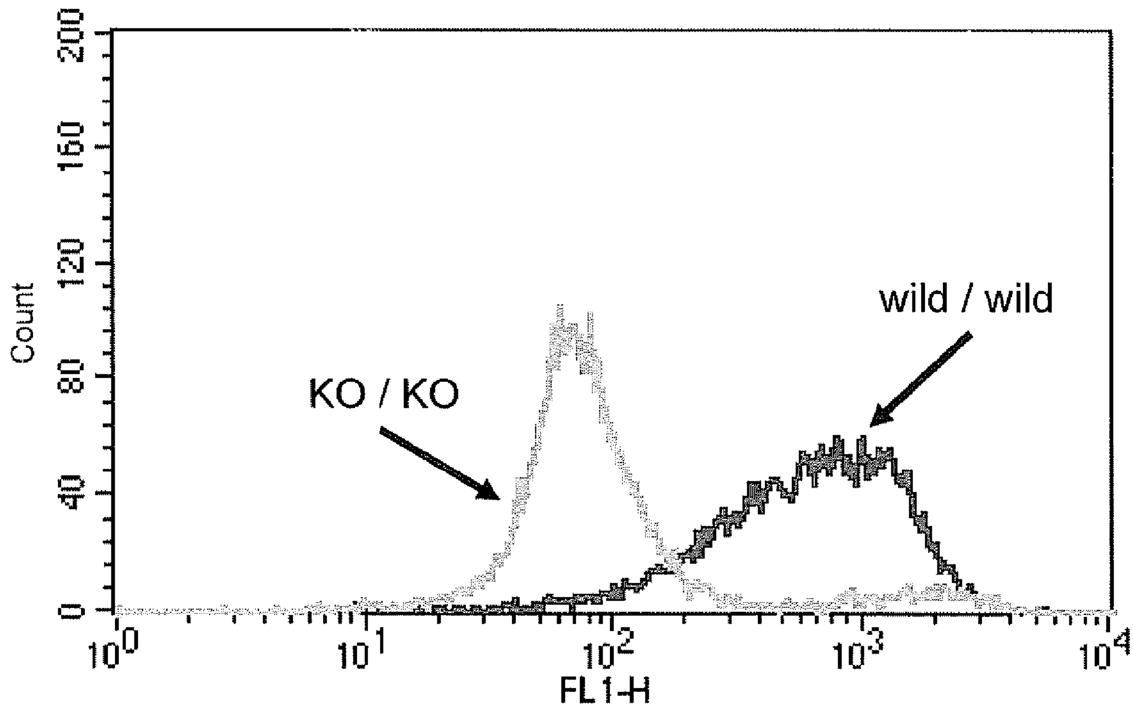

200
160
120
80
40
0
Count
KO / KO
wild / wild
$10^0$
$10^1$
$10^2$
$10^3$
$10^4$
FL1-H

Fig. 8

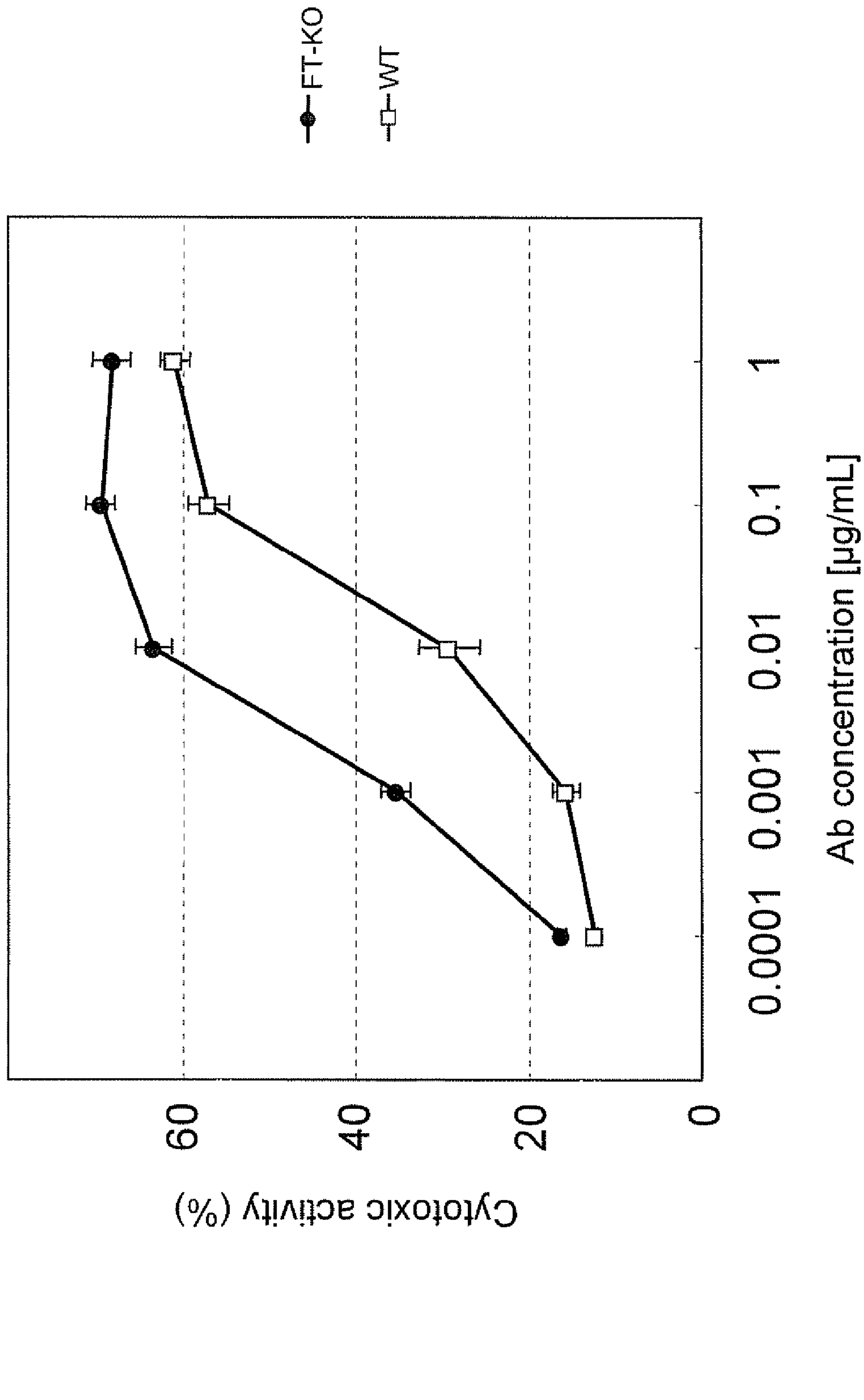
FT-KO
WT
Cytotoxic activity (%)
0
20
40
60
0.0001
0.001
0.01
0.1
1
Ab concentration [µg/mL]

Fig. 9

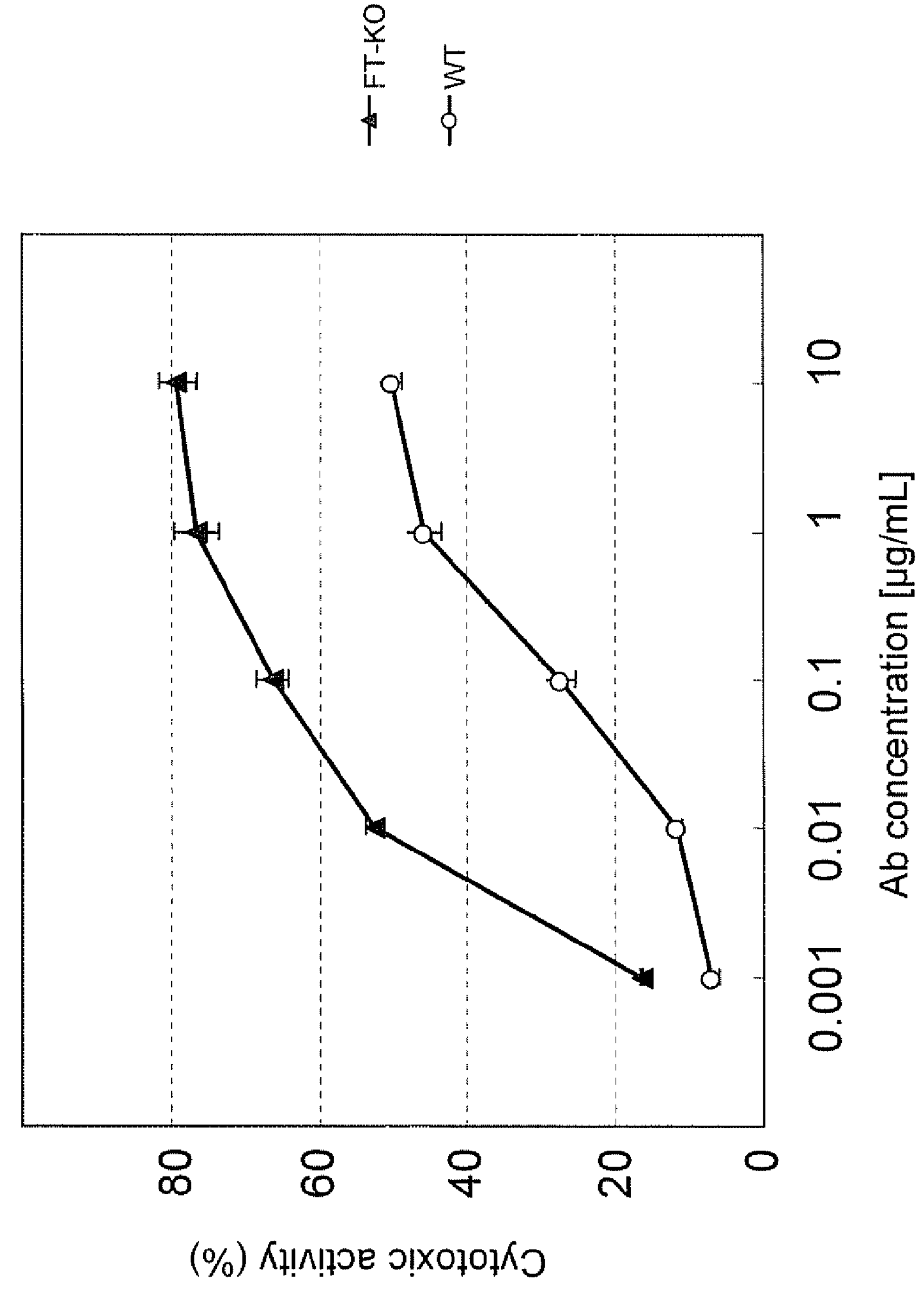
FT-KO
WT
Cytotoxic activity (%)
80
60
40
20
0
0.001
0.01
0.1
1
10
Ab concentration [μg/mL]

Fig. 10

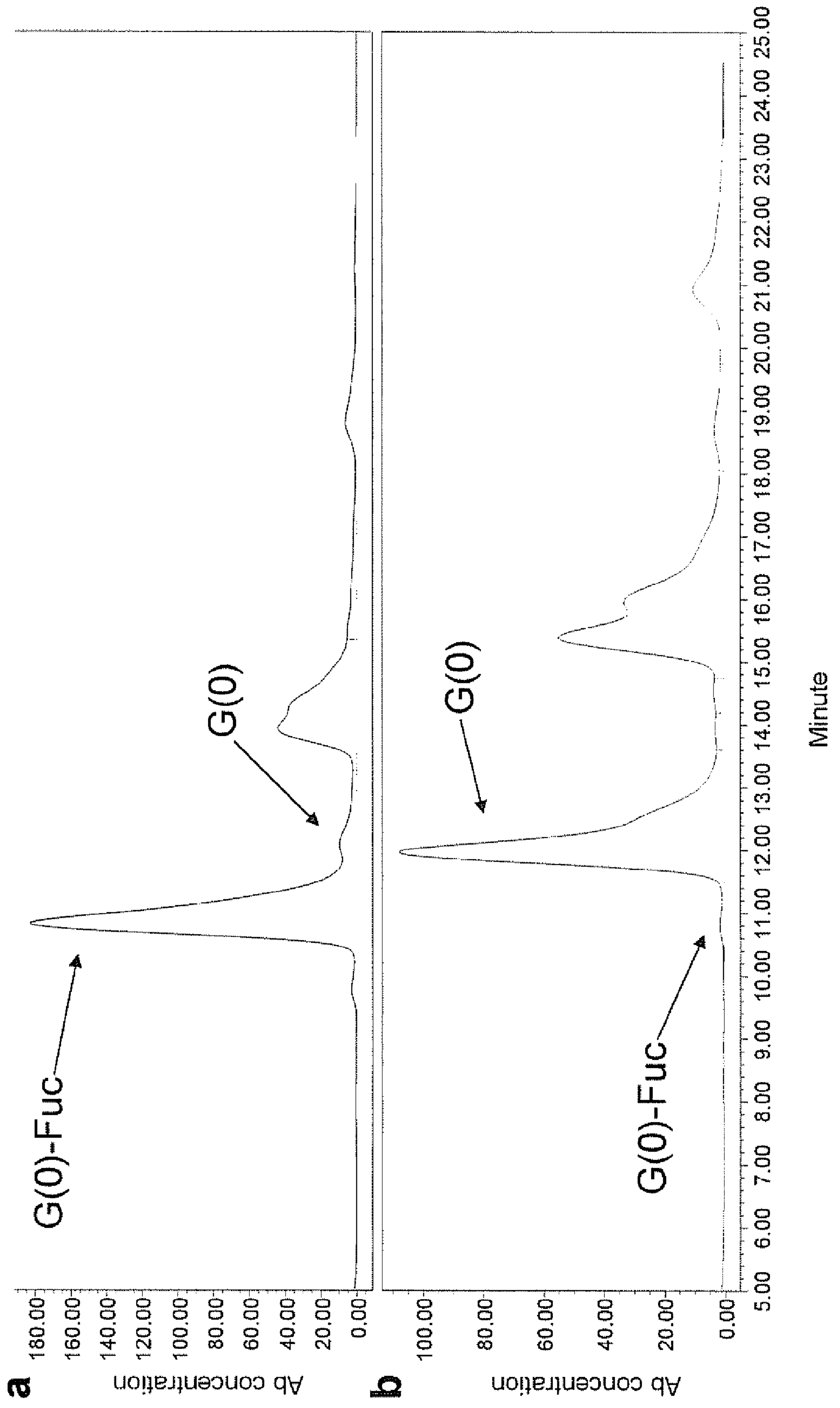
a
b
G(0)-Fuc
G(0)
G(0)-Fuc
G(0)
Ab concentration
Ab concentration
180.00
160.00
140.00
120.00
100.00
80.00
60.00
40.00
20.00
0.00
100.00
80.00
60.00
40.00
20.00
0.00
5.00
6.00
7.00
8.00
9.00
10.00
11.00
12.00
13.00
14.00
15.00
16.00
17.00
18.00
19.00
20.00
21.00
22.00
23.00
24.00
25.00
Minute

Fig. 11

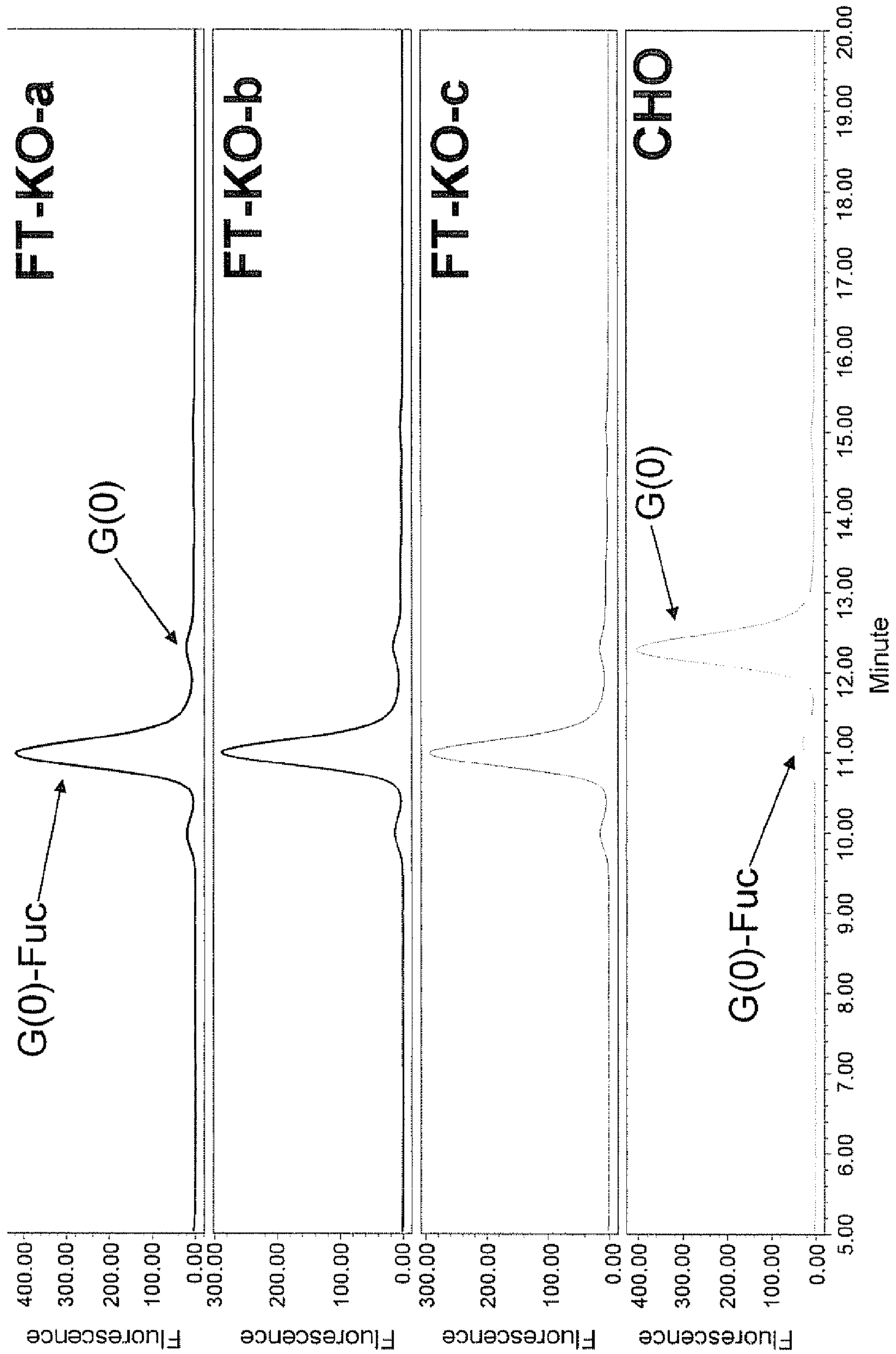
FT-KO-a
FT-KO-b
FT-KO-c
CHO
G(0)-Fuc
G(0)
G(0)-Fuc
G(0)
Fluorescence
Fluorescence
Fluorescence
Fluorescence
400.00
300.00
200.00
100.00
0.00
5.00
6.00
7.00
8.00
9.00
10.00
11.00
12.00
13.00
14.00
15.00
16.00
17.00
18.00
19.00
20.00
Minute

Fig. 12

**G(0)**

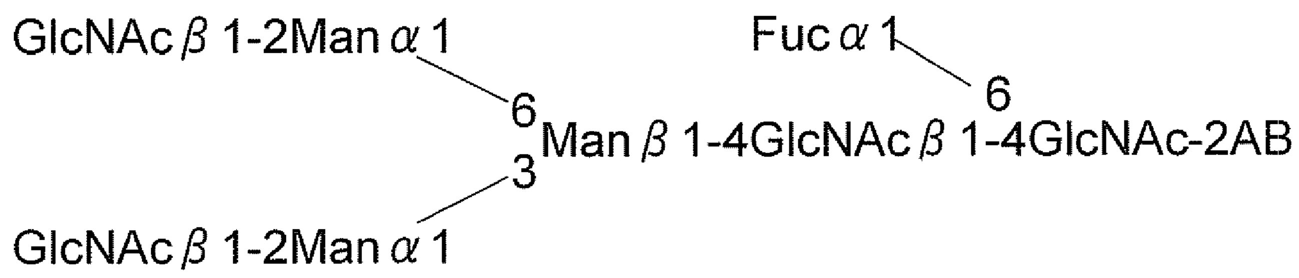

**G(0)-Fuc**

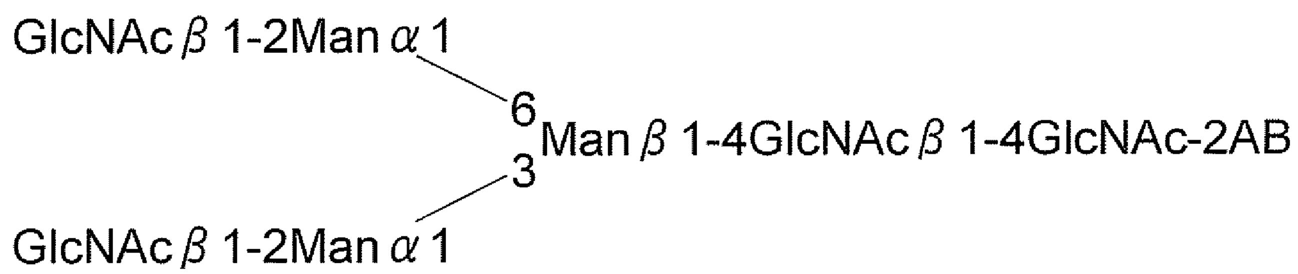

Fig. 13

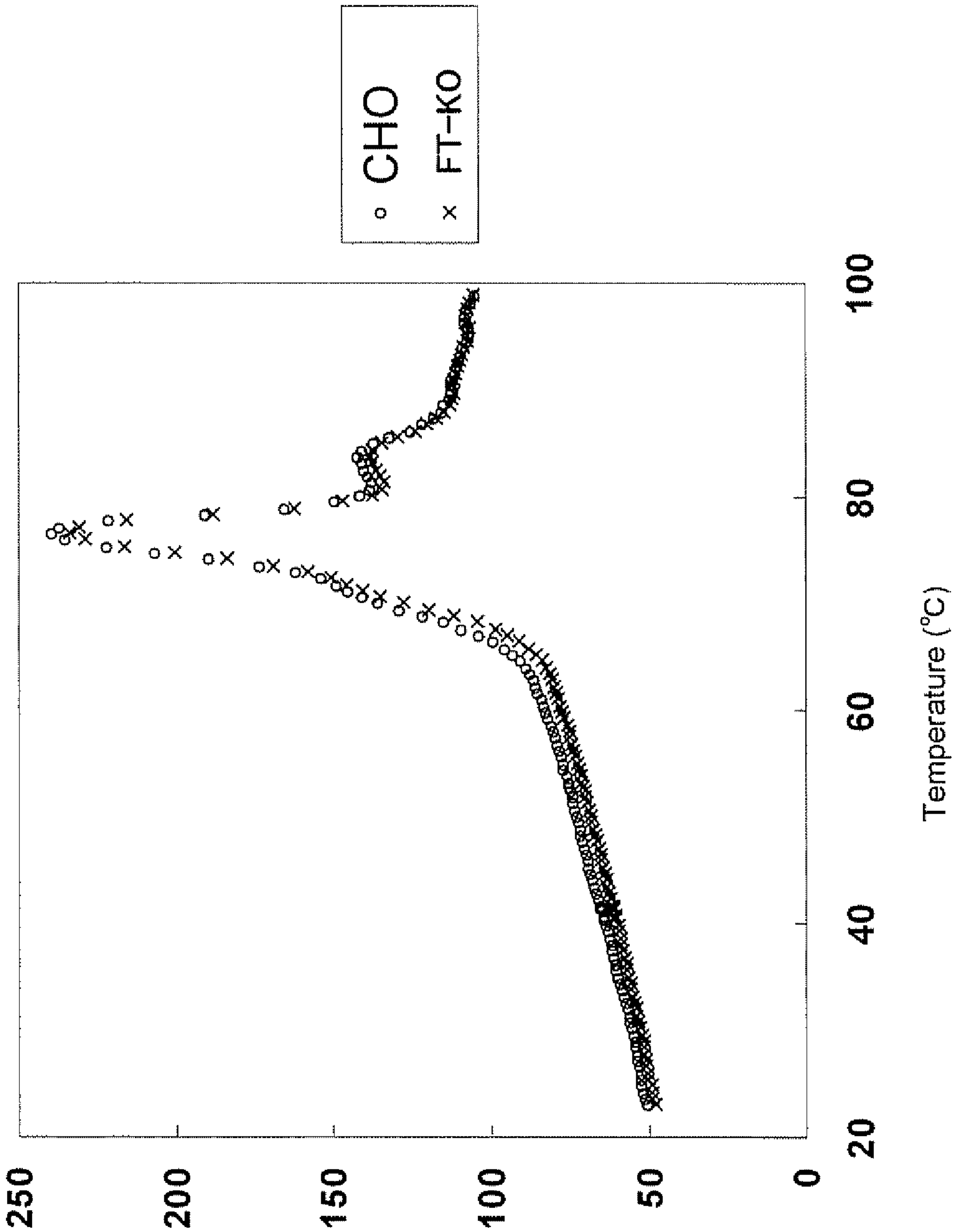
CHO
FT-KO
Molar heat capacity(kcal/kmol)
Temperature (°C)
0
50
100
150
200
250
20
40
60
80
100

Europäisches Patentamt
European Patent Office
Office européen des brevets

# EUROPEAN SEARCH REPORT

Application Number
EP 10 16 2932

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | YAMANE-OHNUKI N ET AL: "Establishment of FUT8 knockout chinese hanster ovary cells: an ideal host cell line for producing completely defucosylated antibodies with enhanced antibody-dependent cellular cytotoxicity" BIOTECHNOLOGY AND BIOENGINEERING, WILEY & SONS, HOBOKEN, NJ, US LNKD- DOI:10.1002/BIT.20151, vol. 87, no. 5, 6 August 2004 (2004-08-06), pages 614-622, XP002983758 ISSN: 0006-3592 * the whole document * | 1-4 | INV. C12N15/00 A01H5/00 A01K67/027 A61K39/395 A61P9/00 A61P29/00 A61P31/04 A61P31/12 A61P35/00 A61P37/00 A61P37/08 |
| Y | US 2004/110282 A1 (KANDA YUTAKA [JP] ET AL) 10 June 2004 (2004-06-10) * figure 2 * | 1-4 | |
| A | MORTENSEN R M ET AL: "Inactivation of G-protein genes: double knockout in cell lines" 1 January 1994 (1994-01-01), METHODS IN ENZYMOLOGY; HETEROTRIMERIC G PROTEINS; [METHODS IN ENZYMOLOGY], ACADEMIC PRESS INC, SAN DIEGO, CA, US LNKD- DOI:10.1016/S0076-6879(94)37075-3, PAGE(S) 356 - 366, XP008115526 ISSN: 0076-6879 *Introduction* | 1-4 | TECHNICAL FIELDS SEARCHED (IPC): C12N C07K |

The present search report has been drawn up for all claims

| Place of search | Date of completion of the search | Examiner |
|---|---|---|
| The Hague | 6 July 2010 | Cupido, Marinus |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT ON EUROPEAN PATENT APPLICATION NO.** EP 10 16 2932

This annex lists the patent family members relating to the patent documents cited in the above-mentioned European search report.
The members are as contained in the European Patent Office EDP file on
The European Patent Office is in no way liable for these particulars which are merely given for the purpose of information.

06-07-2010

| Patent document cited in search report | Publication date | Patent family member(s) | Publication date |
|---|---|---|---|
| US 2004110282 A1 | 10-06-2004 | AU 2003236020 A1 | 20-10-2003 |
| | | CA 2481656 A1 | 16-10-2003 |
| | | EP 1500698 A1 | 26-01-2005 |
| | | WO 03085102 A1 | 16-10-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0061739 A
- JP 2004019261 W
- EP 239400 A
- WO 9602576 A
- JP 1059878 B
- WO 9312227 A
- WO 9203918 A
- WO 9402602 A
- WO 9425585 A
- WO 9634096 A
- WO 9633735 A
- WO 9201047 A
- WO 9220791 A
- WO 9306213 A
- WO 9311236 A
- WO 9319172 A
- WO 9501438 A
- WO 9515388 A
- EP 404097 A
- WO 9311161 A
- JP 11503015 A
- WO 0233054 A
- WO 9846777 A
- WO 2005014651 A
- WO 03057881 A1


### Non-patent literature cited in the description


- **Shields et al.** *J Biol Chem,* 2002, vol. 277 (30), 26733-26740
- **Pate L. Smith et al.** *J. Cell Biol.,* 2002, vol. 158, 801-815
- **Sato, K. et al.** *Cancer Res,* 1993, vol. 53, 851-856
- **Huston, J. S. et al.** *Proc. Natl Acad. Sci. U.S.A.,* 1998, vol. 85, 5879-5883
- **Co, M. S. et al.** *J. Immunol.,* 1994, vol. 152, 2968-2976
- **Better, M ; Horwitz, A. H.** *Methods Enzymol.,* 1989, vol. 178, 476-496
- **Pluckthun, A. ; Skerra, A.** *Methods Enzymol.,* 1989, vol. 178, 497-515
- **Lamoyi, E.** *Methods Enzymol.,* 1986, vol. 121, 652-663
- **Rousseaux, J. et al.** *Methods Enzymol,* 1986, vol. 121, 663-669
- **Bird, R. E. ; Walker, B. W.** *Trends Biotechnol.,* 1991, vol. 9, 132-137
- **P. Hoiliger et al.** *Proc Natl. Acad. Sci. U.S.A.,* 1993, vol. 90, 6444-6448
- **Johnson et al.** *Methods in Enzymology,* 1991, vol. 203, 88-98
- **Holliger et al.** *Protein Engineering,* 1996, vol. 9, 299-305
- **Perisic et al.** *Structure,* 1994, vol. 2, 1217-1226
- **John et al.** *Protein Engineering,* 1999, vol. 12 (7), 597-604
- **Holliger et al.** *Proc Natl. Acad. Sci. U.S.A,* 1993, vol. 90, 6444-6448
- **Atwell et al.** *Mol. Immunol.,* 1996, vol. 33, 1301-1312
- **Carl, A. K. Borrebaeck ; James, W. Larrick.** THERAPEUTIC MONOCLONAL ANTIBODIES. MACMILLAN PUBLISHERS LTD, 1990
- *J. Exp. Med.,* 1995, vol. 108, 945
- **Valle et al.** *Nature,* 1981, vol. 291, 358-340
- *Proc Natl. Acad. Sci. U.S.A.,* 1980, vol. 77, 4216-4220
- *Proc. Natl. Acad. Sci. U.S.A.,* 1968, vol. 60, 1275
- *Nucleic Acids. Res.,* 1990, vol. 19 (17), 5322
- **Mulligan et al.** *Nature,* 1979, vol. 277, 108
- **Mizushima et al.** *Nucleic Acids Res.,* 1990, vol. 18, 5322
- **Kenichi Yamamura et al.** Transgenic Animal. KYORITSU SHUPPAN CO., LTD, 01 March 1997
- **Shinichi Aizawa.** Gene Targeting, Production of Mutant Mice using ES cells, Bio Manual Series 8. YODOSHA CO., LTD, 1995
- **Hogan et al.** Manipulating the Mouse Embryo. Cold Spring Harbor Laboratory Press, 1944
- **Joyner, A.L.** Gene Targeting, A Practical Approach Series. IRL Press, 1993
- **Masami Matsumura et al.** Experimental Medicine, Separate Volume, New Genetic Engineering Handbook. YODOSHA CO., LTD,
- **Vicki Glaser.** *SPECTRUM Biotechnology Applications,* 1993
- **Ebert, K.M. et al.** *Bio/Technology,* 1994, vol. 12, 699-702

- Strategies for Protein Purification and Characterization: A Laboratory Course Manual. Cold Spring Harbor Laboratory Press, 1996
- **Kohler, G ; Milstein, C.** *Methods Enzymol.,* 1981, vol. 73, 3-46
- Antibodies: A Laboratory Manual. Cold Spring Harbor Laboratory, 1988
- Antibodies A Laboratory Manual. Cold Spring Harbor Laboratory, 1988
- *Anal Biochem,* 1988, vol. 171, 73
- Experimental Methods 23-Methods for Studying Glycoprotein Sugar Chains. Center for Academic Publications, 1989
- Current protocols in Immunology. Immunologic studies in humans. John Wiley & Sons, Inc, 1993
- **Yagi T.** *Proc. Natl. Acad. Sci. U.S.A.,* 1990, vol. 87, 9918-9922
- **Popo H.** *Biochemical Genetics,* 1990, vol. 28, 299-308
- **Weitzhandler M et al.** *Journal of Pharmaceutical Sciences,* 1994, vol. 83 (12), 1670-1675
- **Schenk B et al.** *The Journal of Clinical Investigation,* 2001, vol. 108 (11), 1687-1695
- **Bigge I C. et al.** *Analytical Biochemistry,* 1995, vol. 230 (2), 229-238
- **Weitzhandler M. et al.** *Journal of Pharmaceutical Sciences,* 1994, vol. 12, 1670-1675
- **Schenk B. et al.** *The Journal of Clinical Investigation,* 2001, vol. 108 (11), 1687-1695
- **Bigge J. C. et al.** *Analytical Biochemistry,* 1995, vol. 230 (2), 229-238
- **Hirata et al.** *FEBS letter,* 1994, vol. 356, 244-248
- **Niwa et al.** *Gene,* 1991, vol. 108, 193-199
- **Niwa et al.** *Gene,* 1991, vol. 108, 193-200
- **Smithson et al.** *Mol Immunol.,* 1999, vol. 36, 113-124
- **Sato et al.** *Mol Immunol.,* 1994, 371-381
- **Rocinsan et al.** *Proc. Natl. Acad. Sci. U.S.A.,* 2001, vol. 98, 944-949